# EUROPEAN PATENT APPLICATION

(11) **EP 4 458 854 A1**
(43) Date of publication of application: **06.11.2024**
(21) Application number: 22915137.8
(22) Date of filing: 30.12.2022
(51) Int. Cl.: C07K 16/28, A61P 35/00, A61K 39/395, A61K 35/17

(54) **GPRC5D ANTIBODY AND APPLICATION THEREOF**

(30) Priority: 31.12.2021 CN 202111668463
(71) Applicant: Shandong Simcere Biopharmaceutical Co., Ltd., Shandong 264006 (CN)
(72) Inventor: FU, Yayuan, Shanghai 201318 (CN); LIAO, Jingli, Shanghai 201318 (CN); YIN, Bowei, Shanghai 201318 (CN); CAO, Zhuoxiao, Shanghai 201318 (CN); TANG, Renhong, Shanghai 201318 (CN); REN, Jinsheng, Nanjing, Jiangsu 210042 (CN)
(74) Representative: Richly & Ritschel Patentanwälte PartG mbB
(86) International application number: PCT/CN2022/143719
(87) International publication number: WO 2023/125888

(57) **Abstract**

The present invention relates to an antibody against G protein coupled receptor C5 family subtype D (GPRCSD) and an application of the antibody. Specifically, disclosed are an antibody specifically binding to GPRCSD or an antigen-binding fragment thereof, and an encoding nucleic acid thereof, an expression vector and an expression cell, a preparation method, a pharmaceutical composition, and a use thereof in the preparation of a pharmaceutical composition for treating diseases, for example, a use in treating tumors. The present invention has important significance for the development of GPRCSD antibody treatment drugs and detection reagents.

## Description

### Cross Reference to Related Patent Application

The present application claims the right of priority for Chinese patent application no. 202111668463.1, entitled "GPRC5D ANTIBODY AND APPLICATION THEREOF" and submitted to the China National Intellectual Property Administration on December 31, 2021. The above-mentioned prior application is incorporated into the present application by reference in its entirety.

### Technical Field

The present application relates to the field of biomedicine, and specifically to a GPRC5D antibody and an application thereof.

### Background

Multiple myeloma (MM) is a plasma cell malignant tumor characterized by the unrestricted proliferation of plasma cells in the bone marrow like tumor cells, accompanied by secretion of monoclonal immunoglobulins, which causes a series of clinical manifestations such as multiple osteolytic damage, hypercalcemia, anemia, kidney damage and repeated infections. Combined treatment with hematopoietic stem cell transplantation and immunomodulators and protease inhibitors can significantly improve the prognosis. In recent years, CAR T therapy has made breakthrough progress in the treatment of multiple myeloma. In particular, CAR T targeting B cell maturation antigen (BCMA) has shown positive clinical results in the treatment of multiple myeloma. However, multiple myeloma patients who are BCMA negative or have low BCMA expression would still relapse after receiving BCMA-targeted CAR-T cell therapy. Therefore, the treatment of multiple myeloma still requires the identification of other new targets with greater potential.

GPRC5D is G protein coupled receptor C5 family subtype D, which is an orphan receptor and is a seven-transmembrane protein. GPRC5D is a new target for multiple myeloma after BCMA. It is found in tissue expression profiling studies that GPRC5D is specifically highly expressed in plasma cells of multiple myeloma, while has a low expression in normal tissues, which is limited to hair follicle regions with immune privilege. Moreover, the expressions of GPRC5D and BCMA do not overlap. For tumor recurrence models with BCMA loss, GPRC5D CAR-T still has a therapeutic effect, overcoming tumor escape. In theory, dual-targeting both GPRC5D and BCMA could cover a larger population and bring about better clinical responses. Therefore, the development of GPRC5D-specific antibodies and related biological products has huge potential clinical demand.

### Summary of the Invention

The present application discloses an antibody or an antigen-binding fragment thereof that specifically binds to G protein coupled receptor C5 family subtype D (GPRC5D), a multispecific antigen-binding molecule, a nucleic acid fragment, a vector, a host cell, an immune effector cell, a preparation method, a pharmaceutical composition, a pharmaceutical use, and a method for treating a tumor or a cancer (e.g., B cell lymphoma or multiple myeloma).

In one aspect, the present application provides an antibody or an antigen-binding fragment thereof that specifically binds to G protein coupled receptor C5 family subtype D (GPRC5D), wherein the antibody or the antigen-binding fragment thereof includes a heavy chain variable region and a light chain variable region, and wherein
(1) the light chain variable region comprises LCDR1, LCDR2 and LCDR3, the LCDR1 has any of a sequence of the following LCDR1, or a sequence with 1, 2, 3 or more amino acid insertions, deletions and/or substitutions compared with the sequence, the LCDR2 has any of a sequence of the following LCDR2, or a sequence with 1, 2, 3 or more amino acid insertions, deletions and/or substitutions compared with the sequence, and the LCDR3 has any of a sequence of the following LCDR3, or a sequence with 1, 2, 3 or more amino acid insertions, deletions and/or substitutions compared with the sequence:

| **No.** | **LCDR1** | **LCDR2** | **LCDR3** |
|---|---|---|---|
| L1 | SEQ ID NO: 35 | SEQ ID NO: 36 | SEQ ID NO: 37 |
| L2 | SEQ ID NO: 38 | SEQ ID NO: 39 | SEQ ID NO: 37 |
| L3 | SEQ ID NO: 46 | SEQ ID NO: 47 | SEQ ID NO: 48 |
| L4 | SEQ ID NO: 49 | SEQ ID NO: 50 | SEQ ID NO: 48 |
| L5 | SEQ ID NO: 57 | SEQ ID NO: 58 | SEQ ID NO: 59 |
| L6 | SEQ ID NO: 60 | SEQ ID NO: 61 | SEQ ID NO: 59 |
| L7 | SEQ ID NO: 68 | SEQ ID NO: 69 | SEQ ID NO: 70 |
| L8 | SEQ ID NO: 71 | SEQ ID NO: 72 | SEQ ID NO: 70 |
| L9 | SEQ ID NO: 79 | SEQ ID NO: 80 | SEQ ID NO: 81 |
| L10 | SEQ ID NO: 82 | SEQ ID NO: 83 | SEQ ID NO: 81 |
| L11 | SEQ ID NO: 90 | SEQ ID NO: 91 | SEQ ID NO: 92 |
| L12 | SEQ ID NO: 93 | SEQ ID NO: 94 | SEQ ID NO: 92 |
| L13 | SEQ ID NO: 101 | SEQ ID NO: 102 | SEQ ID NO: 103 |
| L14 | SEQ ID NO: 104 | SEQ ID NO: 105 | SEQ ID NO: 103 |
| L15 | SEQ ID NO: 112 | SEQ ID NO: 113 | SEQ ID NO: 114 |
| L16 | SEQ ID NO: 115 | SEQ ID NO: 116 | SEQ ID NO: 114 |
| L17 | SEQ ID NO: 217 | SEQ ID NO: 102 | SEQ ID NO: 103 |
| L18 | SEQ ID NO: 227 | SEQ ID NO: 102 | SEQ ID NO: 103 |
| L19 | SEQ ID NO: 228 | SEQ ID NO: 102 | SEQ ID NO: 103 |
| L20 | SEQ ID NO: 229 | SEQ ID NO: 102 | SEQ ID NO: 103 |
| L21 | SEQ ID NO: 230 | SEQ ID NO: 102 | SEQ ID NO: 103 |

and,
(2) the heavy chain variable region comprises HCDR1, HCDR2 and HCDR3, the HCDR1 has any of a sequence of the following HCDR1, or a sequence with 1, 2, 3 or more amino acid insertions, deletions and/or substitutions compared with the sequence, the HCDR2 has any of a sequence of the following HCDR2, or a sequence with 1, 2, 3 or more amino acid insertions, deletions and/or substitutions compared with the sequence, and the HCDR3 has any of a sequence of the following HCDR3, or a sequence with 1, 2, 3 or more amino acid insertions, deletions and/or substitutions compared with the sequence:

| **No.** | **HCDR1** | **HCDR2** | **HCDR3** |
|---|---|---|---|
| H1 | SEQ ID NO: 29 | SEQ ID NO: 30 | SEQ ID NO: 31 |
| H2 | SEQ ID NO: 32 | SEQ ID NO: 33 | SEQ ID NO: 34 |
| H3 | SEQ ID NO: 40 | SEQ ID NO: 41 | SEQ ID NO: 42 |
| H4 | SEQ ID NO: 43 | SEQ ID NO: 44 | SEQ ID NO: 45 |
| H5 | SEQ ID NO: 51 | SEQ ID NO: 52 | SEQ ID NO: 53 |
| H6 | SEQ ID NO: 54 | SEQ ID NO: 55 | SEQ ID NO: 56 |
| H7 | SEQ ID NO: 62 | SEQ ID NO: 63 | SEQ ID NO: 64 |
| H8 | SEQ ID NO: 65 | SEQ ID NO: 66 | SEQ ID NO: 67 |
| H9 | SEQ ID NO: 73 | SEQ ID NO: 74 | SEQ ID NO: 75 |
| H10 | SEQ ID NO: 76 | SEQ ID NO: 77 | SEQ ID NO: 78 |
| H11 | SEQ ID NO: 84 | SEQ ID NO: 85 | SEQ ID NO: 86 |
| H12 | SEQ ID NO: 87 | SEQ ID NO: 88 | SEQ ID NO: 89 |
| H13 | SEQ ID NO: 95 | SEQ ID NO: 96 | SEQ ID NO: 97 |
| H14 | SEQ ID NO: 98 | SEQ ID NO: 99 | SEQ ID NO: 100 |
| H15 | SEQ ID NO: 106 | SEQ ID NO: 107 | SEQ ID NO: 108 |
| H16 | SEQ ID NO: 109 | SEQ ID NO: 110 | SEQ ID NO: 111 |
| H17 | SEQ ID NO: 40 | SEQ ID NO: 142 | SEQ ID NO: 42 |
| H18 | SEQ ID NO: 40 | SEQ ID NO: 143 | SEQ ID NO: 42 |
| H19 | SEQ ID NO: 51 | SEQ ID NO: 158 | SEQ ID NO: 53 |
| H20 | SEQ ID NO: 51 | SEQ ID NO: 159 | SEQ ID NO: 53 |

In another aspect, the present application provides a multispecific antigen-binding molecule, wherein the multispecific antigen-binding molecule comprises the aforementioned antibody or the antigen-binding fragment thereof, and an additional antigen-binding molecule binding to an antigen other than GPRC5D or binding to a different GPRC5D epitope than that of the aforementioned antibody or the antigen-binding fragment thereof.

In another aspect, the present application provides a chimeric antigen receptor (CAR), wherein the chimeric antigen receptor at least includes a signal peptide, an extracellular antigen-binding domain, a hinge region, a transmembrane domain, and an intracellular signaling domain, the extracellular antigen-binding domain includes the aforementioned GPRC5D antibody or the antigen-binding fragment thereof, or the aforementioned multispecific antigen-binding molecule.

In another aspect, the present application provides an immune effector cell, wherein the immune effector cell expresses the aforementioned chimeric antigen receptor, or contains a nucleic acid fragment encoding the aforementioned chimeric antigen receptor.

In another aspect, the present application provides an isolated nucleic acid fragment, wherein the nucleic acid fragment encodes the aforementioned antibody or the antigen-binding fragment thereof, the aforementioned multispecific antigen-binding molecule, or the aforementioned chimeric antigen receptor.

In another aspect, the present application provides a vector, wherein the vector includes the aforementioned nucleic acid fragment.

In another aspect, the present application provides a host cell, wherein the host cell includes the aforementioned vector.

In another aspect, the present application provides a method for preparing the aforementioned antibody or the antigen-binding fragment thereof or the aforementioned multispecific antigen-binding molecule, wherein the method comprises culturing the aforementioned cell, and isolating the antibody, antigen-binding fragment or multispecific antigen-binding molecule expressed by the cell.

In another aspect, the present application provides a method for preparing the aforementioned immune effector cells, wherein the method comprises introducing a nucleic acid fragment encoding the aforementioned CAR into an immune effector cell.

In another aspect, the present application provides a pharmaceutical composition, wherein the pharmaceutical composition comprises the aforementioned antibody or the antigen-binding fragment thereof, the aforementioned multispecific antigen-binding molecule, the aforementioned immune effector cell, the aforementioned nucleic acid fragment, the aforementioned vector, or a product prepared according to the aforementioned method.

In another aspect, the present application provides a method for treating tumors or cancers, wherein the method comprises administering to a subject an effective amount of the aforementioned antibody or the antigen-binding fragment thereof, the aforementioned multispecific antigen-binding molecule, the aforementioned immune effector cell, the aforementioned nucleic acid fragment, the aforementioned vector, or a product prepared according to the aforementioned method or the aforementioned pharmaceutical composition; the tumor or cancer is a tumor or cancer that expresses GPRC5D.

In another aspect, the present application provides the use of the aforementioned antibody or the antigen-binding fragment thereof, the aforementioned multispecific antigen-binding molecule, the aforementioned immune effector cell, the aforementioned nucleic acid fragment, the aforementioned vector, or a product prepared according to the aforementioned method or the aforementioned pharmaceutical composition in preparing a drug for treating a tumor or a cancer; the tumor or cancer is a tumor or cancer that expresses GPRC5D.

In another aspect, the present application provides the aforementioned antibody or the antigen-binding fragment thereof, the aforementioned multispecific antigen-binding molecule, the aforementioned immune effector cell, the aforementioned nucleic acid fragment, the aforementioned vector, or a product prepared according to the aforementioned method or the aforementioned pharmaceutical composition for treating a tumor or a cancer; the tumor or cancer is a tumor or cancer that expresses GPRC5D.

In another aspect, the present application provides a kit, wherein the kit comprises the aforementioned antibody or the antigen-binding fragment thereof, the aforementioned multispecific antigen-binding molecule, the aforementioned immune effector cell, the aforementioned nucleic acid fragment, the aforementioned vector, or a product prepared according to the aforementioned method or the aforementioned pharmaceutical composition.

In another aspect, the present application provides a method for detecting GPRC5D expression in a biological sample, wherein the method comprises contacting the biological sample with the aforementioned antibody or the antigen-binding fragment thereof under conditions that allow the formation of a complex from the antibody or the antigen-binding fragment thereof and GPRC5D.

In another aspect, the present application provides the use of the aforementioned antibody or the antigen-binding fragment thereof in preparing a GPRC5D detection reagent.

BCMA negative or low expression is a common cause of relapse or refractory multiple myeloma. The present application focuses on GPRC5D, another target of relapsed/refractory multiple myeloma, and provides an antibody targeting human GPRC5D or an antigen-binding fragment thereof. The antibody or the antigen-binding fragment thereof can bind to human or monkey GPRC5D at high affinity, but does not bind or binds at low affinity to GPRC5A, GPRC5B or GPRC5C proteins, which is of great significance for the further development of antibodies (monoclonal antibodies or multispecific antibodies) and cell therapy products targeting the GPRC5D target.

### Brief Description of the Figures

Fig.1 shows FACS detection of GPRC5D expression quantity in endogenous cells.
Fig.2A shows FACS detection results of CHOK1 stable transfection cell line expressing human GPRC5A protein; Fig.2B shows FACS detection results of CHOK1 stable transfection cell line expressing human GPRC5B protein; Fig.2C shows FACS detection results of CHOK1 stable transfection cell line expressing human GPRC5C protein; Fig.2D shows FACS detection results of CHOK1 stable transfection cell line expressing human GPRC5D protein.
Fig.3 shows FACS detection results of HEK293T stable transfection cell line expressing human GPRC5D protein.
Fig.4A shows FACS detection results of CHOK1 stable transfection cell line expressing monkey GPRC5D protein; Fig.4B shows FACS detection results of HEK293T stable transfection cell line expressing monkey GPRC5D protein.
Fig.5A shows the FACS detection of the binding activity of control antibodies with NCI-H929 tumor cells; Fig.5B shows FACS detection of the binding activity of control antibodies with 293T-human-GPRC5D recombinant cells; Fig.5C shows FACS detection of the binding activity of control antibodies with 293T-monkey-GPRC5D recombinant cells.
Figs.6A-6C show Cell based ELISA detection of the binding activity of chimeric antibodies with CHOK1-hGPRC5D.
Figs.7A-7C show Cell based ELISA detection of the binding activity of chimeric antibodies with CHOK1-cynoGPRC5D.
Figs.8A-8C show Cell based ELISA detection of the binding activity of chimeric antibodies with CHOK1.
Figs.9A-9C show FACS detection of the binding activity of chimeric antibodies with NCI-H929 (high expression) cells.
Figs.10A-10C show FACS detection of the binding activity of chimeric antibodies with MolP-8 (medium expression) cells.
Figs.11A-11C show FACS detection of the binding activity of chimeric antibodies with RPMI-8226 (low expression) cells.
Figs.12A-12C show ELISA detection of the competitive binding activity of chimeric antibodies and positive control antibody 5F11.
Figs.13A-13J show Cell based ELISA detection of the binding activity of humanized antibodies with CHOK1-hGPRCSD.
Figs.14A-14J show Cell based ELISA detection of the binding activity of humanized antibodies with CHOK1-cynoGPRCSD.
Figs.15A-15J show FACS detection of the binding activity of GPRC5D humanized antibodies with NCI-H929.

### Detailed Description of the Invention

### Definition and Description of Terminology

Unless defined otherwise in the present application, scientific and technical terms related to the present application shall have the meanings understood by one of ordinary skill in the art.

In addition, unless otherwise specified herein, terms in the singular form herein shall include that in the plural form, and terms in the plural form shall include that in the singular form. More specifically, as used in the description and the appended claims, the singular forms "a/an" and "this" include plural referents, unless otherwise clearly stated.

The terms "include", "comprise" and "have" are used interchangeably herein and are intended to indicate the inclusiveness of the solution, meaning that the solution can have other elements than those listed. Furthermore, it should be understood that the description of "include", "comprise" and "have" as used herein also provides the solution of "consist of'.

The term "and/or" as used herein includes the meanings of "and", "or" and "all or any other combination of elements linked by the term".

The term GPRC5D herein refers to G protein coupled receptor C5 family subtype D, which is an orphan receptor and is a seven-transmembrane protein. GPRC5D is highly expressed on the surface of primary multiple myeloma cells, while its expression in normal tissues is limited to the hair follicle area. Studies have shown that 65% of patients with multiple myeloma have a GPRC5D expression threshold exceeding 50%. With this characteristic, GPRC5D has become a potential target for the treatment of MM.

The term "specifically bind" herein means that an antigen-binding molecule (e.g., an antibody) typically specifically binds to an antigen and substantially the same antigen with a high affinity, but does not bind to an unrelated antigen with a high affinity. The equilibrium dissociation constant KD can be measured using well-known methods in the art, such as surface plasmon resonance (e.g., Biacore) or equilibrium dialysis assay.

The term "antigen-binding molecule" is used herein in the broadest sense and refers to a molecule specifically binding to an antigen. Exemplarily, the antigen-binding molecule includes, but is not limited to, an antibody or an antibody mimetic. The "antibody mimetic" refers to an organic compound or a binding domain that can specifically bind to an antigen, but is not structurally related to an antibody. Exemplarily, the antibody mimetic includes, but is not limited to, affibody, affitin, affilin, a designed ankyrin repeat protein (DARPin), a nucleic acid aptamer or a Kunitz-type domain peptide.

The term "antibody" is used herein in the broadest sense and refers to a polypeptide or a combination of polypeptides comprising sufficient sequences from a heavy chain variable region of an immunoglobulin and/or sufficient sequences from a light chain variable region of an immunoglobulin to be able to specifically bind to an antigen. The "antibody" herein encompasses various forms and various structures as long as they exhibit the desired antigen-binding activity. The "antibody" herein includes an alternative protein scaffold or artificial scaffold with grafted complementarity determining regions (CDRs) or CDR derivatives. Such scaffolds include antibody-derived scaffolds comprising mutations introduced, e.g., to stabilize the three-dimensional structure of the antibody, and fully synthetic scaffolds comprising, e.g., biocompatible polymers. See, for example, Korndorfer et al., 2003, Proteins: Structure, Function, and Bioinformatics, 53(1): 121-129 (2003); Roque et al., Biotechnol. Prog. 20:639-654 (2004). Such scaffolds may also include non-antibody-derived scaffolds, for example, scaffold proteins known in the art which can be used for grafting CDRs, including but not limited to tenascin, fibronectin, a peptide aptamer, etc.

The term "antibody" includes an intact antibody and any antigen-binding fragment (i.e., "antigen-binding moiety") or single chain thereof. The "antibody" refers to a glycoprotein comprising at least two heavy (H) chains and two light (L) chains interconnected by a disulfide bond, or an antigen-binding moiety thereof. Each heavy chain consists of a heavy chain variable region (abbreviated herein as VH) and a heavy chain constant region. The heavy chain constant region consists of three domains, CH1, CH2 and CH3. Each light chain consists of a light chain variable region (abbreviated herein as VL) and a light chain constant region. The light chain constant region consists of one domain, CL. The VH and VL regions can be further subdivided into hypervariable regions known as complementarity determining regions (CDRs), which are interspersed among more conserved regions known as framework regions (FRs). Each VH and VL consists of three CDRs and four FRs, which are arranged in the following order from amino terminus to carboxyl terminus: FR1, CDR1, FR2, CDR2, FR3, CDR3, FR4. The variable regions of the heavy and light chains contain a binding domain that can interact with an antigen. The constant region of an antibody can mediate the binding of an immunoglobulin to a host tissue or factor, and the host tissue or factor includes various cells of the immune system (e.g., effector cells) and a first component of the classical complement system (C1q). Since the amino acid composition and arrangement sequence of the heavy chain constant region of an immunoglobulin are different, the antigenicity of the immunoglobulin is also different. Accordingly, "immunoglobulin" herein can be divided into five classes, or isotypes of immunoglobulins, namely IgM, IgD, IgG, IgA and IgE, and the corresponding heavy chains thereof are a µ chain, a δ chain, a γ chain, an α chain and an ε chain, respectively. The same class of Ig can also be divided into different subclasses according to the differences in the amino acid composition of the hinge region thereof and the number and position of heavy chain disulfide bonds. For example, IgG can be divided into IgG1, IgG2, IgG3 and IgG4, and IgA can be divided into IgA1 and IgA2. The light chains are divided into a κ chain or a λ chain by the difference in the constant region. Each Ig class of the five Ig classes can have either a κ chain or a λ chain.

"Antibody" herein also includes an antibody that does not comprise light chains, for example, heavy-chain antibodies (HCAbs) produced from camelids, such as Camelus dromedarius, Camelus bactrianus, Lama glama, Lama guanicoe and Vicugna pacos, and Ig new antigen receptors (IgNARs) found in Chondrichthyes such as sharks.

The term "antibody" herein can be derived from any animals, including but not limited to humans and non-human animals. The non-human animals can be selected from primates, mammals, rodents and vertebrates, such as camelids, Lama glama, Lama guanicoe, Vicugna pacos, goats, rabbits, mice, rats or Chondrichthyes (such as sharks).

The term "heavy chain antibody" herein refers to an antibody that lacks light chains of a conventional antibody. The term specifically includes, but is not limited to, a homodimeric antibody comprising a VH antigen-binding domain and CH2 and CH3 constant domains in the absence of a CH1 domain.

The term "nanobody" herein refers to a natural heavy chain antibody without light chains in camel, *etc.*, and cloning the variable region thereof can obtain a single domain antibody, also known as VHH (Variable domain of heavy chain of heavy chain antibody), which only consists of a heavy chain variable region, and is the smallest functional antigen-binding fragment.

The terms "VHH domain", "nanobody" and "single domain antibody" (sdAb) herein have the same meaning and are used interchangeably, and refer to a single domain antibody consisting of only one heavy chain variable region constructed by cloning the variable region of a heavy chain antibody, which is the smallest antigen-binding fragment with full functionality. Generally, a single domain antibody consisting of only one heavy chain variable region is constructed by obtaining a heavy chain antibody that naturally lacks light chains and heavy chain constant region 1 (CH1), and then cloning the heavy chain variable region of the antibody.

Further descriptions of "heavy chain antibody" and "single domain antibody", and "VHH domain" and "nanobody" can be found in Hamers-Casterman et al., Nature. 1993; 363; 446-8; Muyldermans' review article (Reviews in Molecular Biotechnology 74: 277-302, 2001); and the following patent applications mentioned as general background art: WO 94/04678, WO 95/04079 and WO 96/34103; WO 94/25591, WO 99/37681, WO 00/40968, WO 00/43507, WO 00/65057, WO 01/40310, WO 01/44301, EP 1134231 and WO 02/48193; WO 97/49805, WO 01/21817, WO 03/035694, WO 03/054016 and WO 03/055527; WO 03/050531; WO 01/90190; WO 03/025020; and WO 04/041867, WO 04/041862, WO 04/041865, WO 04/041863, WO 04/062551, WO 05/044858, WO 06/40153, WO 06/079372, WO 06/122786, WO 06/122787 and WO 06/122825, and other prior art mentioned in these applications.

The term "multispecific" herein refers to the ability of an antibody or an antigen-binding fragment thereof to bind to, for example, different antigens or at least two different epitopes on the same antigen. Therefore, the terms such as "bispecific", "trispecific" and "tetraspecific" refer to the number of different epitopes to which an antibody can bind. For example, a conventional monospecific IgG-type antibody has two identical antigen-binding sites (paratopes) and thus can only bind to the same epitope (rather than bind to different epitopes). In contrast, a multispecific antibody has at least two different types of paratopes/binding sites and thus can bind to at least two different epitopes. As described herein, "complementarity determining region" refers to an antigen-binding site of an antibody. Furthermore, single "specific" may refer to one, two, three or more identical complementarity determining regions in a single antibody (the actual number of complementarity determining regions/binding sites in a single antibody molecule is referred to as "valent"). For example, a single natural IgG antibody is monospecific and bivalent because it has two identical paratopes. Accordingly, a multispecific antibody comprises at least two (different) complementarity determining regions/binding sites. Therefore, the term "multispecific antibody" refers to an antibody that has more than one paratope and has the ability to bind to two or more different epitopes. The term "multispecific antibody" particularly includes the bispecific antibody as defined above, and generally also includes a protein, e.g., an antibody or a scaffold specifically binding to three or more different epitopes, i.e., an antibody having three or more paratopes/binding sites.

The term "valence" herein refers to the presence of a defined number of binding sites in an antibody/an antigen-binding molecule. Therefore, the terms "monovalent", "bivalent", "tetravalent" and "hexavalent" indicate the presence of one binding site, two binding sites, four binding sites and six binding sites in an antibody/antigen-binding molecule, respectively.

The "full-length antibody", "complete antibody" and "intact antibody" are used interchangeably herein and mean that they have a structure substantially similar to that of a natural antibody.

The "antigen-binding fragment" and "antibody fragment" are used interchangeably herein, which do not possess the full structure of an intact antibody, and only comprise a part of an intact antibody or a variant of the part, wherein the part of the intact antibody or the variant of the part has the ability to bind to an antigen. Exemplarily, the "antigen-binding fragment" or "antibody fragment" herein includes, but is not limited to, Fab, F(ab')2, Fab', Fab'-SH, Fd, Fv, scFv, diabody and a single domain antibody.

The term "chimeric antibody" herein refers to an antibody that has a variable sequence of an immunoglobulin from a source organism (such as a rat, a mouse, a rabbit or a llama) and a constant region of an immunoglobulin from a different organism (such as human). Methods for producing a chimeric antibody are known in the art. See, for example, Morrison, 1985, Science 229(4719) :1202-7; Oi et al., 1986, Bio Techniques 4:214-221; and Gillies et al., 1985 J Immunol Methods 125:191-202; which are incorporated herein by reference.

The term "humanized antibody" herein refers to a non-human antibody that has been genetically engineered, with amino acid sequences modified to improve the homology with the sequences of a human antibody. Generally speaking, all or part of the CDR regions of a humanized antibody come from a non-human antibody (a donor antibody), and all or part of the non-CDR regions (for example, a variable region FR and/or a constant region) come from a human immunoglobulin (a receptor antibody). A humanized antibody usually retains or partially retains the expected properties of a donor antibody, including but not limited to, antigen specificity, affinity, reactivity, ability to enhance immune cell activity or ability to enhance immune response, etc.

The term "fully human antibody" herein refers to an antibody having variable regions in which both the FRs and CDRs are derived from human germline immunoglobulin sequences. Furthermore, if the antibody comprises a constant region, the constant region also is derived from human germline immunoglobulin sequences. The "fully human antibody" herein may include amino acid residues not encoded by human germline immunoglobulin sequences (for example, mutations introduced by random or site-specific mutagenesis in vitro or by somatic mutation in vivo). However, the "fully human antibody" herein does not include an antibody in which CDR sequences derived from the germline of another mammalian species (e.g., a mouse) have been grafted onto human framework sequences.

The term "variable region" herein refers to a region in the heavy or light chain of an antibody that is involved in enabling the antibody to bind to an antigen. "Heavy chain variable region", "VH" and "HCVR" are used interchangeably, and "light chain variable region", "VL" and "LCVR" are used interchangeably. The variable domains of the heavy and light chains of a natural antibody generally have similar structures, and each domain comprises four conserved framework regions (FRs) and three hypervariable regions (HVRs). See, for example, Kindt et al., Kuby Immunology, 6th ed., W.H. Freeman and Co., p. 91 (2007). A single VH or VL domain may be sufficient to confer antigen binding specificity.

The terms "complementarity determining region" and "CDR" are used interchangeably herein and generally refer to hypervariable regions (HVRs) found in both light and heavy chain variable domains. The more conservative portion of a variable domain is called the framework region (FR). As understood in the art, the amino acid positions representing the hypervariable region of an antibody may vary according to the context and various definitions known in the art. Some positions within variable domains can be considered heterozygous hypervariable positions because these positions can be considered to be within the hypervariable regions under one set of criteria (such as IMGT or KABAT) but outside the hypervariable regions under a different set of criteria (such as KABAT or IMGT). One or more of these positions may also be found in extended hypervariable regions. The present application includes an antibody comprising modifications in these heterozygous hypervariable positions. The heavy chain variable region CDR can be abbreviated as HCDR, and the light chain variable region CDR can be abbreviated as LCDR. The variable domains of a natural heavy chain and light chain each comprise four framework regions predominantly in a sheet configuration, which are linked by three CDRs (CDR1, CDR2 and CDR3) that form a loop linking the sheet structure, and in some cases form part of the sheet structure. The CDRs in each chain are closely held together in order of FR1-CDR1-FR2-CDR2-FR3-CDR3-FR4 by the FR region, and together with the CDRs from other antibody chains, contribute to the formation of an antigen-binding site of an antibody (see Kabat et al., Sequences of Proteins of Immunological Interest, National Institute of Health, Bethesda, Md. 1987; which is incorporated herein by reference).

For a further description of CDR, with reference to Kabat et al., J. Biol. Chem., 252:6609-6616 (1977); Kabat et al., United States Department of Health and Human Services, "Sequences of proteins of immunological interest" (1991); Chothia et al., J. Mol. Biol. 196:901-917 (1987); Al-Lazikani B. et al., J. Mol. Biol., 273: 927-948 (1997); MacCallum et al., J. Mol. Biol. 262:732-745 (1996); Abhinandan and Martin, Mol. Immunol., 45: 3832-3839 (2008); Lefranc M.P. et al., Dev. Comp. Immunol., 27: 55-77 (2003); and Honegger and Plückthun, J. Mol. Biol., 309:657-670 (2001). The "CDRs" herein can be marked and defined by well-known methods in the art, including but not limited to Kabat numbering system, Chothia numbering system or IMGT numbering system. The tool websites used include, but are not limited to, AbRSA website (http://cao.labshare.cn/AbRSA/cdrs.php), abYsis website (www.abysis.org/abysis/sequence_input/key_annotation/key_annotation.cgi) and IMGT website (http://www.imgt.org/3Dstructure-DB/cgi/DomainGapAlign.cgi#results). The CDRs herein include overlaps and subsets of amino acid residues defined in different ways.

The term "Kabat numbering system" herein generally refers to the immunoglobulin alignment and numbering system proposed by Elvin A. Kabat (see, for example, Kabat et al, Sequences of Proteins of Immunological Interest, 5th Ed. Public Health Service, National Institutes of Health, Bethesda, Md., 1991).

The term "Chothia numbering system" herein generally refers to the immunoglobulin numbering system proposed by Chothia *et al.,* which is a classical rule for identifying the boundaries of CDR regions based on the location of structural loop regions (see, for example, Chothia & Lesk (1987) J. Mol. Biol. 196:901-917; Chothia et al. (1989) Nature 342:878-883).

The term "IMGT numbering system" herein generally refers to the numbering system based on The International ImMunoGeneTics Information System (IMGT) proposed by Lefranc *et al.,* see Lefranc et al., Dev. Comparat. Immunol. 27:55-77, 2003.

The term "heavy chain constant region" herein refers to the carboxyl terminus portion of an antibody heavy chain, which is not directly involved in the binding of an antibody to an antigen, but exhibits an effector function, such as an interaction with an Fc receptor, and has a more conservative amino acid sequence relative to the variable domain of an antibody. The "heavy chain constant region" can be selected from a CH1 domain, a hinge region, a CH2 domain, a CH3 domain, or a variant or fragment thereof. The "heavy chain constant region" includes a "full-length heavy chain constant region" and a "heavy chain constant region fragment", the former has a structure substantially similar to that of a natural antibody constant region, while the latter only includes "a portion of the full-length heavy chain constant region". Exemplarily, a typical "full-length antibody heavy chain constant region" consists of CH1 domain-hinge region-CH2 domain-CH3 domain, which also includes a CH4 domain when an IgE antibody is referred to, and does not include the CH1 domain when a heavy chain antibody is referred to. Exemplarily, a typical "heavy chain constant region fragment" can be selected from Fc or a CH3 domain.

The term "light chain constant region" herein refers to the carboxyl terminus portion of an antibody light chain, which is not directly involved in the binding of an antibody to an antigen, and the light chain constant region can be selected from a constant κ domain or a constant λ domain.

The term "Fc region" herein is used to define the C-terminal region of an antibody heavy chain that contains at least a portion of the constant region. The term includes a native sequence Fc region and a variant Fc region. Exemplarily, human IgG heavy chain Fc region can extend from Cys226 or Pro230 to the carboxyl terminus of the heavy chain. However, an antibody generated from a host cell may undergo post-translational cleavage whereby one or more, particularly one or two amino acids are cleaved off from the C-terminus of the heavy chain. Therefore, by expression of a specific nucleic acid molecule encoding a full-length heavy chain, the antibody generated from a host cell can include the full-length heavy chain, or a cleavage variant of the full-length heavy chain. This may be the case when the last two C-terminal amino acids of the heavy chain are glycine (G446) and lysine (K447, numbering according to the Kabat EU index). Therefore, the C-terminal lysine (Lys447), or the C-terminal glycine (Gly446) and lysine (Lys447) of the Fc region may or may not be present. Typically, an IgG Fc region comprises IgG CH2 and IgG CH3 domains, and optionally, on this basis, the IgG Fc region can also comprise a complete or partial hinge region, but does not comprise a CH1 domain. The "CH2 domain" of a human IgG Fc region generally extends from an amino acid residue at about position 231 to an amino acid residue at about position 340. In one embodiment, a carbohydrate chain is attached to the CH2 domain. The CH2 domain herein can be a native sequence CH2 domain or a variant CH2 domain. The "CH3 domain" comprises the stretch of residues at the C-terminus of the CH2 domain in the Fc region (i.e., from an amino acid residue at about position 341 to an amino acid residue at about position 447 of IgG). The CH3 region herein can be a native sequence CH3 domain or a variant CH3 domain (e.g., a CH3 domain having a "knob" introduced in one chain thereof and a "hole" correspondingly introduced in the other chain thereof; see US Patent No. 5,821,333, which is expressly incorporated herein by reference). As described herein, such variant CH3 domains can be used to facilitate heterodimerization of two non-identical antibody heavy chains.

Unless otherwise specified herein, the numbering of amino acid residues in an Fc region or a constant region is according to the EU numbering system, also known as the EU index, as described in Kabat et al., Sequences of Proteins of Immunological Interest,5th Ed. Public Health Service, National Institutes of Health, Bethesda, MD, 1991.

The term "Fc variant" herein refers to changes in the structure or function of Fc caused by one or more amino acid substitution, insertion or deletion mutations at appropriate positions on the Fc. The "interaction between Fc variants" refers to the space-filling effect, electrostatic steering, hydrogen bonding effect, hydrophobic effect, etc. formed between Fc variants that have been subjected to a mutation design. The interaction between Fc variants contributes to the formation of a stable heterodimeric protein. A preferred mutation design is a mutation design in a "Knob-into-Hole" form.

The mutation design technique of Fc variants has been widely used in the field to prepare bispecific antibodies or heterodimeric Fc fusion protein forms. Representative ones include the "Knob-into-Hole" form proposed by Cater et al. (Protein Engineering vol. 9 no. 7 pp. 617-621, 1996); an Fc-containing heterodimer form formed by using Electrostatic Steering by the technician in Amgen (US 20100286374 A1); The heterodimer form (SEEDbodies) formed by IgG/Ig chain exchange proposed by Jonathan H. Davis et al. (Protein Engineering, Design & Selection pp. 1-8, 2010); Bispecific molecules formed by Genmab's DuoBody (Science, 2007. 317 (5844)) platform technology; heterodimeric protein forms formed by combining structural calculation and Fc amino acid mutations and combining different modes of action by Xencor's technical staff (mAbs 3: 6, 546-557; November/December 2011); a heterodimeric protein form obtained by the charge network-based Fc modification method of Alphamab Oncology (CN 201110459100.7); and other genetic engineering methods for forming heterodimeric functional proteins based on Fc amino acid changes or functional modification means. The Knob/Hole structure on the Fc variant fragments of the present application means that the two Fc fragments are mutated respectively, and can be combined in a "Knob-into-hole" form after the mutations. Preferably, the Fc regions are subjected to site mutation modification using the "Knob-into-hole" model of Cater et al., so that the obtained first Fc variant and second Fc variant can be combined together in a "Knob-into-hole" form to form a heterodimer. The selection of particular immunoglobulin Fc regions from particular immunoglobulin classes and subclasses is within the range of those skilled in the art. The Fc regions of human antibodies IgG1, IgG2, IgG3 and IgG4 are preferred, and the Fc region of human antibody IgG1 is more preferred. Either of the first Fc variant or the second Fc variant is randomly selected for knob mutation and the other for hole mutation.

The term "conservative amino acid" herein generally refers to amino acids that belong to the same class or have similar characteristics (such as charge, side chain size, hydrophobicity, hydrophilicity, backbone conformation and rigidity). Exemplarily, the following amino acids in each group are each other's conservative amino acid residues, and a substitution of amino acid residues in the group is a substitution of conservative amino acids:
1) Alanine (A), Serine (S) and Threonine (T);
2) Aspartic acid (D) and Glutamic acid (E);
3) Asparagine (N) and Glutamine (Q);
4) Arginine (R), Lysine (K) and Histidine (H);
5) Isoleucine (I), Leucine (L), Methionine (M) and Valine (V); and
6) Phenylalanine (F), Tyrosine (Y) and Tryptophan (W).

The term "identity" herein can be calculated by the following method: To determine the percent "identity" of two amino acid sequences or two nucleic acid sequences, the sequences are aligned for optimal comparison purposes (for example, gaps can be introduced in either or both of the first and second amino acid sequences or nucleic acid sequences for optimal alignment or non-homologous sequences can be discarded for comparison purposes). The amino acid residues or nucleotides at corresponding amino acid positions or nucleotide positions are then compared. When the position in the first sequence is occupied by the same amino acid residue or nucleotide as that at the corresponding position in the second sequence, the molecules are identical at that position. The percent identity between two sequences will vary with the identical positions shared by the sequences, considering the number of gaps that need to be introduced to optimally align the two sequences and the length of each gap.

The sequence comparison and the calculation of percent identity between two sequences can be achieved using a mathematical algorithm. For example, the percent identity between two amino acid sequences is determined using the Needleman and Wunsch algorithm ((1970) J. Mol. Biol. 48:444-453) in the GAP program that has been integrated into the GCG software package (available at www.gcg.com) and using the Blossum 62 matrix or the PAM250 matrix, with a gap weight of 16, 14, 12, 10, 8, 6 or 4 and a length weight of 1, 2, 3, 4, 5 or 6. For another example, the percent identity between two nucleotide sequences is determined using the GAP program in the GCG software package (available at www.gcg.com) and using the NWSgapdna.CMP matrix, with a gap weight of 40, 50, 60, 70, or 80 and a length weight of 1, 2, 3, 4, 5, or 6. A particularly preferred parameter set (and one parameter set that should be used unless otherwise stated) is the Blossum62 scoring matrix with a gap penalty of 12, a gap extension penalty of 4, and a gap frameshift penalty of 5. The percent identity between two amino acid sequences or nucleotide sequences can also be determined using the PAM120 weighted remainder table, with a gap length penalty of 12 and a gap penalty of 4, and using the E. Meyers and W. Miller algorithm ((1989) CABIOS, 4:11-17) that has been incorporated into the ALIGN program (version 2.0).

Additionally or alternatively, the nucleic acid and protein sequences of the present application can further be used as "query sequences" to perform searches against public databases, e.g., to identify other family member sequences or related sequences. For example, the NBLAST and XBLAST programs (version 2.0) of Altschul et al., (1990) J. Mol. Biol. 215:403-10 can be used to perform such searches. BLAST nucleotide searches can be performed with the NBLAST program, with score = 100 and word length = 12, to obtain the nucleotide sequences homologous to the nucleic acid molecules of the present application. BLAST protein searches can be performed with the XBLAST program, with score = 50 and word length = 3, to obtain the amino acid sequences homologous to the protein molecule of the present application. To obtain gapped alignment results for comparison purposes, gapped BLAST can be used as described in Altschul et al., (1997) Nucleic Acids Res. 25:3389-3402. When BLAST and gapped BLAST programs are used, the default parameters of the corresponding programs (e.g., XBLAST and NBLAST) can be used. See www.ncbi.nlm.nih.gov.

The term "chimeric antigen receptor (CAR)" herein refers to an artificial cell surface receptor engineered to express on immune effector cells and specifically bind to antigens, comprising at least (1) an extracellular antigen-binding domain, such as a heavy chain variable region and/or a light chain variable region of an antibody, (2) a transmembrane domain anchoring the CAR into an immune effector cell, and (3) an intracellular signaling domain. The CAR can redirect T cells and other immune effector cells to selected targets, such as cancer cells, in a non-MHC-restricted way by using an extracellular antigen-binding domain.

The term "nucleic acid" herein includes any compound and/or substance comprising a polymer of nucleotides. Each nucleotide consists of a base, specifically a purine or pyrimidine base (i.e., cytosine (C), guanine (G), adenine (A), thymine (T) or uracil (U)), a sugar (i.e., deoxyribose or ribose) and a phosphate group. Generally, a nucleic acid molecule is described by a sequence of bases, whereby the bases represent the primary structure (linear structure) of the nucleic acid molecule. The sequence of bases is usually expressed as 5' to 3'. In this context, the term nucleic acid molecule encompasses deoxyribonucleic acid (DNA), including for example complementary DNA (cDNA) and genomic DNA, ribonucleic acid (RNA), especially messenger RNA (mRNA), synthetic forms of DNA or RNA, and a polymer comprising a mixture of two or more of these molecules. The nucleic acid molecule can be linear or circular. Furthermore, the term nucleic acid molecule includes both sense strand and antisense strand, as well as single-stranded form and double-stranded form. Furthermore, the nucleic acid molecules described herein may contain naturally occurring or non-naturally occurring nucleotides. Examples of non-naturally occurring nucleotides include modified nucleotide bases with derivatized sugar or phosphate backbone linkages or chemically modified residues. The nucleic acid molecule also encompasses DNA and RNA molecules which are suitable as vectors for direct expression of the antibody of the present application *in vitro* and/or *in vivo,* for example in a host or patient. Such DNA (e.g., cDNA) or RNA (e.g., mRNA) vectors may be unmodified or modified. For example, mRNA can be chemically modified to enhance the stability of the RNA vector and/or the expression of the encoded molecule, so that the mRNA can be injected into a subject to generate an antibody in vivo (see, for example, Stadler et al., Nature Medicine 2017, Published online June 12, 2017, doi: 10.1038/nm.4356 or EP 2101823 B1).

The "isolated" nucleic acid herein refers to a nucleic acid molecule that has been separated from components of the natural environment thereof. The isolated nucleic acid includes a nucleic acid molecule contained in a cell that normally contains the nucleic acid molecule, but which is present extrachromosomally or at a chromosomal location other than the natural chromosomal location thereof.

The term "vector" herein refers to a nucleic acid molecule capable of amplifying another nucleic acid to which it is linked. The term includes a vector having a self-replicating nucleic acid structure and a vector that is integrated into the genome of a host cell into which the vector has been introduced. Certain vectors can direct the expression of the nucleic acid to which they are operably linked. Such vectors are referred to herein as "expression vectors".

The term "host cell" herein refers to a cell into which a foreign nucleic acid is introduced, including the progenies of such a cell. The host cell includes "transformant" and "transformed cell" which include the primary transformed cell and progeny derived therefrom, regardless of the number of passages. The progeny may not be identical to the parental cell in nucleic acid content, and may contain a mutation. The mutant progeny with the same function or biological activity as those screened or selected in the initially transformed cells are included herein.

As used herein, the term "pharmaceutical composition" refers to a preparation that is present in a form which allows the active ingredients contained therein to be biologically effective and does not contain additional ingredients that would be unacceptably toxic to the subject to which the pharmaceutical composition is administered.

The term "pharmaceutically acceptable carrier" herein includes any and all solvents, dispersion media, coating materials, surfactants, antioxidants, preservatives (e.g., antibacterial agents and antifungal agents), isotonic agents, absorption delaying agents, salts, preservatives, drug stabilizers, binders, excipients, disintegrants, lubricants, sweeteners, flavoring agents, dyes, *etc.,* and a combination thereof, which are known to those skilled in the art (see, for example, Remington's Pharmaceutical Sciences, 18th edition. MackPrinting Company, 1990, pages 1289-1329). Except in cases of incompatibility with the active ingredient, any conventional carrier is contemplated for use in a therapeutic or pharmaceutical composition.

The term "treatment" herein refers to surgical or therapeutic treatment, the purpose of which is to prevent and slow down (reduce) an undesired physiological change or pathology in a subject being treated, such as cancers and tumors. Beneficial or desired clinical outcomes include, but are not limited to, the alleviation of symptoms, the weakening of disease severity, the stabilization of disease state (i.e., no deterioration), the delay or slowing down of disease progression, the amelioration or mitigation of disease state, and remission (whether partial or complete), whether detectable or undetectable. Objects in need of treatment include those who already have a disorder or disease, those who are susceptible to a disorder or disease or those who intend to prevent a disorder or disease. When terms such as slowing down, alleviation, weakening, mitigation, and remission are referred to, their meanings also include elimination, disappearance, non-occurrence and other circumstances.

The term "subject" refers to an organism receiving treatment for a particular disease or condition as described herein. Exemplarily, a "subject" includes a mammal, such as a human, a primate (e.g., monkey) or a non-primate mammal, receiving treatment for a disease or a condition.

The term "effective amount" herein refers to an amount of a therapeutic agent effective to prevent or relieve a disease or a disorder or the progression of the disease when administered alone or in combination with another therapeutic agent to a cell, tissue or subject. The "effective amount" also refers to an amount of a compound sufficient to relieve symptoms, such as treating, curing, preventing or relieving related medical disorders, or increasing the speed of treating, curing, preventing or relieving these disorders. When the active ingredient is administered to an individual alone, a therapeutically effective dose refers to the ingredient alone. When a certain combination is applied, a therapeutically effective dose refers to the combined dosage of the active ingredients that produce a therapeutic effect, whether administered in combination, sequentially or simultaneously.

The term "cancer" herein refers to or describes the physiological condition in mammals that is typically characterized by unregulated cell growth. Both benign and malignant cancers are included in this definition. The term "tumor" or "neoplasm" herein refers to all neoplastic cell growth and proliferation, whether malignant or benign, and to all pre-cancerous and cancerous cells and tissues. The terms "cancer" and "tumor" are not mutually exclusive when referred to herein.

The term "EC50" herein refers to the half-maximal effective concentration, which includes the concentration of an antibody that induces a response halfway between baseline and maximum after a specified exposure time. EC50 essentially represents the concentration of an antibody at which 50% of the maximal effect thereof is observed and which can be measured by methods known in the art.

### Detailed Description of Embodiments

In a first aspect, the present application provides an antibody or an antigen-binding fragment thereof that specifically binds to G protein coupled receptor C5 family subtype D (GPRC5D), wherein the antibody or the antigen-binding fragment thereof includes a heavy chain variable region and a light chain variable region, and wherein
(1) the light chain variable region comprises LCDR1, LCDR2 and LCDR3, the LCDR1 has any of a sequence of the following LCDR1, or a sequence with 1, 2, 3 or more amino acid insertions, deletions and/or substitutions compared with the sequence, the LCDR2 has any of a sequence of the following LCDR2, or a sequence with 1, 2, 3 or more amino acid insertions, deletions and/or substitutions compared with the sequence, and the LCDR3 has any of a sequence of the following LCDR3, or a sequence with 1, 2, 3 or more amino acid insertions, deletions and/or substitutions compared with the sequence:

| **No.** | **LCDR1** | **LCDR2** | **LCDR3** |
|---|---|---|---|
| L1 | SEQ ID NO: 35 | SEQ ID NO: 36 | SEQ ID NO: 37 |
| L2 | SEQ ID NO: 38 | SEQ ID NO: 39 | SEQ ID NO: 37 |
| L3 | SEQ ID NO: 46 | SEQ ID NO: 47 | SEQ ID NO: 48 |
| L4 | SEQ ID NO: 49 | SEQ ID NO: 50 | SEQ ID NO: 48 |
| L5 | SEQ ID NO: 57 | SEQ ID NO: 58 | SEQ ID NO: 59 |
| L6 | SEQ ID NO: 60 | SEQ ID NO: 61 | SEQ ID NO: 59 |
| L7 | SEQ ID NO: 68 | SEQ ID NO: 69 | SEQ ID NO: 70 |
| L8 | SEQ ID NO: 71 | SEQ ID NO: 72 | SEQ ID NO: 70 |
| L9 | SEQ ID NO: 79 | SEQ ID NO: 80 | SEQ ID NO: 81 |
| L10 | SEQ ID NO: 82 | SEQ ID NO: 83 | SEQ ID NO: 81 |
| L11 | SEQ ID NO: 90 | SEQ ID NO: 91 | SEQ ID NO: 92 |
| L12 | SEQ ID NO: 93 | SEQ ID NO: 94 | SEQ ID NO: 92 |
| L13 | SEQ ID NO: 101 | SEQ ID NO: 102 | SEQ ID NO: 103 |
| L14 | SEQ ID NO: 104 | SEQ ID NO: 105 | SEQ ID NO: 103 |
| L15 | SEQ ID NO: 112 | SEQ ID NO: 113 | SEQ ID NO: 114 |
| L16 | SEQ ID NO: 115 | SEQ ID NO: 116 | SEQ ID NO: 114 |
| L17 | SEQ ID NO: 217 | SEQ ID NO: 102 | SEQ ID NO: 103 |
| L18 | SEQ ID NO: 227 | SEQ ID NO: 102 | SEQ ID NO: 103 |
| L19 | SEQ ID NO: 228 | SEQ ID NO: 102 | SEQ ID NO: 103 |
| L20 | SEQ ID NO: 229 | SEQ ID NO: 102 | SEQ ID NO: 103 |
| L21 | SEQ ID NO: 230 | SEQ ID NO: 102 | SEQ ID NO: 103 |

and,
(2) the heavy chain variable region comprises HCDR1, HCDR2 and HCDR3, the HCDR1 has any of a sequence of the following HCDR1, or a sequence with 1, 2, 3 or more amino acid insertions, deletions and/or substitutions compared with the sequence, the HCDR2 has any of a sequence of the following HCDR2, or a sequence with 1, 2, 3 or more amino acid insertions, deletions and/or substitutions compared with the sequence, and the HCDR3 has any of a sequence of the following HCDR3, or a sequence with 1, 2, 3 or more amino acid insertions, deletions and/or substitutions compared with the sequence:

| **No.** | **HCDR1** | **HCDR2** | **HCDR3** |
|---|---|---|---|
| H1 | SEQ ID NO: 29 | SEQ ID NO: 30 | SEQ ID NO: 31 |
| H2 | SEQ ID NO: 32 | SEQ ID NO: 33 | SEQ ID NO: 34 |
| H3 | SEQ ID NO: 40 | SEQ ID NO: 41 | SEQ ID NO: 42 |
| H4 | SEQ ID NO: 43 | SEQ ID NO: 44 | SEQ ID NO: 45 |
| H5 | SEQ ID NO: 51 | SEQ ID NO: 52 | SEQ ID NO: 53 |
| H6 | SEQ ID NO: 54 | SEQ ID NO: 55 | SEQ ID NO: 56 |
| H7 | SEQ ID NO: 62 | SEQ ID NO: 63 | SEQ ID NO: 64 |
| H8 | SEQ ID NO: 65 | SEQ ID NO: 66 | SEQ ID NO: 67 |
| H9 | SEQ ID NO: 73 | SEQ ID NO: 74 | SEQ ID NO: 75 |
| H10 | SEQ ID NO: 76 | SEQ ID NO: 77 | SEQ ID NO: 78 |
| H11 | SEQ ID NO: 84 | SEQ ID NO: 85 | SEQ ID NO: 86 |
| H12 | SEQ ID NO: 87 | SEQ ID NO: 88 | SEQ ID NO: 89 |
| H13 | SEQ ID NO: 95 | SEQ ID NO: 96 | SEQ ID NO: 97 |
| H14 | SEQ ID NO: 98 | SEQ ID NO: 99 | SEQ ID NO: 100 |
| H15 | SEQ ID NO: 106 | SEQ ID NO: 107 | SEQ ID NO: 108 |
| H16 | SEQ ID NO: 109 | SEQ ID NO: 110 | SEQ ID NO: 111 |
| H17 | SEQ ID NO: 40 | SEQ ID NO: 142 | SEQ ID NO: 42 |
| H18 | SEQ ID NO: 40 | SEQ ID NO: 143 | SEQ ID NO: 42 |
| H19 | SEQ ID NO: 51 | SEQ ID NO: 158 | SEQ ID NO: 53 |
| H20 | SEQ ID NO: 51 | SEQ ID NO: 159 | SEQ ID NO: 53 |

In a preferred embodiment, the antibody or the antigen-binding fragment thereof comprises the sequences of six CDRs in the following combination of heavy chain variable region and light chain variable region: L1+H1, L2+H2, L3+H3, L4+H4, L5+H5, L6+H6, L7+H7, L8+H8, L9+H9, L10+H10, L11+H11, L12+H12, L13+ H13, L14+H14, L15+H15, L16+H16, L3+H17, L3+H18, L5+H19, L5+H20, L17+H13, L18+H13, L19+H13, L20+H13 or L21+H13, or sequences of six CDRs having 1, 2, 3 or more amino acid insertions, deletions and/or substitutions compared with the sequences of the six CDRs.

In a particular embodiment, the present application provides an antibody or an antigen-binding fragment thereof, wherein:
(1) the light chain variable region sequence comprises a sequence as shown in any one of SEQ ID NOs: 14, 16, 18, 20, 22, 24, 26, 28, 118-120, 130-131, 144-145, 160-163, 172-175, 184-187, 196-197, 206-208, 218-221 and 231-233, or a sequence having 80%, 85%, 90%, 95%, 96%, 97%, 98%, 99% or higher identity to the sequence;
   and,
(2) the heavy chain variable region sequence comprises a sequence as shown in any one of SEQ ID NOs: 13, 15, 17, 19, 21, 23, 25, 27, 121-125, 132-137, 146-152, 164-166, 176-178, 188-190, 198-201, 209-212, 222 and 234-238, or a sequence having 80%, 85%, 90%, 95%, 96%, 97%, 98%, 99% or higher identity to the sequence.

In a preferred embodiment, the antibody or the antigen-binding fragment thereof has a light chain variable region and a heavy chain variable region as follows:
(1) the light chain variable region and the heavy chain variable region comprise sequences as shown in SEQ ID NO: 14 and SEQ ID NO: 13 respectively;
(2) the light chain variable region and the heavy chain variable region comprise sequences as shown in SEQ ID NO: 16 and SEQ ID NO: 15 respectively;
(3) the light chain variable region and the heavy chain variable region comprise sequences as shown in SEQ ID NO: 18 and SEQ ID NO: 17 respectively;
(4) the light chain variable region and the heavy chain variable region comprise sequences as shown in SEQ ID NO: 20 and SEQ ID NO: 19 respectively;
(5) the light chain variable region and the heavy chain variable region comprise sequences as shown in SEQ ID NO: 22 and SEQ ID NO: 21 respectively;
(6) the light chain variable region and the heavy chain variable region comprise sequences as shown in SEQ ID NO: 24 and SEQ ID NO: 23 respectively;
(7) the light chain variable region and the heavy chain variable region comprise sequences as shown in SEQ ID NO: 26 and SEQ ID NO: 25 respectively;
(8) the light chain variable region and the heavy chain variable region comprise sequences as shown in SEQ ID NO: 28 and SEQ ID NO: 27 respectively;
(9) the light chain variable region comprises a sequence as shown in any one of SEQ ID NOs: 118-120, and the heavy chain variable region comprises a sequence as shown in any one of SEQ ID NOs: 121-125;
(10) the light chain variable region comprises a sequence as shown in any one of SEQ ID NOs: 130-131, and the heavy chain variable region comprises a sequence as shown in any one of SEQ ID NOs: 132-137;
(11) the light chain variable region comprises a sequence as shown in any one of SEQ ID NOs: 144-145, and the heavy chain variable region comprises a sequence as shown in any one of SEQ ID NOs: 146-152;
(12) the light chain variable region comprises a sequence as shown in any one of SEQ ID NOs: 160-163, and the heavy chain variable region comprises a sequence as shown in any one of SEQ ID NOs: 164-166;
(13) the light chain variable region comprises a sequence as shown in any one of SEQ ID NOs: 172-175, and the heavy chain variable region comprises a sequence as shown in any one of SEQ ID NOs: 176-178;
(14) the light chain variable region comprises a sequence as shown in any one of SEQ ID NOs: 184-187, and the heavy chain variable region comprises a sequence as shown in any one of SEQ ID NOs: 188-190;
(15) the light chain variable region comprises a sequence as shown in any one of SEQ ID NOs: 196-197, and the heavy chain variable region comprises a sequence as shown in any one of SEQ ID NOs: 198-201;
(16) the light chain variable region comprises a sequence as shown in any one of SEQ ID NOs: 206-208, and the heavy chain variable region comprises a sequence as shown in any one of SEQ ID NOs: 209-212;
(17) the light chain variable region comprises a sequence as shown in any one of SEQ ID NOs: 218-221, and the heavy chain variable region comprises a sequence as shown in any one of SEQ ID NO: 222;
(18) the light chain variable region comprises a sequence as shown in any one of SEQ ID NOs: 231-233, and the heavy chain variable region comprises a sequence as shown in any one of SEQ ID NOs: 234-238;
or (19) the light chain variable region comprises a sequence having 80%, 85%, 90%, 95%, 96%, 97 %, 98%, 99% or higher identity to the light chain variable region as shown in any one of the above (1) to (18), and the heavy chain variable region comprises a sequence having 80%, 85%, 90%, 95%, 96%, 97 %, 98%, 99% or higher identity to the heavy chain variable region as shown in any one of the above (1) to (18).

In a particular embodiment, the antibody or the antigen-binding fragment thereof is chimeric, humanized or fully human.

In a particular embodiment, the antibody or the antigen-binding fragment thereof is capable of binding to human or monkey GPRC5D.

In a particular embodiment, the antibody or the antigen-binding fragment thereof comprises a constant region sequence of any one of human or murine antibody IgG1, IgG2, IgG3, IgG4, IgA, IgM, IgE or IgD; preferably, it comprises a constant region sequence of human or murine antibody IgG1, IgG2, IgG3 or IgG4, or comprises a sequence having 80%, 85%, 90%, 95%, 96%, 97%, 98%, 99% or higher identity to the constant region sequence of human or murine antibody IgG1, IgG2, IgG3 or IgG4. Furthermore, the antibody or the antigen-binding fragment thereof is further coupled with a therapeutic agent or a tracer; preferably, the therapeutic agent is selected from a radioisotope, a chemotherapeutic drug or an immunomodulator, and the tracer is selected from a radiological contrast agent, a paramagnetic ion, a metal, a fluorescent tag, a chemiluminescence tag, an ultrasonic contrast agent and a photosensitizer; more preferably, the cytotoxic agent is selected from alkaloids, methotrexate, anthracycline antibiotics (doxorubicin), taxanes, pyrrolobenzodiazepine (PBD) or toxin compounds.

In a particular embodiment, the antigen-binding fragment is selected from one or more of F(ab')₂, Fab', Fab, Fv, scFv, nanobody or affibody.

In a second aspect, the present application further provides a multispecific antigen-binding molecule, wherein the multispecific antigen-binding molecule comprises the aforementioned antibody or the antigen-binding fragment thereof, and an additional antigen-binding molecule binding to an antigen other than GPRC5D or binding to a different GPRC5D epitope than that of the aforementioned antibody or the antigen-binding fragment thereof.

Preferably, the additional antigen-binding molecule is an antibody or an antigen-binding fragment thereof.

Preferably, the multispecific antigen-binding molecule can be bispecific, trispecific or tetraspecific.

Preferably, the multispecific antigen-binding molecule can be bivalent, trivalent, tetravalent, pentavalent or hexavalent.

In a third aspect, the present application further provides a chimeric antigen receptor (CAR), wherein the chimeric antigen receptor at least comprises a signal peptide, an extracellular antigen-binding domain, a hinge region, a transmembrane domain, and an intracellular signaling domain, the extracellular antigen-binding domain comprises the GPRC5D antibody or the antigen-binding fragment thereof of any one of the aforementioned aspects, or the aforementioned multispecific antigen-binding molecule.

In a fourth aspect, the present application further provides an immune effector cell, wherein the immune effector cell expresses the aforementioned chimeric antigen receptor, or contains a nucleic acid fragment encoding the aforementioned chimeric antigen receptor; preferably, the immune effector cell is selected from a T cell, a natural killer cell (a NK cell), an NKT cell (a natural killer T cell), a DNT cell (a double negative T cell), a monocyte, a macrophage, a dendritic cell or a mast cell, and the T cell is preferably selected from a cytotoxic T cell (CTL), a regulatory T cell or a helper T cell; and preferably, the immune effector cell is an autologous immune effector cell or an allogeneic immune effector cell.

In a fifth aspect, the present application further provides an isolated nucleic acid fragment, wherein the nucleic acid fragment encodes the aforementioned antibody or the antigen-binding fragment thereof, the multispecific antigen-binding molecule, or the chimeric antigen receptor.

In a sixth aspect, the present application further provides a vector, wherein the vector comprises the aforementioned nucleic acid fragment.

In a seventh aspect, the present application further provides a host cell, wherein the host cell comprises the aforementioned vector; and preferably, the cell is a prokaryotic cell or a eukaryotic cell, such as a bacterial (*Escherichia coli*) cell, a fungal (yeast) cell, an insect cell or a mammalian cell (a CHO cell line or a 293T cell line).

In an eighth aspect, the present application further provides a method for preparing the antibody or the antigen-binding fragment thereof or the aforementioned multispecific antigen-binding molecule, wherein the method comprises culturing the aforementioned cell, and isolating the antibody, antigen-binding fragment or multispecific antigen-binding molecule expressed by the cell.

In a ninth aspect, the present application further provides a method for preparing the aforementioned immune effector cell, comprising introducing a nucleic acid fragment encoding the aforementioned CAR into the immune effector cell, and optionally, further comprising enabling the immune effector cell to express the aforementioned CAR.

In a tenth aspect, the present application further provides a pharmaceutical composition, wherein the pharmaceutical composition comprises the aforementioned antibody or the antigen-binding fragment thereof, the multispecific antigen-binding molecule, the immune effector cell, the nucleic acid fragment or the vector, or a product prepared according to the aforementioned method; optionally, the pharmaceutical composition also comprises a pharmaceutically acceptable carrier, a diluent or an auxiliary agent; and optionally, the pharmaceutical composition further comprises an additional anti-tumor agent.

In some embodiments, the pharmaceutically acceptable carrier is a carrier that does not weaken the viability and function of immune cells and does not affect the specific binding of antibodies or antigen-binding fragments thereof to antigens, and includes but not limited to cell culture media, buffers, physiological saline, balanced salt solution, *etc.* Examples of buffers include isotonic phosphate, acetate, citrate, borate, carbonate, *etc.* In a particular embodiment, the pharmaceutically acceptable carrier is phosphate buffered saline containing 1% serum.

In an eleventh aspect, the present application further provides a method for treating a tumor or a cancer, wherein the method comprises administering to a subject an effective amount of the aforementioned antibody or antigen-binding fragment thereof, the multispecific antigen-binding molecule, the immune effector cell, nucleic acid fragment, the vector, a product prepared according to the aforementioned method or the pharmaceutical composition.

In a preferred embodiment, the tumor or cancer is a GPRC5D-expressing tumor or cancer, preferably a B-cell lymphoma, and more preferably multiple myeloma (MM).

In a twelfth aspect, the present application further provides the use of the aforementioned antibody or the antigen-binding fragment thereof, the multispecific antigen-binding molecule, the immune effector cell, the nucleic acid fragment, the vector, a product prepared according to the aforementioned method or the pharmaceutical composition in preparing a drug for treating a tumor or a cancer.

In a preferred embodiment, the tumor or cancer is a GPRC5D-expressing tumor or cancer, preferably a B-cell lymphoma, and more preferably multiple myeloma (MM).

In a thirteenth aspect, the present application further provides a kit, wherein the kit comprises the aforementioned antibody or the antigen-binding fragment thereof, the multispecific antigen-binding molecule, the immune effector cell, the nucleic acid fragment, the vector, a product prepared according to the aforementioned method or the pharmaceutical composition.

In a fourteenth aspect, the present application further provides a method for detecting GPRC5D expression in a biological sample, wherein the method comprises contacting the biological sample with the aforementioned antibody or the antigen-binding fragment thereof under conditions that allow the formation of a complex from the antibody or the antigen-binding fragment thereof and GPRC5D; preferably, the method further comprises detecting the formation of the complex, thereby indicating the presence or expression level of GPRC5D in the sample.

In a fifteenth aspect, the present application further provides the use of the aforementioned antibody or the antigen-binding fragment thereof in preparing a GPRC5D detection reagent.

### Examples

The present application will be further described below in conjunction with specific examples, and the advantages and characteristics of the present application will become clearer along with the description. If no specific conditions are indicated in the examples, conventional conditions or the conditions suggested by the manufacturer shall be followed. Any reagents or instruments used, unless the manufactures stated, are conventional products that are commercially available.

The examples of the present application are only exemplary and do not limit the scope of the present application in any way. Those skilled in the art should understand that the details and forms of the technical solutions of the present application can be modified or replaced without departing from the spirit and scope of the present application, but these modifications and replacements all fall within the scope of protection of the present application.

### Example 1 Preparation of control antibody, identification of endogenous cell and preparation of overexpression cell line

### 1.1 Preparation of control antibody

The sequence of JNJ7564 comes from International Patent Application No. WO 2018017786 A2, and the sequence of 5F11 comes from International Patent Application No. WO 2019154890 A1. The cloned VH and VL sequences that recognize the human GPRC5D epitope were recombined into the human IgG1 CH and CL expression vectors to obtain recombinant plasmids. The control antibody, CH, CL and Fc sequences are shown in Table 1.

The plasmids and transfection reagent PEI (Polysciences, catalog No. 24765-1) were added to OPTI-MEM (Gibco, catalog No. 11058021), mixed well, let stand for 15 min, and then added to Expi293 cells (Manufacturer: Thermofisher, catalog No. A14527), the obtained mixture was put in a shaker for culture at 5% CO₂, 120 rpm and 37°C. On the second day of transfection, OPM-293 ProFeed (Shanghai OPM Biosciences Co., Ltd., catalog No. F081918-001) and 6 g/L glucose (Manufacturer: Sigma, catalog No. G7528) were added. On the sixth day of transfection, the cell supernatant was collected.

A 0.22 µm disposable filter was used to filter the sample, then 0.5M NaOH was used to sterilize the system for 30 min, 0.1M NaOH was used to sterilize the chromatography column for 5 min. Each flow path of the AKTA pure 150 was filled with the respective buffer solutions. The sequence is as follows: **A2** was charged with Mab affinity elution liquid (anhydrous disodium hydrogen phosphate at a concentration of 2.69 g/L (No. 20040618, Sinopharm Chemical Reagent Co., Ltd.), sodium dihydrogen phosphate dihydrate at 0.17 g/L (No. 20040718, Sinopharm Chemical Reagent Co., Ltd.), and sodium chloride at 58.44 g/L (No. 10019318, Sinopharm Chemical Reagent Co., Ltd.); **B1** was filled with Mab affinity eluent (citric acid monohydrate 3.92 g/L (No. 10007118, Sinopharm Chemical Reagent Co., Ltd.) and trisodium citrate dihydrate 1.88 g/L (No. 10019418, Sinopharm Chemical Reagent Co., Ltd.)); **A1** was filled with Mab affinity equilibration buffer (20 × PBS Buffer was diluted to 1 × PBS by adding ddH₂O (No. B548117-050, Sangon Biotech (Shanghai) Co., Ltd.)), and the Buffer pump was filled with Mab affinity equilibration buffer (20 × PBS Buffer was diluted to 1 × PBS by adding ddH₂O (No. B548117-050, Sangon Biotech (Shanghai) Co., Ltd.)); **S1** was loaded with the sample , and a flow rate of 5 mL/min was employed for sample loading. Upon completion of the sample loading, the buffer pump was utilized for **wash1** (Mab affinity equilibration buffer) until the UV decreased to below 10 mAU. Subsequently, the inlet was switched to **A2** for **wash2** with a high-salt solution (Mab affinity elution liquid) to remove impurities. Then the inlet was switched to **A1** to displace the high-salt solution in the flow path until the conductivity dropped to approximately 20 ms/cm. Thereafter, the inlet was switched to **B1** for the elution of the target protein. The target peak was collected, 1M Tris (121.2 g/L, pH 8.0) was added to adjust the pH to neutral, and Nano drop 8000 was used for concentration determination.

**Table 1 Control antibody sequence information**

| **Designation of sequence** | **Sequence number** | **Amino acid sequence** |
|---|---|---|
| JNJ7564 VH | SEQ ID NO: 1 | QVQLVQSGAEVKKPGASVKVSCKASGYSFTGYTMNWVRQAPGQGLEW MGLINPYNSDTNYAQKLQGRVTMTTDTSTSTAYMELRSLRSDDTAVYYCA RVALRVALDYWGQGTLVTVSS |
| JNJ7564 VL | SEQ ID NO: 2 | DIQMTQSPSSLSASVGDRVTITCKASQNVATHVGWYQQKPGKAPKRLIYS ASYRYSGVPSRFSGSGSGTEFTLTISNLQPEDFATYYCQQYNRYPYTFGQG TKLEIK |
| 5F11 VH | SEQ ID NO: 3 | EVQLVESGGGLVQPGRSLKLSCAASGFSFSNYGMAWVRQAATKGLEWVA SISTGGGNTYYRDSVKGRFIVSRDNAKNTQYLQMDSLRSEDTATYYCTRH DRGGLYWGQGVMVTVSS |
| 5F11 VL | SEQ ID NO: 4 | DIVMTQAPLSVSVTPGESASISCRSSKSLLHSNGITYVYWFQKPGKSPQV LIYRMSNLASGVPDRFSGSGSETDFTLKISRVEAEDVGIYHCGQLLENPYTF GAGTELELK |
| Human IgG1 heavy chain constant region | SEQ ID NO: 5 | ASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKS CDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEY KCKVSNKALPAPIEKTISKAKGQPREPQVYTLPPSREEMTKNQVSLTCLVK GFYPSDIAVEWESNGQPENNYKTTPPVLDSDGSFFLYSKLTVDKSRWQQG NVFSCSVMHEALHNHYTQKSLSLSPGK |
| Human IgG1 light chain constant region | SEQ ID NO: 6 | |
| Human IgG1 Fc | SEQ ID NO: 7 | EPKSSDKTHTCPPCPAPELLGGPSVFLFPPKPKDTLMISRTPEVTCVVVDV SHEDPEVKFNWYVDGVEVHNAKTKPREEQYNSTYRVVSVLTVLHQDWL NGKEYKCKVSNKALPAPIEKTISKAKGQPREPQVYTLPPSREEMTKNQVSL TCLVKGFYPSDIAVEWESNGQPENNYKTTPPVLDSDGSFFLYSKLTVDKSR WQQGNVFSCSVMHEALHNHYTQKSLSLSPGK |

| | | |
|---|---|---|
| Notes: In the human IgG1 Fc sequence, the hinge region is highlighted in bold and contains the C220S mutation, which is underlined. | | |

### 1.2 Identification of cell line endogenously expressing human GPRC5D protein

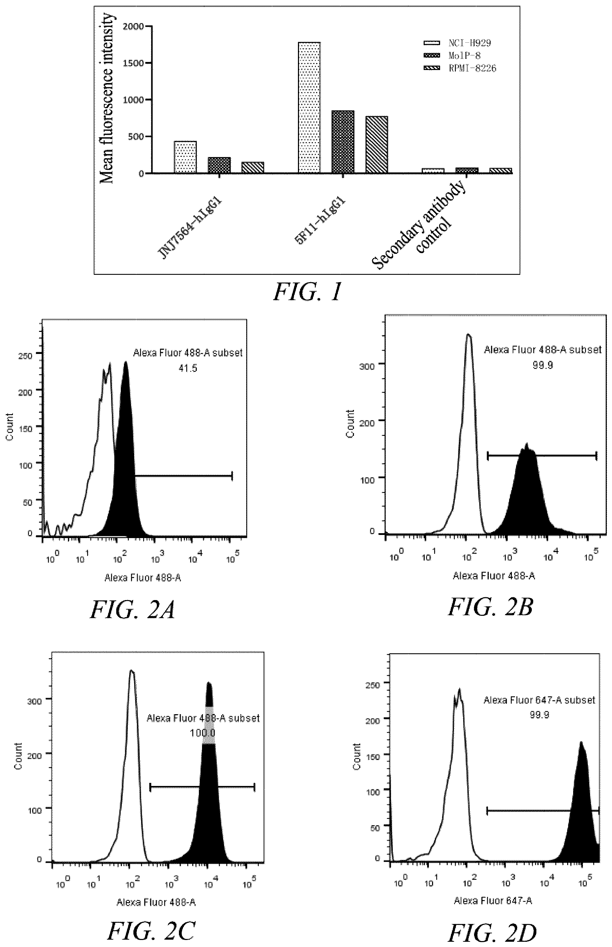

Cells endogenously expressing human GPRC5D protein were scale-up cultured in T-75 cell culture flasks to the logarithmic growth phase, the supernatant of the medium was discarded by centrifugation, and the cell pellet was washed twice with PBS. 20 nM JNJ7564-hIgG1 and 5F11-hIgG1 antibodies were used as primary antibodies and Alexa Fluor^{®} 647 AffiniPure Goat Anti-Human IgG (H+L) was used as a secondary antibody (purchased from Jackson Immuno, catalog No. 109-605-088), and FACS (FACS CantoTM, purchased from BD Company) was used for detection and analysis. The results are shown in Table 2 and Fig.1, indicating that NCI-H929, MolP-8 and RPMI-8226 cells all endogenously express human GPRC5D protein, and control antibodies JNJ7564-hIgG1 and 5F11-hIgG1 have binding activity with all cells.

**Table 2 FACS detection of endogenous GPRC5D expression quantity in tumor cells**

| **No.** | **Cell line having endogenous expression** | **Mean fluorescence intensity** | | |
|---|---|---|---|---|
| | | **Secondary antibody control** | **JNJ7564-hIgG 1** | **5F11-hIgG1** |
| 1 | NCI-H929 | 69 | 438 | 1784 |
| 2 | MolP-8 | 81 | 217 | 850 |
| 3 | RPMI-8226 | 74 | 156 | 776 |

### 1.3 Preparation of CHO-K1 recombinant cell lines expressing human GPRC5A, GPRC5B, GPRC5C, and GPRC5D proteins

The nucleotide sequences encoding the amino acid sequences of humanGPRC5A (Uniprot: Q8NFJ5-1, SEQ ID NO: 8), humanGPRC5B (Uniprot: Q9NZH0-1, SEQ ID NO: 9), humanGPRC5C (Uniprot: Q9NQ84, SEQ ID NO: 10), and humanGPRC5D (Uniprot: Q9NZD1, SEQ ID NO: 11) were cloned into the pLVX lentiviral vector, respectively, and viral particles were prepared in HEK293T cells. Following the infection of CHOK1 cell lines (purchased from the Chinese Academy of Sciences) with lentivirus, the cells were selectively cultured for 1 week in Advanced DMEM/F12 Medium (Gibco, catalog No. 12634028) supplemented with 10 µg/ml puromycin (Gibco, catalog No. A1113803) and 10% (v/v) fetal bovine serum (ExCell Bio, catalog No. FND500). The cells were stained using humanGPRC5A Alexa Fluor 488-conjugated antibody (R&D Systems, catalog No. IC5239G-100UG), humanGPRC5B Alexa Fluor 488-conjugated antibody (R&D Systems, catalog No. FAB10253G-100UG), and humanGPRC5C Alexa Fluor 488-conjugated antibody (R&D Systems, catalog No. FAB6594G-100UG), respectively. CHOK1 transfected with humanGPRC5D was stained using human anti-human GPRC5D antibody (JNJ7564, produced in house) and goat anti-human IgG (H+L) antibody (Jackson, catalog No. 109605088). Then, the positive cell pool with high-expression level was sorted into a 96-well plate on a flow cytometer FACS Aria III (purchased from BD Biosciences), and cultured at 37°C and 5% (v/v) CO₂. Approximately two weeks later, a subset of the cells was selected for proliferation. The positive cell pool with better growth, higher fluorescence intensity and better homogeneity was selected for further scale-up culture and then cryopreserved in liquid nitrogen. The results of expression quantity identification are shown in Table 3 and Figs. 2A-2D. The results show that CHOK1 stable transfection cell lines expressing human GPRC5A, GPRC5B, GPRC5C and GPRC5D after puromycin stress screening have a relatively single positive peak, and can be used for FACS to detect the binding activity of antibodies with human GPRC5A, GPRC5B, GPRC5C and GPRC5D proteins.

**Table 3 FACS detection results of CHOK1 stable transfection cell line of human GPRC5 protein**

| **No.** | **Stably transfected positive cell pool** | **Mean fluorescence intensity** | |
|---|---|---|---|
| | | **IgG subtype control** | **Human positive antibody** |
| 1 | CHOK1-hGPRC5A high clone C7 | 38.7 | 168 |
| 2 | CHOK1-hGPRC5B high pool | 101 | 4124 |
| 3 | CHOK1-hGPRC5C high pool | 103 | 10559 |
| 4 | CHOK1-hGPRCSD high pool | 56.4 | 95486 |

Human GPRC5A full-length amino acid sequence (Uniprot: Q8NFJ5-1, SEQ ID NO: 8): 
MATTVPDGCRNGLKSKYYRLCDKAEAWGIVLETVATAGVVTSVAFMLTLPILVCKVQ
DSNRRKMLPTQFLFLLGVLGIFGLTFAFIIGLDGSTGPTRFFLFGILFSICFSCLLAHAVSLTKL
VRGRKPLSLLVILGLAVGFSLVQDVIAIEYIVLTMNRTNVNVFSELSAPRRNEDFVLLLTYVL
FLMALTFLMSSFTFCGSFTGWKRHGAHIYLTMLLSIAIWVAWITLLMLPDFDRRWDDTILSS
ALAANGWVFLLAYVSPEFWLLTKQRNPMDYPVEDAFCKPQLVKKSYGVENRAYSQEEITQ
GFEETGDTLYAPYSTHFQLQNQPPQKEFSIPRAHAWPSPYKDYEVKKEGS
Human GPRC5B full-length amino acid sequence (Uniprot: Q9NZH0-1, SEQ ID NO: 9): 
ENASTSRGCGLDLLPQYVSLCDLDAIWGIVVEAVAGAGALITLLLMLILLVRLPFIKEKE
KKSPVGLHFLFLLGTLGLFGLTFAFIIQEDETICSVRRFLWGVLFALCFSCLLSQAWRVRRLV
RHGTGPAGWQLVGLALCLMLVQVIIAVEWLVLTVLRDTRPACAYEPMDFVMALIYDMVLL
VVTLGLALFTLCGKFKRWKLNGAFLLITAFLSVLIWVAWMTMYLFGNVKLQQGDAWNDP
 
TLAITLAASGWVFVIFHAIPEIHCTLLPALQENTPNYFDTSQPRMRETAFEEDVQLPRAYME
NKAFSMDEHNAALRTAGFPNGSLGKRPSGSLGKRPSAPFRSNVYQPTEMAVVLNGGTIPTA
PPSHTGRHLW
Human GPRC5C full-length amino acid sequence (Uniprot: Q9NQ84, SEQ ID NO: 10): 
QGHVPPGCSQGLNPLYYNLCDRSGAWGIVLEAVAGAGIVTTFVLTIILVASLPFVQDTK
KRSLLGTQVFFLLGTLGLFCLVFACVVKPDFSTCASRRFLFGVLFAICFSCLAAHVFALNFLA
RKNHGPRGWVIFTVALLLTLVEVIINTEWLIITLVRGSGEGGPQGNSSAGWAVASPCAIANM
DFVMALIYVMLLLLGAFLGAWPALCGRYKRWRKHGVFVLLTTATSVAIWVVWIVMYTYG
NKQHNSPTWDDPTLAIALAANAWAFVLFYVIPEVSQVTKSSPEQSYQGDMYPTRGVGYETI
LKEQKGQSMFVENKAFSMDEPVAAKRPVSPYSGYNGQLLTSVYQPTEMALMHKVPSEGA
YDIILPRATANSQVMGSANSTLRAEDMYSAQSHQAATPPKDGKNSQVFRNPYVWD
Human GPRC5D full-length amino acid sequence (Uniprot: Q9NZD1, SEQ ID NO: 11):

MYKDCIESTGDYFLLCDAEGPWGIILESLAILGIVVTILLLLAFLFLMRKIQDCSQWNVL
PTQLLFLLSVLGLFGLAFAFIIELNQQTAPVRYFLFGVLFALCFSCLLAHASNLVKLVRGCVS
FSWTTILCIAIGCSLLQIIIATEYVTLIMTRGMMFVNMTPCQLNVDFVVLLVYVLFLMALTFF
VSKATFCGPCENWKQHGRLIFITVLFSIIIWVVWISMLLRGNPQFQRQPQWDDPVVCIALVT
NAWVFLLLYIVPELCILYRSCRQECPLQGNACPVTAYQHSFQVENQELSRARDSDGAEEDVA
LTSYGTPIQPQTVDPTQECFIPQAKLSPQQDAGGV

### 1.4 Preparation of HEK293T recombinant cell line expressing human GPRC5D protein

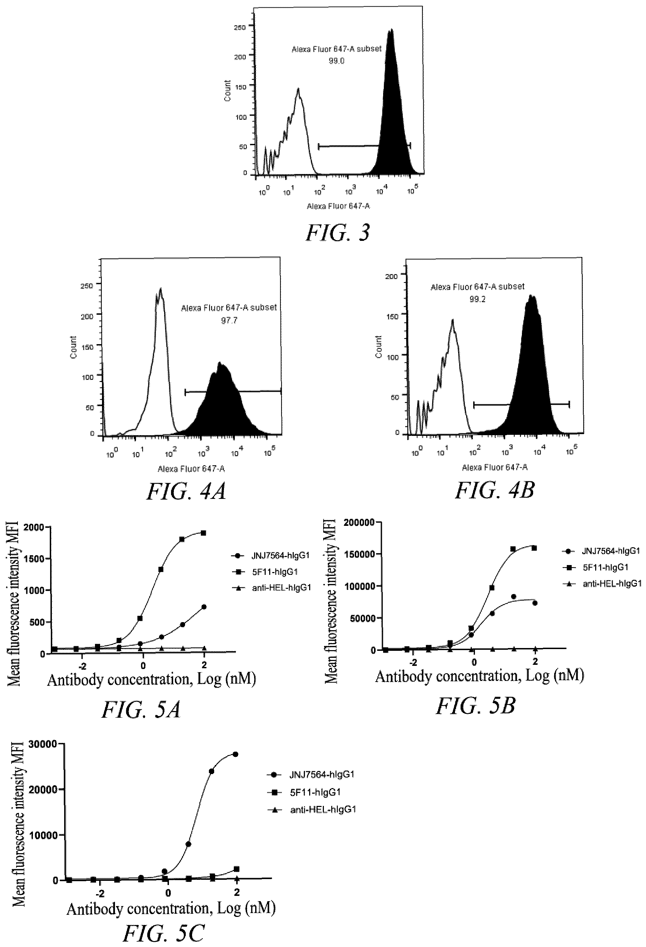

The specific method referred to Example 1.3. The HEK293T cell line was infected with lentivirus packaging the human GPRC5D plasmid vector and selectively cultured in DMEM Medium (Gibco, catalog No. 10569044) containing 2 µg/ml puromycin (Gibco, catalog No. A1113803) and 10% (v/v) fetal bovine serum (ExCell Bio, catalog No. FND500) for 2 weeks. Human anti-human GPRC5D antibody (JNJ7564, produced in house) and goat anti-human IgG (H+L) antibody (Jackson, catalog No. 109605088) were used for detection on the flow cytometer FACS CantoII (purchased from BD Biosciences). The results of the expression quantity identification of cell lines having good expression are shown in Table 4 and Fig. 3. The results show that HEK293T-hGPRC5D after puromycin stress screening has a relatively single positive peak, and can be used for FACS to detect the binding activity of antibodies with human GPRC5D protein.

**Table 4 FACS detection results of 293T stable transfection cell line of human GPRC5D protein**

| **No.** | **Stably transfected positive cell pool** | **Cell mean fluorescence density** | |
|---|---|---|---|
| | | **IgG subtype control** | **Human GPRC5D antibody** |
| 1 | HEK293T-hGPRC5D high pool | 18.9 | 30536 |

### 1.5 Preparation of CHO-K1 and HEK293T recombinant cell line expressing monkey GPRC5D protein

The specific method of preparing the CHO-K1 recombinant cell line stably expressing monkey GPRC5D (cynoGPRC5D) referred to Example 1.3. cynoGPRC5D amino acid sequence, NCBI: XP_005570249.1 (SEQ ID NO: 12). Human anti-cynoGPRC5D antibody (JNJ7564, produced in house) and goat anti-human IgG (H+L) antibody (Jackson, catalog No. 109605088) were used for detection on the flow cytometer FACS CantoII (purchased from BD Biosciences). The results of the expression quantity identification of cell lines having good expression are shown in Table 5 and Fig. 4A. The results show that CHOK1-cynoGPRC5D after puromycin stress screening has a relatively single positive peak, and can be used for FACS to detect the cross-activity of antibodies with monkey GPRC5D protein.

**Table 5 FACS detection results of CHOK1 stable transfection cell line of cynoGPRC5D protein**

| **No.** | **Stably transfected positive cell pool** | **Mean fluorescence density** | |
|---|---|---|---|
| | | **IgG subtype control** | **GPRC5D antibody** |
| 1 | CHOK1-cynoGPRC5D high pool | 52.9 | 13093 |

The specific method of preparing the HEK293T recombinant cell line stably expressing monkey GPRC5D (cynoGPRC5D) referred to Example 1.4. The results of the expression quantity identification of cell lines having good expression are shown in Table 6 and Fig. 4B. The results show that HEK293T-cynoGPRC5D after puromycin stress screening has a relatively single positive peak, and can be used for FACS to detect the cross-activity of antibodies with monkey GPRC5D protein.

**Table 6 FACS detection results of HEK293T stable transfection cell line of cynoGPRC5D protein**

| **No.** | **Stably transfected positive cell pool** | **Mean fluorescence density** | |
|---|---|---|---|
| | | **IgG subtype control** | **GPRC5D antibody** |
| 1 | HEK293T-cynoGPRC5D high pool | 18.2 | 8259 |

Monkey GPRC5D full-length amino acid sequence (NCBI: XP_005570249.1, SEQ ID NO: 12):

MYKDCIESTGDYFLPCDSEGPWGIVLESLAILGIVVTILLLLAFLFLMRKIQDCSQWNV
LPTQLLFLLSVLGLFGLAFAFIIQLNQQTAPVRYFLFGVLFALCFSCLLAHASNLVKLVRGRV
SFSWTTILCIAIGCSLLQVIIAIEYVTLIMTRGMMFVHMTPYQLNVDFVVLLVYVLFLMALTF
FVSKATFCGPCENWKQHGRLIFITVLFSIIIWVVWISMLLRGNPQFQRQPQWDDPVVCIALV
TNAWVFLLLYIVPELCILYRSCRQECPSQGHACPVTAYQRSFQVENQELSRARDSDGAEEDV
ALTSFGTPIQPQTVDPTQECFIPRAKLSPQQDAGV

### 1.6 Experiments on the binding of recombinant cell line with control antibody

FACS detections of the binding activity of control antibodies with cells expressing human GPRC5D and monkey GPRC5D are shown in Figs. 5A to 5C. The IgG subtype control was human IgG1. JNJ7564-hIgG1 and 5F11-hIgG1 had good binding activity with NCI-H929 tumor cells expressing human GPRC5D protein, and HEK293T-hGPRC5D and HEK293T-cynoGPRC5D recombinant cells. 5F11-hIgG1 had a relatively stronger binding activity with NCI-H929 cells and HEK293T-hGPRC5D recombinant cells. JNJ7564-hIgG1 had a relatively stronger binding activity with HEK293T-cynoGPRC5D recombinant cells.

### Example 2 Preparation of anti-human GPRC5D hybridoma monoclonal antibody

### 2.1 Animal immunization

An anti-human GPRC5D monoclonal antibody was produced by immunizing mice. Female SJL mice aged 6 to 8 weeks (purchased from Shanghai Slack Experimental Animal Co., Ltd.) were used in the experiment. The mice were raised under SPF conditions. After the mice were purchased, they were raised in a laboratory environment for 1 week, with a 12/12-hour light/dark cycle adjustment, and a temperature of 20°C-25°C; humidity 40%-60%. The acclimatized mice were immunized according to the following scheme. For the primary immunization, the human GPRC5D overexpression cell line (HEK293T-hGPRC5D, in-house) was washed twice with PBS and mixed with oligonucleotide CpG (ODN 1826, synthesized from Sangon Biotech (Shanghai) Co., Ltd.). Each mouse was first intraperitoneally injected with 50 µl of emulsified Titer max (purchased from sigma, Cat. T2684). After waiting for 15 min, each mouse was intraperitoneally injected with 5 × 10⁶ cells/100 µl. For the first booster immunization, the human GPRC5D overexpression cell line was washed twice with PBS and mixed with CpG, and each mouse was intraperitoneally injected with 5 × 10⁶ cells/100 µl. Subsequent booster immunizations were performed alternately in the same manner as the primary immunization and the first booster immunization. Monkey GPRC5D overexpression cell line (HEK293T-cynoGPRC5D, in-house) and human GPRC5D overexpression cell line (HEK293T-hGPRC5D) were alternately used as immunogens. There was an interval of 7 days between each immunization. Blood collection from mice was performed on day 5 after the second and fourth booster immunizations. The serum was separated and a cell-based ELISA method was used to determinate the titer of a specific antibody in the serum.

In order to detect the titer of specific antibody in serum, the HEK293T-hGPRC5D, HEK293T-cynoGPRC5D, CHOK1-hGPRC5D, CHOK1-cynoGPRC5D recombinant cells obtained in Example 1 and the blank control cells were inoculated into a 96-well cell plate (manufacturer: corning, catalog No. 3599) at 4 × 10⁴ cells per well, and incubated overnight in a cell culture incubator. The supernatant medium was removed, and 50 µl fixative (manufacturer: Beyotime, catalog No. P0098-500ML) was added to each well. The plate was placed in a fume hood at room temperature for 30 min, the supernatant fixative was removed, washing was performed 2 times with PBST, and then 0.5% skimmed milk (manufacturer: Sangon, catalog No. A600669-0250) was added. Blocking was performed at room temperature for 2 h, the blocking solution was poured off, the plate was washed 2 times with PBST, and mouse serum diluted at a starting dilution of 1 : 100 and 3-fold gradient was added at 50 µl/well. After incubation at room temperature for 1 h, the plate was washed 3 times with PBST. HRP (horseradish peroxidase)-labeled secondary antibody (purchased from Jackson, catalog No. 109-035-088) was added, the mixture was incubated at room temperature for 1 h, and the plate was washed 5 times with PBST. TMB substrate was added at 50 µl/well, and the mixture was incubated at room temperature for 5-10 min, and then a stop solution (1.0N HCl) was added at 50 µl/well. The OD450 nm values were read by an ELISA microplate reader (Multimode Plate Reader, EnSight, purchased from Perkin Elmer). The results of the cell-based ELISA showed that the sera of mice immunized with the above cells all bind to the immunogen to varying degrees, showing antigen-antibody reactions.

### 2.2 Splenocyte fusion and hybridoma screening

Each selected mouse was intraperitoneally injected with 5 × 10⁶ HEK293T-hGPRC5D cells. The mice were sacrificed 3 days later, and splenocytes and lymphocytes were collected. After centrifugation at 1500 rpm, the supernatant was discarded and ACK lysis buffer (Gibco, Cat. A1049201) was added to the cells to lyse the doped red blood cells in the cells to obtain a cell suspension. The cells were washed 3 times with DMEM basic medium (purchased from Gibco, catalog No. 10569044) at 1500 revolutions per minute, the cells were counted, and then subjected to cell fusion with mouse myeloma cells SP2/0 (purchased from ATCC, Cat. CRL-1581) at a ratio of 2 : 1 of viable cells using electrofusion method. The fused cells were diluted in DMEM medium contain 20% (v/v) fetal calf serum (purchased from ExCell Bio, Cat. FND500), 1 × HAT (purchased from sigma, Cat. H0262-10VL), bovine insulin (purchased from Yeason, Cat. 40107ES25) and NEAA (purchased from Gibco, Cat. 11140050), then added into a 96-well cell culture plate at 5 × 10⁴ cells/200 µL per well, and the plate was put in an incubator at 5% (v/v) CO₂ and 37°C for culture. After 7 days, a cell based ELISA was used to screen the supernatant of the fusion plate to confirm the binding activity with human GPRC5D overexpression cells. For the positive clone supernatant, a cell based ELISA was used to confirm the binding activity with monkey GPRC5D overexpression cells and FACS was used to confirm the binding activity with endogenous cell NCI-H929.

According to the screening results, the qualified positive clones were selected and subcloned with semi-solid medium (purchased from stemcell, Cat. 03810). 7 days later, the grown clones were picked into a 96-well culture plate one by one, and scale-up cultured in DMEM medium containing 10% (w/w) fetal bovine serum and 1 × HT (purchased from sigma, Cat. H0137-10VL). 1 day later, a cell-based ELISA was used for preliminary screening, and positive single clones were picked to a 24-well plate and amplified for further culture. 3 days later, the culture supernatant was further tested to evaluate the binding activity with monkey GPRC5D overexpression cells and endogenous cells NCI-H929. Based on the test results of the samples in the 24-well plate, the optimal clones were selected and scale-up cultured in DMEM medium containing 10% (v/v) FBS at 37°C and 5% (v/v) CO₂, and then frozen and stored in liquid nitrogen to obtain the hybridoma cells of the present application.

### Example 3 Determination of amino acid sequence of variable regions of light and heavy chains of positive hybridoma clones

The hybridoma cells in the logarithmic growth phase were collected, and were fully lysed with Trizol (Invitrogen, Cat No. 15596-018) and stored at -80°C for testing. For the samples, GENEWIZ Suzhou was entrusted to determine the amino acid sequences of the variable regions of the light and heavy chains of the positive hybridoma clones. The sequencing results were analyzed by MOE software, and the phylogenetic tree was constructed according to the amino acid sequence of the protein encoded by the variable region. After eliminating the sequences that were close to each other on the phylogenetic tree according to the sequence similarity, 8 clones were obtained: GPRC5D-mab01-08 (see Table 7 for sequences SEQ ID NOs: 13-28).

**Table 7 Amino acid sequence information of variable regions of light and heavy chains of GPRC5D positive clones**

| **Designation of sequence** | **Sequence number** | **Amino acid sequence** |
|---|---|---|
| GPRC5D-mab01-VH | SEQ ID NO: 13 | QVQLKESGPGLVAPSQSLSITCTVSGFSLTNYGVSWVRQPPGKGL EWLGVIWGDGSTNYHSALISRLSIRKDNSKSQVFLKLNSLQTDDT ATYYCAKPASYYGRRTYAMDFWGHGTSVTVSS |
| GPRC5D-mab01-VL | SEQ ID NO: 14 | QIVLSQSPAILSASPGEKVTMTCRASSSVSYMHWYQQKPGSSPKP WIYATSHLASGVPARFSGSGSGTSYSLTISRVEAEDAATYYCQQW SSNPPMTFGGGTKLEIK |
| GPRCSD-mab02-VH | SEQ ID NO: 15 | QVQLQQPGAELVKPGASVKMSCKASGYTFSSYWITWVKQRPGQ GLDWIGDFYPRNGDTNYNKKFKNKATLTVDTSSSTAYMQLSSLT SEDSAVYYCARSRAYYGRRGFDYWGQGTTLTVSS |
| GPRC5D-mab02-VL | SEQ ID NO: 16 | QIVLTQSPAIMSASPGEKVTMTCSAGSSVSYMYWYQQKPGSSPR VLIYDTSNLASGVPVRFSGSGSGTSYSLIISRMEAEDAATYYCQQ WSNYPLTFGAGTKLELK |
| GPRC5D-mab03-VH | SEQ ID NO: 17 | QAYLQQSGAELVRPGASVKMSCKASGYTFTSYNMNWVKQTPRQ GLEWIGAIYPGNGDTSYNQKFKGKATLTVDKSSSTAYMQLSSLTS EDSAVYFCARVRLRYAMDYWGQGTSVTVSS |
| GPRC5D-mab03-VL | SEQ ID NO: 18 | DIVMTQSQKFMSTTVGDRVTITCKASQNVGTAVAWYQQKPGQSP KLLISSASNRYTGVPERFTGSGSGTDFTLTIRNMQSEDLADYFCQ QYSSYPWTFGGGTELEIK |
| GPRC5D-mab04-VH | SEQ ID NO: 19 | EVQLVESGGGLVKPGGSLKLSCAASRFTFSRYAMSWVRQTPEKR LEWVATISDGGSYTYYPDNVKGRFTISRDNAKNNLYLQMSHLKS EDTAIYYCARDRYYYGKGGYFDVWGTGTTVTVSS |
| GPRC5D-mab04-VL | SEQ ID NO: 20 | DIQMTQSPASLSVSVGETVTITCRASENIYRNLAWYQQKQGKSPQ LLVYAATNLADGVPSRFSGSGSGTQYSLKINSLQSEDFGSYYCQH FWGTPRTFGGGTKLEIK |
| GPRCSD-mab05-VH | SEQ ID NO: 21 | QVQLQQSGAELARPGASVMLSCKASGYTFTSYGISWVKQRTGQ GLEWIGEIYPRSGNTYYNEKFKGKATLTADKSSSTAYMELRSLTSE DSAVYFCARGRAYGRNYPMDYWGQGTSVTVSS |
| GPRC5D-mab05-VL | SEQ ID NO: 22 | DILLTQSPAILSVSVGERVSFSCRASQSIGTSIHWYQQRTNGSPRLL IKYASESISGIPSRFSGSGSGTDFTLSINSVESEDIADYYCQQSNSW PYTFGGGTKLEIK |
| GPRC5D-mab06-VH | SEQ ID NO: 23 | QVQLKQSGPGLVQPSQSLSITCTVSGFSLTSYGVNWIRQSPGKGL EWLGVIWSGGNTDYNAAFRSRLSISKDNSKSQVFFKMNSLQADD TAIYYCARGQLGRPYWYFDVWGTGTSVTVSS |
| GPRC5D-mab06-VL | SEQ ID NO: 24 | DIVMTQSHKFMSTSVGDRVSITCKASQDVRTAVAWYQQKPGQSP KLLIYWASTRHTGVPDRFTGSRSGTDYTLTISSVQAEDLALYYCQ QHYSTPLTFGAGTKLELK |
| GPRCSD-mab07-VH | SEQ ID NO: 25 | QVQLQQSGAEVVRPGTSVKVSCKASGYAFTNYLIEWLKQRPGQ GLEWIGMINPGRGNTNYNEKFKGKATLTADKASSTAYMQISSLTS EDSAVYFCARNWDVWGQGTTLTVSS |
| GPRC5D-mab07 - VL | SEQ ID NO: 26 | DVVMTQTPLSLPVSLGDQASISCRSSQSLVYSNGNTYLHWYLQK PGQSPKLLIYKVSNRFSGVPDRFSGSGSGTDFTLKISRVEAEDLGV YFCSQSTHVPWTFGGGTKLEIK |
| GPRCSD-mab08-VH | SEQ ID NO: 27 | QVQLQQSGAELARPGASVKLSCKTSGYIFTNFGITWVKQRTGQG LEWIGENYPRRINTYYNEKFKGKATLTADKSSNTAYLELRSLTSED SAVYFCSRKDIFGLSYALDYWGQGTSVTVSS |
| GPRCSD-mab08-VL | SEQ ID NO: 28 | DIVMTQSQKFMSTSVGDRVSVTCKASQNVGTNVAWYQHKPGQS PKALIYSASYQYSGVPDRFTGSGSGTDFTLTISNVQSEDLAEYICQ QYKNYPYTFGGGTKLEIK |

The Kabat and IMGT numbering systems were used respectively for CDR division of the sequences in Table 7. The sequences of CDRs are shown in Table 8:

**Table 8 Sequence list of CDRs of GPRC5D positive clones**

| **Antibody** | **Method for division** | **CDR1** | **CDR2** | **CDR3** |
|---|---|---|---|---|
| GPRC5D-mab01-VH | Kabat | NYGVS (SEQ ID NO: 29) | VIWGDGSTNYHSALIS (SEQ ID NO: 30) | PASYYGRRTYAMDF (SEQ ID NO: 31) |
| | IMGT | GFSLTNYG (SEQ ID NO: 32) | IWGDGST (SEQ ID NO: 33) | AKPASYYGRRTYAMDF (SEQ ID NO: 34) |
| GPRC5D-mab01-VL | Kabat | RASSSVSYMH (SEQ ID NO: 35) | ATSHLAS (SEQ ID NO: 36) | QQWSSNPPMT (SEQ ID NO: 37) |
| | IMGT | SSVSY (SEQ ID NO: 38) | ATS (SEQ ID NO: 39) | QQWSSNPPMT (SEQ ID NO: 37) |
| GPRC5D-mab02-VH | Kabat | SYWIT (SEQ ID NO: 40) | DFYPRNGDTNYNKKFKN (SEQ ID NO: 41) | SRAYYGRRGFDY (SEQ ID NO: 42) |
| | IMGT | GYTFSSYW (SEQ ID NO: 43) | FYPRNGDT (SEQ ID NO: 44) | ARSRAYYGRRGFDY (SEQ ID NO: 45) |
| GPRC5D-mab02-VL | Kabat | SAGSSVSYMY (SEQ ID NO: 46) | DTSNLAS (SEQ ID NO: 47) | QQWSNYPLT (SEQ ID NO: 48) |
| | IMGT | SSVSY (SEQ ID NO: 49) | DTS (SEQ ID NO: 50) | QQWSNYPLT (SEQ ID NO: 48) |
| GPRC5D-mab03-VH | Kabat | SYNMN (SEQ ID NO: 51) | AIYPGNGDTSYNQKFKG (SEQ ID NO: 52) | VRLRYAMDY (SEQ ID NO: 53) |
| | IMGT | GYTFTSYN (SEQ ID NO: 54) | IYPGNGDT (SEQ ID NO: 55) | ARVRLRYAMDY (SEQ ID NO: 56) |
| GPRC5D-mab03-VL | Kabat | KASQNVGTAVA (SEQ ID NO: 57) | SASNRYT (SEQ ID NO: 58) | QQYSSYPWT (SEQ ID NO: 59) |
| | IMGT | QNVGTA (SEQ ID NO: 60) | SAS (SEQ ID NO: 61) | QQYSSYPWT (SEQ ID NO: 59) |
| GPRC5D-mab04-VH | Kabat | RYAMS (SEQ ID NO: 62) | TISDGGSYTYYPDNVKG (SEQ ID NO: 63) | DRYYYGKGGYFDV (SEQ ID NO: 64) |
| | IMGT | RFTFSRYA (SEQ ID NO: 65) | ISDGGSYT (SEQ ID NO: 66) | ARDRYYYGKGGYFDV (SEQ ID NO: 67) |
| GPRC5D-mab04-VL | Kabat | RASENIYRNLA (SEQ ID NO: 68) | AATNLAD (SEQ ID NO: 69) | QHFWGTPRT (SEQ ID NO: 70) |
| | IMGT | ENIYRN (SEQ ID NO: 71) | AAT (SEQ ID NO: 72) | QHFWGTPRT (SEQ ID NO: 70) |
| GPRC5D-mab05-VH | Kabat | SYGIS (SEQ ID NO: 73) | EIYPRSGNTYYNEKFKG (SEQ ID NO: 74) | GRAYGRNYPMDY (SEQ ID NO: 75) |
| | IMGT | GYTFTSYG (SEQ ID NO: 76) | IYPRSGNT (SEQ ID NO: 77) | ARGRAYGRNYPMDY (SEQ ID NO: 78) |
| GPRC5D-mab05-VL | Kabat | RASQSIGTSIH (SEQ ID NO: 79) | YASESIS (SEQ ID NO: 80) | QQSNSWPYT (SEQ ID NO: 81) |
| | IMGT | QSIGTS (SEQ ID NO: 82) | YAS (SEQ ID NO: 83) | QQSNSWPYT (SEQ ID NO: 81) |
| GPRC5D-mab06-VH | Kabat | SYGVN (SEQ ID NO: 84) | VIWSGGNTDYNAAFRS (SEQ ID NO: 85) | GQLGRPYWYFDV (SEQ ID NO: 86) |
| | IMGT | GFSLTSYG (SEQ ID NO: 87) | IWSGGNT (SEQ ID NO: 88) | ARGQLGRPYWYFDV (SEQ ID NO: 89) |
| GPRC5D-mab06-VL | Kabat | KASQDVRTAVA (SEQ ID NO: 90) | WASTRHT (SEQ ID NO: 91) | QQHYSTPLT (SEQ ID NO: 92) |
| | IMGT | QDVRTA (SEQ ID NO: 93) | WAS (SEQ ID NO: 94) | QQHYSTPLT (SEQ ID NO: 92) |
| GPRC5D-mab07-VH | Kabat | NYLIE (SEQ ID NO: 95) | MINPGRGNTNYNEKFKG (SEQ ID NO: 96) | NWDV (SEQ ID NO: 97) |
| | IMGT | GYAFTNYL (SEQ ID NO: 98) | INPGRGNT (SEQ ID NO: 99) | ARNWDV (SEQ ID NO: 100) |
| GPRC5D-mab07-VL | Kabat | RSSQSLVYSNGNT YLH (SEQ ID NO: 101) | KVSNRFS (SEQ ID NO: 102) | SQSTHVPWT (SEQ ID NO: 103) |
| | IMGT | QSLVYSNGNTY (SEQ ID NO: 104) | KVS (SEQ ID NO: 105) | SQSTHVPWT (SEQ ID NO: 103) |
| GPRC5D-mab08-VH | Kabat | NFGIT (SEQ ID NO: 106) | ENYPRRINTYYNEKFKG (SEQ ID NO: 107) | KDIFGLSYALDY (SEQ ID NO: 108) |
| | IMGT | GYIFTNFG (SEQ ID NO: 109) | NYPRRINT (SEQ ID NO: 110) | SRKDIFGLSYALDY (SEQ ID NO: 111) |
| GPRC5D-mab08-VL | Kabat | KASQNVGTNVA (SEQ ID NO: 112) | SASYQYS (SEQ ID NO: 113) | QQYKNYPYT (SEQ ID NO: 114) |
| | IMGT | QNVGTN (SEQ ID NO: 115) | SAS (SEQ ID NO: 116) | QQYKNYPYT (SEQ ID NO: 114) |

General Biosystems (Anhui) Co., Ltd. was entrusted to synthesize the nucleotide sequences encoding the heavy chain and light chain variable regions of the above 8 clones, and the nucleotide sequences were linked through a linker with the sequence GGGGSGGGGSGGGGS (SEQ ID NO: 117), and then cloned into the expression vector pTT5-huFc (C220s) containing a signal peptide and human antibody Fc (heavy chain constant region sequence SEQ ID NO: 5) to obtain the expression vector of human-mouse chimeric antibody. The expression and purification steps referred to Example 1.1.

### Example 4 Identification of anti-GPRC5D human-murine chimeric antibody

### 4.1 Cell-based enzyme-linked immunosorbent assay (cell-based ELISA) detection of the binding of chimeric antibody with human GPRC5D protein

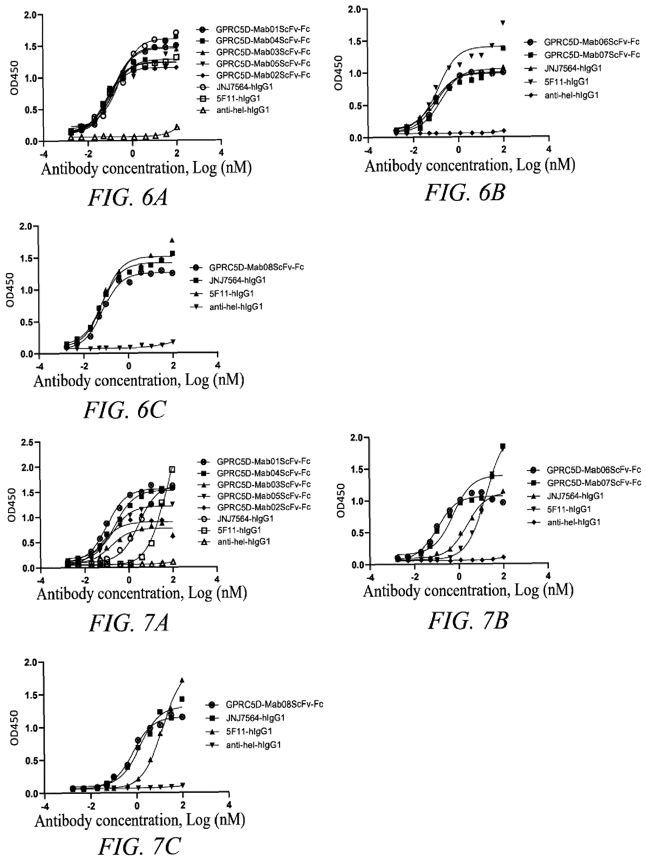

The specific detection method referred to Example 2.1. The CHOK1-hGPRC5D, CHOK1-cynoGPRC5D recombinant cells and CHOK1 cells obtained in Example 1 were fixed, and chimeric antibodies serially diluted 3-fold with a starting concentration of 100 nM and control antibody were added at 50 µl/well for detection. Cell based ELISA detection results of binding activity of the chimeric antibodies with human GPRC5D protein are shown in Figs. 6A-6C, 7A-7C and 8A-8C. The results showed that the produced pure chimeric antibodies bind to human GPRC5D protein to varying degrees at the ELISA level. The negative control antibody hIgG1 was the antibody anti-hel-hIgG1 (purchased from Biointron, catalog No. B117901) against chicken egg lysozyme. The data in the figure are OD450nm values.

### 4.2 Flow cytometry experiment (FACS) to detect the binding of chimeric antibodies to endogenous cells

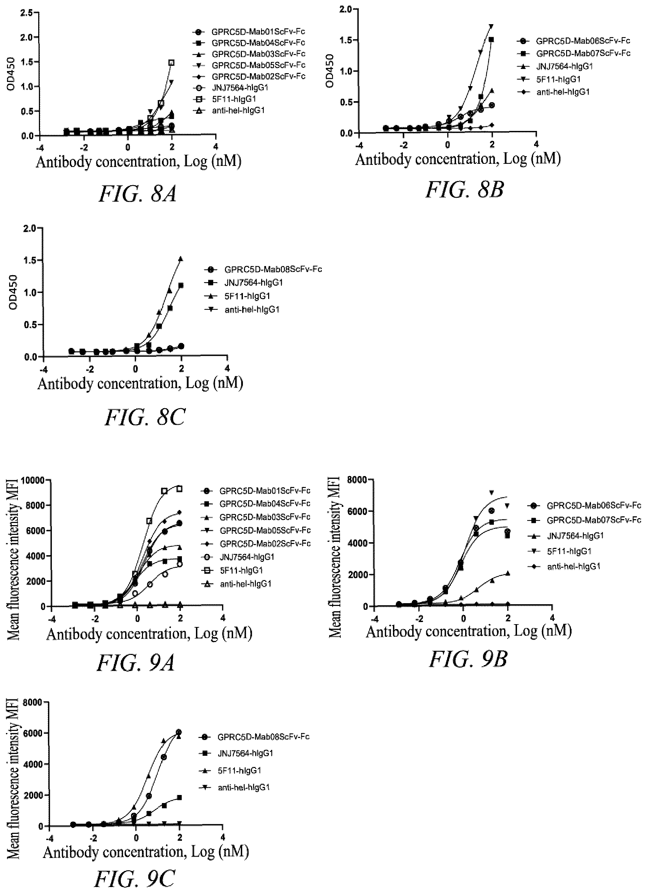

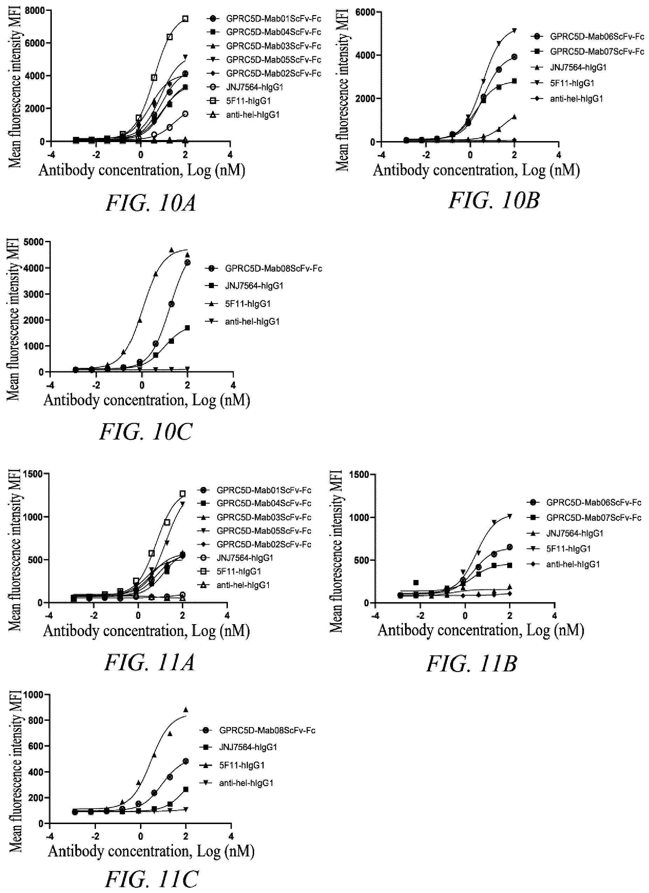

The desired cells were scale-up cultured in a T-75 cell culture flask to the logarithmic growth phase. NCI-H929, MolP-8 and RPMI-8226 cells were collected and washed with PBS buffer 2 times. After counting the cells, the cell pellet was resuspended with [PBS+2% (w/v) BSA] blocking solution to 2 × 10⁶ cells/ml, and added to a 96-well FACS reaction plate at 50 µl/well, and then the chimeric antibody test sample was added at 50 µl/well, and incubated on ice for 1 h. The obtained mixture was centrifuged and washed 3 times with a PBS buffer, Alexa Fluor^{®}647 AffiniPure Goat Anti-Human IgG, Fcγ fragment specific secondary antibody (purchased from Jackson, catalog No. 109-605-098) was added at 50 µl/well , and incubated on ice for 1 h. The obtained mixture was centrifuged and washed 3 times with a PBS buffer, and FACS (FACS CantoII, purchased from BD Company) was used for detection and result analysis. Data analysis was performed by software (Flowjo) to obtain the mean fluorescence intensity (MFI) of the cells. Then a software (GraphPad Prism8) was used for analysis, the analysis results are shown in Table 9 and Figs. 9A-9C, 10A-10C and 11A-11C. The chimeric antibodies can bind well to NCI-H929 (high expression), MolP-8 (medium expression), and RPMI-8226 (low expression) cells.

**Table 9 FACS detection of binding activity of chimeric antibodies with cells**

| **Cell** | **NCI-H929 (high expression)** | | **MoIP-8 (medium expression)** | | **RPMI-8226 (low expression)** | |
|---|---|---|---|---|---|---|
| **Designation of antibody** | **Maximum mean fluorescence intensity** | **EC50 (nM)** | **Maximum mean fluorescence intensity** | **EC50 (nM)** | **Maximum mean fluorescence intensity** | **EC50 (nM)** |
| GPRC5D-Mab01 scFv-Fc | 6501 | 2.20 | 4133 | 8.20 | 552 | 6.74 |
| GPRC5D-Mab04 scFv-Fc | 3692 | 0.72 | 3297 | 9.06 | 561 | 13.88 |
| GPRC5D-Mab03 scFv-Fc | 4585 | 1.09 | 4088 | 2.65 | 584 | 3.98 |
| GPRC5D-Mab05 scFv-Fc | 6453 | 1.82 | 5130 | 7.78 | 1144 | 17.22 |
| GPRC5D-Mab02 scFv-Fc | 7365 | 1.88 | 3344 | 10.28 | 535 | 2.20 |
| JNJ7564-hIgG1 | 3270 | 3.63 | 1672 | 31.80 | 90 | 38.42 |
| 5F11-hIgG1 | 9219 | 1.97 | 7482 | 4.07 | 1266 | 6.48 |
| anti-hel-hIgG1 | 74 | NB | 90 | NB | 85 | NB |
| GPRC5D-Mab06 scFv-Fc | 5968 | 0.73 | 3922 | 3.94 | 653 | 2.43 |
| GPRC5D-Mab07 scFv-Fc | 5241 | 0.77 | 2806 | 1.87 | 440 | 2.30 |
| JNJ7564-hIgG1 | 2043 | 4.94 | 1158 | 35.54 | 192 | 0.26 |
| 5F11-hIgG1 | 7082 | 1.25 | 5127 | 3.62 | 1010 | 3.17 |
| anti-hel-hIgG1 | 89 | NB | 81 | NB | 108 | NB |
| GPRC5D-Mab08 scFv-Fc | 5989 | 10.24 | 4209 | 17.23 | 481 | 8.51 |
| JNJ7564-hIgG1 | 1767 | 7.08 | 1696 | 9.63 | 265 | 89.28 |
| 5F11-hIgG1 | 5726 | 3.50 | 4694 | 1.09 | 884 | 2.86 |
| anti-hel-hIgG1 | 85 | NB | 103 | NB | 106 | NB |

| | | | | | | |
|---|---|---|---|---|---|---|
| Notes: NB means no binding. | | | | | | |

### 4.3 Flow cytometry experiment (FACS) to detect the binding activity of chimeric antibodies with CHOK1-hGPRC5A, CHOK1-hGPRC5B and CHOK1-hGPRC5C

The specific method referred to Example 4.2. CHOK1-hGPRC5A, CHOK1-hGPRC5B and CHOK1-hGPRCSC cells were taken. The results are shown in Table 10. The results show that the chimeric antibodies did not bind to CHOK1-hGPRC5A, CHOK1-hGPRC5B and CHOK1-hGPRC5C cells and had no cross-binding with these subtypes.

**Table 10 FACS detection of binding activity of chimeric antibodies with CHOK1-hGPRCSA, CHOK1-hGPRC5B and CHOK1-hGPRC5C cells**

| **Cell** | **CHOK1-hGPRC5A** | **CHOK1-hGPRC5B** | **CHOK1-hGPRC5C** |
|---|---|---|---|
| **Designation of antibody** | **Maximum mean fluorescence intensity** | **Maximum mean fluorescence intensity** | **Maximum mean fluorescence intensity** |
| GPRC5D-Mab01 scFv-Fc | 95 | 81 | 80 |
| GPRCSD-Mab04 scFv-Fc | 80 | 85 | 77 |
| GPRC5D-Mab03 scFv-Fc | 74 | 78 | 74 |
| GPRC5D-Mab05 scFv-Fc | 97 | 106 | 108 |
| GPRC5D-Mab02 scFv-Fc | 72 | 77 | 71 |
| JNJ7564-hIgG1 | 73 | 79 | 74 |
| 5F11-hIgG1 | 74 | 78 | 72 |
| Blank | 72 | 77 | 72 |
| anti-hGPRC5A-488 | 278 | \ | \ |
| anti-hGPRC5B-488 | \ | 306 | \ |
| anti-hGPRC5C-488 | \ | \ | 2889 |
| GPRC5D-Mab06 scFv-Fc | 76 | 53 | 45 |
| GPRC5D-Mab07 scFv-Fc | 86 | 48 | 42 |
| JNJ7564-hIgG1 | 75 | 51 | 45 |
| 5F11-hIgG1 | 73 | 50 | 44 |
| Blank | 70 | 48 | 42 |
| anti-hGPRC5A-488 | 316 | \ | \ |
| anti-hGPRC5B-488 | \ | 195 | \ |
| anti-hGPRC5C-488 | \ | \ | 1637 |
| GPRC5D-Mab08 scFv-Fc | 72 | 47 | 47 |
| hJNJ7564-hIgG1 | 73 | 49 | 49 |
| h5F11-hIgG1 | 69 | 49 | 46 |
| Blank | 69 | 49 | 47 |
| anti-hGPRC5A-488 | 323 | \ | \ |
| anti-hGPRC5B-488 | \ | 212 | \ |
| anti-hGPRC5C-488 | \ | \ | 1717 |

### 4.4 Identification of competitiveness of chimeric antibodies and positive control antibody 5F11

In order to identify the ability of the chimeric antibodies to bind to the antigen, a competitive ELISA method was used to analyze the competition between the GPRC5D chimeric antibodies and the positive control molecule 5F11 for binding to the antigen. The positive molecule 5F11 was labeled with biotin, 2 µg/mL hGPRC5D-LVP protein (manufacturer: Kactusbio, catalog No. GPR-HM05P) was used to coat ELISA plate. Biotin-5F11 antibody was serially diluted three-fold starting from 100 nM. The OD₄₅₀ value of the Biotin-5F11 antibody was determined, and GraphPad Prism software was used to fit the curve, and the EC80 concentration was calculated as the reference concentration in competitive ELISA.

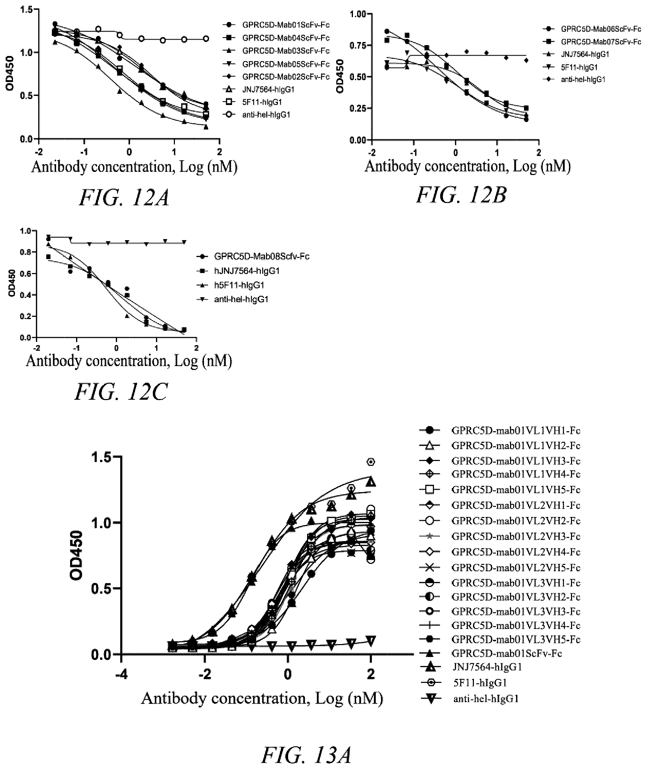

The hGPRC5D-LVP protein was diluted to 2 µg/mL to coat a 96-well high-adsorption ELISA plate at 50 µL/well. After overnight coating at 4°C, 250 µL of a blocking solution (PBS containing 2% (w/v) BSA) was used for blockage at room temperature for two hours. The chimeric antibodies serially diluted three-fold starting from 100 nM were added and then the Biotin-SF11 antibody with the concentration of EC80 (about 1 nM) was added and the mixture was incubated for 1.5 h. Washing was performed with PBS 5 times, and then HRP labeled Streptavidin Peroxidase Conjugate (purchased from sigma-millipore, catalog No. 189733) was added, and the mixture was incubated for 1 h, and the plate was washed 5 times. TMB substrate was added at 50 µL/well, and incubated at room temperature for 10 min, then a stop solution (1.0 M HCl) was added at 50 µL/well. An ELISA plate reader (Insight, purchased from PerkinElmer) was used to read the OD450nm value. According to the OD450nm value, the results are shown in Table 11 and Figs. 12A-12C, that is, the lower the value, the greater the competitiveness of the chimeric antibody. The results show that all 8 chimeric antibodies exhibited an epitope competition relationship with the positive molecule 5F11-hIgG1.

**Table 11 ELISA detection of competitive reaction of chimeric antibodies with positive control antibody 5F11**

| **Chimeric antibody** | **OD450** | | | | | | | |
|---|---|---|---|---|---|---|---|---|
| **Concentration (nM)** | **50.00** | **16.67** | **5.56** | **1.85** | **0.62** | **0.21** | **0.07** | **0** |
| GPRC5D-Mab01ScFv-Fc | 0.40 | 0.48 | 0.62 | 0.81 | 0.93 | 1.16 | 1.20 | 1.33 |
| GPRC5D-Mab04ScFv-Fc | 0.25 | 0.31 | 0.40 | 0.61 | 0.76 | 0.96 | 1.10 | 1.25 |
| GPRC5D-Mab03ScFv-Fc | 0.14 | 0.20 | 0.27 | 0.42 | 0.55 | 0.84 | 1.02 | 1.12 |
| GPRC5D-Mab05ScFv-Fc | 0.22 | 0.31 | 0.41 | 0.55 | 0.78 | 1.00 | 1.13 | 1.30 |
| GPRC5D-Mab02ScFv-Fc | 0.34 | 0.46 | 0.59 | 0.85 | 1.06 | 1.05 | 1.21 | 1.21 |
| JNJ7564-hIgG1 | 0.37 | 0.51 | 0.60 | 0.80 | 1.01 | 1.14 | 1.22 | 1.24 |
| 5F11-hIgG1 | 0.28 | 0.37 | 0.43 | 0.58 | 0.81 | 1.01 | 1.12 | 1.23 |
| anti-hel-hIgG1 | 1.16 | 1.15 | 1.14 | 1.16 | 1.20 | 1.27 | 1.24 | 1.22 |
| GPRC5D-Mab06ScFv-Fc | 0.16 | 0.19 | 0.29 | 0.37 | 0.48 | 0.66 | 0.78 | 0.86 |
| GPRC5D-Mab07ScFv-Fc | 0.25 | 0.29 | 0.37 | 0.47 | 0.59 | 0.73 | 0.83 | 0.79 |
| JNJ7564-hIgG1 | 0.20 | 0.28 | 0.36 | 0.45 | 0.58 | 0.62 | 0.58 | 0.60 |
| 5F11-hIgG1 | 0.18 | 0.23 | 0.29 | 0.38 | 0.46 | 0.56 | 0.69 | 0.61 |
| anti-hel-hIgG1 | 0.63 | 0.65 | 0.69 | 0.70 | 0.67 | 0.68 | 0.62 | 0.57 |
| GPRC5D-Mab08scFv-Fc | 0.06 | 0.10 | 0.18 | 0.45 | 0.51 | 0.64 | 0.61 | 0.91 |
| JNJ7564-hIgG1 | 0.07 | 0.09 | 0.14 | 0.39 | 0.49 | 0.57 | 0.66 | 0.75 |
| 5F11-hIgG1 | 0.07 | 0.08 | 0.12 | 0.20 | 0.50 | 0.62 | 0.75 | 0.87 |
| anti-hel-hIgG1 | 0.89 | 0.90 | 0.87 | 0.89 | 0.88 | 0.87 | 0.92 | 0.94 |

### Example 5 Humanization of GPRC5D murine antibody

### 5.1 Humanization of GPRC5D-mab01

By aligning the IMGT (http://imgt.cines.fr) human antibody heavy and light chain variable region germline gene database, heavy chain and light chain variable region germline genes having high homology to the murine antibody were selected as templates, respectively, and the CDRs of the murine antibody were grafted into the corresponding human template, to form a variable region sequence in the order of FR1-CDR1-FR2-CDR2-FR3-CDR3-FR4. According to needs, the key amino acids in the backbone sequence (FR region sequence) were back-mutated to the corresponding amino acids of the murine antibody to ensure the original affinity. At the same time, sites prone to chemical modification were present in the antibody, and point mutations have been made at these sites to eliminate the risk of modification, resulting in humanized monoclonal antibodies. The CDR amino acid residues of the antibody were determined and annotated by the Kabat numbering system.

The humanization light chain templates of the murine antibody GPRC5D-mab01 were IGKV1-9*01 and IGKJ4*01, and the humanization heavy chain templates were IGHV2-26*01 and IGHJ6*01. The specific mutation designs are shown in Table 12.

**Table 12 Mutation design of humanized antibody of GPRC5D-mab01**

| **VL** | | **VH** | |
|---|---|---|---|
| L1 | Graft(IGKV1-9*01) + L45P | H1 | Graft(IGHV2-26*01) + R97K |
| L2 | Graft(IGKV1-9*01) + L45P,L46W | H2 | Graft(IGHV2-26*01) + S30T,T73N,R97K |
| L3 | Graft(IGKV1-9*01) + A42S,L45P,L46W | H3 | Graft(IGHV2-26*01) + S30T,S70R,T73N,R97K |
| | | H4 | Graft(IGHV2-26*01) + S30T,T73N,V79F,R97K |
| | | H5 | Graft(IGHV2-26*01) + S30T,T73N,R97K,Q114H |

Notes: Graft represents the grafting of the CDRs of the murine antibody into the FR region sequence of the human germline template; L45P represents the mutation of L at position 45 of Graft into P, and so on. The numbering of mutated amino acids is according to the natural sequence numbering.

The specific sequences of the variable regions of the humanized antibodies of GPRC5D-mab01 are as follows:
The amino acid sequence of GPRC5D-mab01.VL1 is as shown in SEQ ID NO: 118: 
DIQLTQSPSFLSASVGDRVTITCRASSSVSYMHWYQQKPGKAPKPLIYATSHLASGVPSRFSGS
GSGTEFTLTISSLQPEDFATYYCQQWSSNPPMTFGGGTKVEIK
The amino acid sequence of GPRC5D-mab01.VL2 is as shown in SEQ ID NO: 119: 
DIQLTQSPSFLSASVGDRVTITCRASSSVSYMHWYQQKPGKAPKPWIYATSHLASGVPSRFSG
SGSGTEFTLTISSLQPEDFATYYCQQWSSNPPMTFGGGTKVEIK
The amino acid sequence of GPRC5D-mab01.VL3 is as shown in SEQ ID NO: 120: 
DIQLTQSPSFLSASVGDRVTITCRASSSVSYMHWYQQKPGKSPKPWIYATSHLASGVPSRFSG
SGSGTEFTLTISSLQPEDFATYYCQQWSSNPPMTFGGGTKVEIK
The amino acid sequence of GPRC5D-mab01.VH1 is as shown in SEQ ID NO: 121: 
EVTLKESGPVLVKPTETLTLTCTVSGFSLSNYGVSWIRQPPGKALEWLAVIWGDGSTNYHSAL
ISRLTISKDTSKSQVVLTMTNMDPVDTATYYCAKPASYYGRRTYAMDFWGQGTTVTVSS
The amino acid sequence of GPRC5D-mab01.VH2 is as shown in SEQ ID NO: 122: 
EVTLKESGPVLVKPTETLTLTCTVSGFSLTNYGVSWIRQPPGKALEWLAVIWGDGSTNYHSAL
ISRLTISKDNSKSQVVLTMTNMDPVDTATYYCAKPASYYGRRTYAMDFWGQGTTVTVSS
The amino acid sequence of GPRC5D-mab01.VH3 is as shown in SEQ ID NO: 123: 
EVTLKESGPVLVKPTETLTLTCTVSGFSLTNYGVSWIRQPPGKALEWLAVIWGDGSTNYHSAL
ISRLTIRKDNSKSQVVLTMTNMDPVDTATYYCAKPASYYGRRTYAMDFWGQGTTVTVSS
The amino acid sequence of GPRC5D-mab01.VH4 is as shown in SEQ ID NO: 124: 
EVTLKESGPVLVKPTETLTLTCTVSGFSLTNYGVSWIRQPPGKALEWLAVIWGDGSTNYHSAL
ISRLTISKDNSKSQVFLTMTNMDPVDTATYYCAKPASYYGRRTYAMDFWGQGTTVTVSS
The amino acid sequence of GPRC5D-mab01.VH5 is as shown in SEQ ID NO: 125: 
EVTLKESGPVLVKPTETLTLTCTVSGFSLTNYGVSWIRQPPGKALEWLAVIWGDGSTNYHSAL
ISRLTISKDNSKSQVVLTMTNMDPVDTATYYCAKPASYYGRRTYAMDFWGHGTTVTVSS
The amino acid sequence of humanization light chain template IGKV1-9*01 is as shown in SEQ ID NO: 126: 
DIQLTQSPSFLSASVGDRVTITCRASQGISSYLAWYQQKPGKAPKLLIYAASTLQSGVPSRFSG
SGSGTEFTLTISSLQPEDFATYYCQQLNSYP
The amino acid sequence of humanization light chain template IGKJ4*01 is as shown in SEQ ID NO: 127:
   *FGGGTKVEIK*
The amino acid sequence of humanization heavy chain template IGHV2-26*01 is as shown in SEQ ID NO: 128: 
QVTLKESGPVLVKPTETLTLTCTVSGFSLSNARMGVSWIRQPPGKALEWLAHIFSNDEKSYST
SLKSRLTISKDTSKSQVVLTMTNMDPVDTATYYCARI
The amino acid sequence of humanization heavy chain template IGHJ6*01 is as shown in SEQ ID NO: 129:
   *WGQGTTVTVSS*

In the present application, different light chain and heavy chain sequences were selected from the above-mentioned mutation design of the light chain and heavy chain variable regions of the humanized antibody of GPRC5D-mab01 for cross combination, respectively, and finally a plurality of humanized antibodies of GPRC5D-mab01 were obtained. The amino acid sequences of the variable regions of each antibody are shown in Table 13 below:

**Table 13 The corresponding amino acid sequences of the variable regions of GPRC5D-mab01 antibody**

| | GPRC5D-mab01.VH1 | GPRC5D-mab01.VH2 | GPRC5D-mab01.VH3 | GPRC5D-mab01.VH4 | GPRC5D-mab01.VH5 |
|---|---|---|---|---|---|
| GPRC5D-mab01.VL1 | GPRC5D-mab01VL1VH1 | GPRC5D-mab01VL1VH2 | GPRC5D-mab01VL1VH3 | GPRC5D-mab01VL1VH4 | GPRC5D-mab01VL1VH5 |
| GPRC5D-mab01.VL2 | GPRC5D-mab01VL2VH1 | GPRC5D-mab01VL2VH2 | GPRC5D-mab01VL2VH3 | GPRC5D-mab01VL2VH4 | GPRC5D-mab01VL2VH5 |
| GPRC5D-mab01.VL3 | GPRC5D-mab01VL3VH1 | GPRC5D-mab01VL3VH2 | GPRC5D-mab01VL2VH3 | GPRC5D-mab01VL3VH4 | GPRC5D-mab01VL3VH5 |

According to the Kabat numbering system, the analysis results of the VH and VL sequences of the above-mentioned humanized antibodies are shown in Table 14.

**Table 14 Kabat analysis results of the VH and VL sequences of the humanized antibodies of GPRC5D-mab01**

| **Variable region** | **CDR1** | **CDR2** | **CDR3** |
|---|---|---|---|
| GPRC5D-mab01.VL1 | RASSSVSYMH (SEQ ID NO: 35) | ATSHLAS (SEQ ID NO: 36) | QQWSSNPPMT (SEQ ID NO: 37) |
| GPRC5D-mab01.VL2 | | | |
| GPRC5D-mab01.VL3 | | | |
| GPRC5D-mab01.VH1 | NYGVS (SEQ ID NO: 29) | VIWGDGSTNYHSALIS (SEQ ID NO: 30) | PASYYGRRTYAMDF (SEQ ID NO: 31) |
| GPRC5D-mab01.VH2 | | | |
| GPRC5D-mab01.VH3 | | | |
| GPRC5D-mab01.VH4 | | | |
| GPRC5D-mab01.VH5 | | | |

### 5.2 Humanization of GPRC5D-mab02

The method was the same as 5.1. The humanization light chain templates of the murine antibody GPRC5D-mab02 were IGKV3-20*02 and IGKJ2*01, and the humanization heavy chain templates were IGHV1-46*01 and IGHJ6*01. The specific mutation designs are shown in Table 15.

**Table 15 Mutation design of humanized antibody of GPRC5D-mab02**

| **VL** | | **VH** | |
|---|---|---|---|
| L1 | Graft(IGKV3-20*02) + L45V | H1 | Graft(IGHV1-46*01) + T30S,R72V |
| L2 | Graft(IGKV3-20*02)+A42S,L45V | H2 | Graft(IGHV1-46*01) + T30S,R72V,V79A |
| | | H2a | Graft(IGHV1-46*01) + T30S,R72V,V79A + N55S |
| | | H3 | Graft(IGHV1-46*01) + T30S,M70L,R72V,V79A |
| | | H3a | Graft(IGHV1-46*01) + T30S,M70L,R72V,V79A + G56A |
| | | H4 | Graft(IGHV1-46*01) + V5Q,T30S,R72V,V79A |

Notes: Graft represents the grafting of the CDRs of the murine antibody into the FR region sequence of the human germline template; L45V represents the mutation of L at position 45 of Graft into V, and so on. The numbering of mutated amino acids is according to the natural sequence numbering.

The specific sequences of the variable regions of the humanized antibodies of GPRC5D-mab02 are as follows:
The amino acid sequence of GPRCSD-mab02.VL1 is as shown in SEQ ID NO: 130: 
EIVLTQSPATLSLSPGERATLSCSAGSSVSYMYWYQQKPGQAPRVLIYDTSNLASGIPARFSGS
GSGTDFTLTISRLEPEDFAVYYCQQWSNYPLTFGQGTKLEIK
The amino acid sequence of GPRC5D-mab02.VL2 is as shown in SEQ ID NO: 131: 
EIVLTQSPATLSLSPGERATLSCSAGSSVSYMYWYQQKPGQSPRVLIYDTSNLASGIPARFSGSG
SGTDFTLTISRLEPEDFAVYYCQQWSNYPLTFGQGTKLEIK
The amino acid sequence of GPRCSD-mab02.VH1 is as shown in SEQ ID NO: 132: 
EVQLVQSGAEVKKPGASVKVSCKASGYTFSSYWITWVRQAPGQGLEWMGDFYPRNGDTNY
NKKFKNRVTMTVDTSTSTVYMELSSLRSEDTAVYYCARSRAYYGRRGFDYWGQGTTVTVSS
The amino acid sequence of GPRCSD-mab02.VH2 is as shown in SEQ ID NO: 133: 
EVQLVQSGAEVKKPGASVKVSCKASGYTFSSYWITWVRQAPGQGLEWMGDFYPRNGDTNY
NKKFKNRVTMTVDTSTSTAYMELSSLRSEDTAVYYCARSRAYYGRRGFDYWGQGTTVTVSS
The amino acid sequence of GPRCSD-mab02.VH2a is as shown in SEQ ID NO: 134: 
EVQLVQSGAEVKKPGASVKVSCKASGYTFSSYWITWVRQAPGQGLEWMGDFYPRSGDTNY
NKKFKNRVTMTVDTSTSTAYMELSSLRSEDTAVYYCARSRAYYGRRGFDYWGQGTTVTVSS
The amino acid sequence of GPRC5D-mab02.VH3 is as shown in SEQ ID NO: 135: 
EVQLVQSGAEVKKPGASVKVSCKASGYTFSSYWITWVRQAPGQGLEWMGDFYPRNGDTNY
NKKFKNRVTLTVDTSTSTAYMELSSLRSEDTAVYYCARSRAYYGRRGFDYWGQGTTVTVSS
The amino acid sequence of GPRC5D-mab02.VH3a is as shown in SEQ ID NO: 136: 
EVQLVQSGAEVKKPGASVKVSCKASGYTFSSYWITWVRQAPGQGLEWMGDFYPRNADTNY
NKKFKNRVTLTVDTSTSTAYMELSSLRSEDTAVYYCARSRAYYGRRGFDYWGQGTTVTVSS
The amino acid sequence of GPRCSD-mab02.VH4 is as shown in SEQ ID NO: 137: 
EVQLQQSGAEVKKPGASVKVSCKASGYTFSSYWITWVRQAPGQGLEWMGDFYPRNGDTNY
NKKFKNRVTMTVDTSTSTAYMELSSLRSEDTAVYYCARSRAYYGRRGFDYWGQGTTVTVSS
The amino acid sequence of humanization light chain template IGKV3-20*02 is as shown in SEQ ID NO: 138: 
EIVLTQSPATLSLSPGERATLSCRASQSVSSSYLAWYQQKPGQAPRLLIYGASSRATGIPARFSG
SGSGTDFTLTISRLEPEDFAVYYCQ
The amino acid sequence of humanization light chain template IGKJ2*01 is as shown in SEQ ID NO: 139:
   *FGQGTKLEIK*
The amino acid sequence of humanization heavy chain template IGHV 1-46*01 is as shown in SEQ ID NO: 140: 
QVQLVQSGAEVKKPGASVKVSCKASGYTFTSYYMHWVRQAPGQGLEWMGIINPSGGSTSYA
QKFQGRVTMTRDTSTSTVYMELSSLRSEDTAVYYCAR
The amino acid sequence of humanization heavy chain template IGHJ6*01 is as shown in SEQ ID NO: 141:
   *WGQGTTVTVSS*

In the present application, different light chain and heavy chain sequences were selected from the above-mentioned mutation design of the light chain and heavy chain variable regions of the humanized antibody of GPRC5D-mab02 for cross combination, respectively, and finally a plurality of humanized antibodies of GPRC5D-mab02 were obtained. The amino acid sequences of the variable regions of each antibody are shown in Table 16 below:

**Table 16 The corresponding amino acid sequences of the variable regions of GPRCSD-mab02 antibody**

| | GPRC5D-mab02.VH1 | GPRC5D-mab02.VH2 | GPRC5D-mab02.VH2a | GPRC5D-mab02.VH3 | GPRC5D-mab02.VH3a | GPRC5D-mab02.VH4 |
|---|---|---|---|---|---|---|
| GPRC5D-mab02.VL1 | GPRC5D-mab02VL1VH1 | GPRC5D-mab02VL1VH2 | GPRC5D-mab02VL1VH2a | GPRC5D-mab02VL1VH3 | GPRC5D-mab02VL1VH3a | GPRC5D-mab02VL1VH4 |
| GPRC5D-mab02.VL2 | GPRC5D-mab02VL2VH1 | GPRC5D-mab02VL2VH2 | GPRC5D-mab02VL2VH2a | GPRC5D-mab02VL2VH3 | GPRC5D-mab02VL2VH3a | GPRC5D-mab02VL2VH4 |

According to the Kabat numbering system, the analysis results of the VH and VL sequences of the above-mentioned humanized antibodies are shown in Table 17.

**Table 17 Kabat analysis results of the VH and VL sequences of the humanized antibodies of GPRCSD-mab02**

| **Variable region** | **CDR1** | **CDR2** | **CDR3** |
|---|---|---|---|
| GPRC5D-mab02.VL1 | SAGSSVSYMY (SEQ ID NO: 46) | DTSNLAS (SEQ ID NO: 47) | QQWSNYPLT (SEQ ID NO: 48) |
| GPRC5D-mab02.VL2 | | | |
| GPRC5D-mab02.VH1 | SYWIT (SEQ ID NO: 40) | DFYPRNGDTNYNKKFKN (SEQ ID NO: 41) | SRAYYGRRGFDY (SEQ ID NO: 42) |
| GPRCSD-mab02.VH2 | | | |
| GPRC5D-mab02.VH3 | | | |
| GPRC5D-mab02.VH4 | | | |
| GPRCSD-mab02.VH2a | SYWIT (SEQ ID NO: 40) | DFYPRSGDTNYNKKFKN (SEQ ID NO: 142) | SRAYYGRRGFDY (SEQ ID NO: 42) |
| GPRC5D-mab02.VH3a | SYWIT (SEQ ID NO: 40) | DFYPRNADTNYNKKFKN (SEQ ID NO: 143) | SRAYYGRRGFDY (SEQ ID NO: 42) |

### 5.3 Humanization of GPRC5D-mab03

The method was the same as 5.1. The humanization light chain templates of the murine antibody GPRC5D-mab03 were IGKV4-1*01/IGKV1-39*01 and IGKJ4*01, and the humanization heavy chain templates were IGHV1-3*01 and IGHJ6*01. The specific mutation designs are shown in Table 18.

**Table 18 Mutation design of humanized antibody of GPRCSD-mab03**

| **VL** | | **VH** | |
|---|---|---|---|
| L1 | Graft(IGKV1-39*01) + A43S,Y49S | H1 | Graft(IGHV1-3*01) + R72V,T74K |
| L2 | Graft(IGKV4-1*01) + P43S,Y49S | H2 | Graft(IGHV1-3*01) + V2A,R72V,T74K |
| | | H2a | Graft(IGHV1-3*01) + V2A,R72V,T74K + N55S |
| | | H3 | Graft(IGHV1-3*01) + V2A,I70L,R72V,T74K |
| | | H4 | Graft(IGHV1-3*01) + V2A,Q3Y,V5Q,R72V,T74K |
| | | H4a | Graft(IGHV1-3*01) + V2A,Q3Y,V5Q,R72V,T74K + G56A |
| | | H5 | Graft(IGHV1-3*01) + V2A,G42R,R44G,R72V,T74K |

Notes: Graft represents the grafting of the CDRs of the murine antibody into the FR region sequence of the human germline template; A43S represents the mutation of A at position 43 of Graft into S, and so on. The numbering of mutated amino acids is according to the natural sequence numbering.

The specific sequences of the variable regions of the humanized antibodies of GPRC5D-mab03 are as follows:
The amino acid sequence of GPRC5D-mab03.VL1 is as shown in SEQ ID NO: 144: 
DIQMTQSPSSLSASVGDRVTITCKASQNVGTAVAWYQQKPGKSPKLLISSASNRYTGVPSRFS
GSGSGTDFTLTISSLQPEDFATYYCQQYSSYPWTFGGGTKVEIK
The amino acid sequence of GPRC5D-mab03.VL2 is as shown in SEQ ID NO: 145: 
DIVMTQSPDSLAVSLGERATINCKASQNVGTAVAWYQQKPGQSPKLLISSASNRYTGVPDRFS
GSGSGTDFTLTISSLQAEDVAVYYCQQYSSYPWTFGGGTKVEIK
The amino acid sequence of GPRCSD-mab03.VH1 is as shown in SEQ ID NO: 146: 
EVQLVQSGAEVKKPGASVKVSCKASGYTFTSYNMNWVRQAPGQRLEWMGAIYPGNGDTSY
NQKFKGRVTITVDKSASTAYMELSSLRSEDTAVYYCARVRLRYAMDYWGQGTTVTVSS
The amino acid sequence of GPRCSD-mab03.VH2 is as shown in SEQ ID NO: 147: 
EAQLVQSGAEVKKPGASVKVSCKASGYTFTSYNMNWVRQAPGQRLEWMGAIYPGNGDTSY
NQKFKGRVTITVDKSASTAYMELSSLRSEDTAVYYCARVRLRYAMDYWGQGTTVTVSS
The amino acid sequence of GPRCSD-mab03.VH2a is as shown in SEQ ID NO: 148: 
EAQLVQSGAEVKKPGASVKVSCKASGYTFTSYNMNWVRQAPGQRLEWMGAIYPGSGDTSY
NQKFKGRVTITVDKSASTAYMELSSLRSEDTAVYYCARVRLRYAMDYWGQGTTVTVSS
The amino acid sequence of GPRCSD-mab03.VH3 is as shown in SEQ ID NO: 149: 
EAQLVQSGAEVKKPGASVKVSCKASGYTFTSYNMNWVRQAPGQRLEWMGAIYPGNGDTSY
NQKFKGRVTLTVDKSASTAYMELSSLRSEDTAVYYCARVRLRYAMDYWGQGTTVTVSS
The amino acid sequence of GPRCSD-mab03.VH4 is as shown in SEQ ID NO: 150: 
EAYLQQSGAEVKKPGASVKVSCKASGYTFTSYNMNWVRQAPGQRLEWMGAIYPGNGDTSY
NQKFKGRVTITVDKSASTAYMELSSLRSEDTAVYYCARVRLRYAMDYWGQGTTVTVSS
The amino acid sequence of GPRC5D-mab03.VH4a is as shown in SEQ ID NO: 151: 
EAYLQQSGAEVKKPGASVKVSCKASGYTFTSYNMNWVRQAPGQRLEWMGAIYPGNADTSY
NQKFKGRVTITVDKSASTAYMELSSLRSEDTAVYYCARVRLRYAMDYWGQGTTVTVSS
The amino acid sequence of GPRC5D-mab03.VH5 is as shown in SEQ ID NO: 152: 
EAQLVQSGAEVKKPGASVKVSCKASGYTFTSYNMNWVRQAPRQGLEWMGAIYPGNGDTSY
NQKFKGRVTITVDKSASTAYMELSSLRSEDTAVYYCARVRLRYAMDYWGQGTTVTVSS
The amino acid sequence of humanization light chain template IGKV4-1*01 is as shown in SEQ ID NO: 153: 
DIVMTQSPDSLAVSLGERATINCKSSQSVLYSSNNKNYLAWYQQKPGQPPKLLIYWASTRESG
VPDRFSGSGSGTDFTLTISSLQAEDVAVYYCQQYYSTP
The amino acid sequence of humanization light chain template IGKV1-39*01 is as shown in SEQ ID NO: 154: 
DIQMTQSPSSLSASVGDRVTITCRASQSISSYLNWYQQKPGKAPKLLIYAASSLQSGVPSRFSG
SGSGTDFTLTISSLQPEDFATYYCQQSYSTP
The amino acid sequence of humanization light chain template IGKJ4*01 is as shown in SEQ ID NO: 155:
   *FGGGTKVEIK*
The amino acid sequence of humanization heavy chain template IGHV1-3*01 is as shown in SEQ ID NO: 156: 
QVQLVQSGAEVKKPGASVKVSCKASGYTFTSYAMHWVRQAPGQRLEWMGWINAGNGNTK
YSQKFQGRVTITRDTSASTAYMELSSLRSEDTAVYYCAR
The amino acid sequence of humanization heavy chain template IGHJ6*01 is as shown in SEQ ID NO: 157:
   *WGQGTTVTVSS*

In the present application, different light chain and heavy chain sequences were selected from the above-mentioned mutation design of the light chain and heavy chain variable regions of the humanized antibody of GPRC5D-mab03 for cross combination, respectively, and finally a plurality of humanized antibodies of GPRC5D-mab03 were obtained. The amino acid sequences of the variable regions of each antibody are shown in Table 19 below:

**Table 19 The corresponding amino acid sequences of the variable regions of GPRCSD-mab03 antibody**

| | GPRC5D-mab03.VH1 | GPRC5D-mab03.V H2 | GPRC5D-mab03.VH2a | GPRC5D-mab03.V H3 | GPRC5D-mab03.V H4 | GPRC5D-mab03.VH4a | GPRC5D-mab03.V H5 |
|---|---|---|---|---|---|---|---|
| GPRC5D-mab03.VL1 | GPRC5D-mab03VL1 VH1 | GPRC5D-mab03VL 1VH2 | GPRC5D-mab03VL1V H2a | GPRC5D-mab03VL 1VH3 | GPRC5D-mab03VL 1VH4 | GPRC5D-mab03VL1V H4a | GPRC5D-mab03VL 1VH5 |
| GPRC5D-mab03.VL2 | GPRC5D-mab03VL2 VH1 | GPRC5D-mab03VL 2VH2 | GPRC5D-mab03VL2V H2a | GPRC5D-mab03VL 2VH3 | GPRC5D-mab03VL 2VH4 | GPRC5D-mab03VL2V H4a | GPRC5D-mab03VL 2VH5 |

According to the Kabat numbering system, the analysis results of the VH and VL sequences of the above-mentioned humanized antibodies are shown in Table 20.

**Table 20 Kabat analysis results of the VH and VL sequences of the humanized antibodies of GPRCSD-mab03**

| **Variable region** | **CDR1** | **CDR2** | **CDR3** |
|---|---|---|---|
| GPRCSD-mab03.VL1 | KASQNVGTAVA (SEQ ID NO: 57) | SASNRYT (SEQ ID NO: 58) | QQYSSYPWT (SEQ ID NO: 59) |
| GPRCSD-mab03.VL2 | | | |
| GPRCSD-mab03.VH1 | SYNMN (SEQ ID NO: 51) | AIYPGNGDTSYNQKFKG (SEQ ID NO: 52) | VRLRYAMDY (SEQ ID NO: 53) |
| GPRCSD-mab03.VH2 | | | |
| GPRCSD-mab03.VH3 | | | |
| GPRCSD-mab03.VH4 | | | |
| GPRCSD-mab03.VH5 | | | |
| GPRC5D-mab03.VH2a | SYNMN (SEQ ID NO: 51) | AIYPGSGDTSYNQKFKG (SEQ ID NO: 158) | VRLRYAMDY (SEQ ID NO: 53) |
| GPRC5D-mab03.VH4a | SYNMN (SEQ ID NO: 51) | AIYPGNADTSYNQKFKG (SEQ ID NO: 159) | VRLRYAMDY (SEQ ID NO: 53) |

### 5.4 Humanization of GPRC5D-mab04

The method was the same as 5.1. The humanization light chain templates of the murine antibody GPRC5D-mab04 were IGKV1-39*01/IGKV2-28*01 and IGKJ4*01, and the humanization heavy chain templates were IGHV3-7*01 and IGHJ6*01. The specific mutation designs are shown in Table 21.

**Table 21 Mutation design of humanized antibody of GPRCSD-mab04**

| **VL** | | **VH** | |
|---|---|---|---|
| L1 | Graft(IGKV1-39*01) + A43S,I48V | H1 | Graft(IGHV3-7*01) |
| L2 | Graft(IGKV1-39*01) + A43S,K45Q,I48V | H2 | Graft(IGHV3-7*01) + G26R |
| L3 | Graft(IGKV2-28*01) + 148V | H3 | Graft(IGHV3-7*01) + G26R,G42E,G44R |
| L4 | Graft(IGKV2-28*01) + P40Q,I48V | | |

Notes: Graft represents the grafting of the CDRs of the murine antibody into the FR region sequence of the human germline template; A43S represents the mutation of A at position 43 of Graft into S, and so on. The numbering of mutated amino acids is according to the natural sequence numbering.

The specific sequences of the variable regions of the humanized antibodies of GPRC5D-mab04 are as follows:
The amino acid sequence of GPRC5D-mab04.VL1 is as shown in SEQ ID NO: 160: 
DIQMTQSPSSLSASVGDRVTITCRASENIYRNLAWYQQKPGKSPKLLVYAATNLADGVPSRFS
GSGSGTDFTLTISSLQPEDFATYYCQHFWGTPRTFGGGTKVEIK
The amino acid sequence of GPRC5D-mab04.VL2 is as shown in SEQ ID NO: 161: 
DIQMTQSPSSLSASVGDRVTITCRASENIYRNLAWYQQKPGKSPQLLVYAATNLADGVPSRFS
GSGSGTDFTLTISSLQPEDFATYYCQHFWGTPRTFGGGTKVEIK
The amino acid sequence of GPRC5D-mab04.VL3 is as shown in SEQ ID NO: 162: 
DIVMTQSPLSLPVTPGEPASISCRASENIYRNLAWYLQKPGQSPQLLVYAATNLADGVPDRFS
GSGSGTDFTLKISRVEAEDVGVYYCQHFWGTPRTFGGGTKVEIK
The amino acid sequence of GPRC5D-mab04.VL4 is as shown in SEQ ID NO: 163: 
DIVMTQSPLSLPVTPGEPASISCRASENIYRNLAWYLQKQGQSPQLLVYAATNLADGVPDRFS
GSGSGTDFTLKISRVEAEDVGVYYCQHFWGTPRTFGGGTKVEIK
The amino acid sequence of GPRCSD-mab04.VH1 is as shown in SEQ ID NO: 164: 
EVQLVESGGGLVQPGGSLRLSCAASGFTFSRYAMSWVRQAPGKGLEWVATISDGGSYTYYP
DNVKGRFTISRDNAKNSLYLQMNSLRAEDTAVYYCARDRYYYGKGGYFDVWGQGTTVTVSS
The amino acid sequence of GPRCSD-mab04.VH2 is as shown in SEQ ID NO: 165: 
EVQLVESGGGLVQPGGSLRLSCAASRFTFSRYAMSWVRQAPGKGLEWVATISDGGSYTYYPD
NVKGRFTISRDNAKNSLYLQMNSLRAEDTAVYYCARDRYYYGKGGYFDVWGQGTTVTVSS
The amino acid sequence of GPRC5D-mab04.VH3 is as shown in SEQ ID NO: 166: 
EVQLVESGGGLVQPGGSLRLSCAASRFTFSRYAMSWVRQAPEKRLEWVATISDGGSYTYYPD
NVKGRFTISRDNAKNSLYLQMNSLRAEDTAVYYCARDRYYYGKGGYFDVWGQGTTVTVSS
The amino acid sequence of humanization light chain template IGKV1-39*01 is as shown in SEQ ID NO: 167: 
DIQMTQSPSSLSASVGDRVTITCRASQSISSYLNWYQQKPGKAPKLLIYAASSLQSGVPSRFSG
SGSGTDFTLTISSLQPEDFATYYCQQSYSTP
The amino acid sequence of humanization light chain template IGKV2-28*01 is as shown in SEQ ID NO: 168: 
DIVMTQSPLSLPVTPGEPASISCRSSQSLLHSNGYNYLDWYLQKPGQSPQLLIYLGSNRASGV
PDRFSGSGSGTDFTLKISRVEAEDVGVYYCMQALQTP
The amino acid sequence of humanization light chain template IGKJ4*01 is as shown in SEQ ID NO: 169:
   *FGGGTKVEIK*
The amino acid sequence of humanization heavy chain template IGHV3-7*01 is as shown in SEQ ID NO: 170: 
EVQLVESGGGLVQPGGSLRLSCAASGFTFSSYWMSWVRQAPGKGLEWVANIKQDGSEKYY
VDSVKGRFTISRDNAKNSLYLQMNSLRAEDTAVYYCAR
The amino acid sequence of humanization heavy chain template IGHJ6*01 is as shown in SEQ ID NO: 171:
   *WGQGTTVTVSS*

In the present application, different light chain and heavy chain sequences were selected from the above-mentioned mutation design of the light chain and heavy chain variable regions of the humanized antibody of GPRC5D-mab04 for cross combination, respectively, and finally a plurality of humanized antibodies of GPRC5D-mab04 were obtained. The amino acid sequences of the variable regions of each antibody are shown in Table 22 below:

**Table 22 The corresponding amino acid sequences of the variable regions of GPRCSD-mab04 antibody**

| | GPRC5D-mab04.VH1 | GPRCSD-mab04.VH2 | GPRCSD-mab04.VH3 |
|---|---|---|---|
| GPRCSD-mab04.VL1 | GPRC5D-mab04VL1VH1 | GPRC5D-mab04VL1VH2 | GPRC5D-mab04VL1VH3 |
| GPRC5D-mab04.VL2 | GPRC5D-mab04VL2VH1 | GPRC5D-mab04VL2VH2 | GPRC5D-mab04VL2VH3 |
| GPRCSD-mab04.VL3 | GPRC5D-mab04VL3VH1 | GPRC5D-mab04VL3VH2 | GPRC5D-mab04VL3VH3 |
| GPRCSD-mab04.VL4 | GPRC5D-mab04VL4VH1 | GPRC5D-mab04VL4VH2 | GPRC5D-mab04VL4VH3 |

According to the Kabat numbering system, the analysis results of the VH and VL sequences of the above-mentioned humanized antibodies are shown in Table 23.

**Table 23 Kabat analysis results of the VH and VL sequences of the humanized antibodies of GPRCSD-mab04**

| **Variable region** | **CDR1** | **CDR2** | **CDR3** |
|---|---|---|---|
| GPRC5D-mab04.VL1 | RASENIYRNLA (SEQ ID NO: 68) | AATNLAD (SEQ ID NO: 69) | QHFWGTPRT (SEQ ID NO: 70) |
| GPRC5D-mab04.VL2 | | | |
| GPRC5D-mab04.VL3 | | | |
| GPRC5D-mab04.VL4 | | | |
| GPRC5D-mab04.VH1 | RYAMS (SEQ ID NO: 62) | TISDGGSYTYYPDNVKG (SEQ ID NO: 63) | DRYYYGKGGYFDV (SEQ ID NO: 64) |
| GPRC5D-mab04.VH2 | | | |
| GPRC5D-mab04.VH3 | | | |

### 5.5 Humanization of GPRC5D-mab05

The method was the same as 5.1. The humanization light chain templates of the murine antibody GPRC5D-mab05 were IGKV6-21*01/IGKV3-11*01 and IGKJ4*01, and the humanization heavy chain templates were IGHV1-69*02 and IGHJ6*01. The specific mutation designs are shown in Table 24.

**Table 24 Mutation design of humanized antibody of GPRCSD-mab05**

| **VL** | | **VH** | |
|---|---|---|---|
| L1 | Graft(IGKV6-21 *01) + V58I | H1 | Graft(IGHV1-69*02) + G27Y |
| L2 | Graft(IGKV6-21*01) + Q42G,V581 | H2 | Graft(IGHV1-69*02) + G27Y,S30T |
| L3 | Graft(IGKV6-21*01) + P40T,Q42G,V58I | H3 | Graft(IGHV1-69*02) + G27Y,S30T,I70L |
| L4 | Graft(IGKV3-11*01) + A43S,Y49K | | |

Notes: Graft represents the grafting of the CDRs of the murine antibody into the FR region sequence of the human germline template; V58I represents the mutation of V at position 58 of Graft into I, and so on. The numbering of mutated amino acids is according to the natural sequence numbering.

The specific sequences of the variable regions of the humanized antibodies of GPRC5D-mab05 are as follows:
The amino acid sequence of GPRC5D-mab05. VL1 is as shown in SEQ ID NO: 172: 
EIVLTQSPDFQSVTPKEKVTITCRASQSIGTSIHWYQQKPDQSPKLLIKYASESISGIPSRFSGS
GSGTDFTLTINSLEAEDAATYYCQQSNSWPYTFGGGTKVEIK
The amino acid sequence of GPRC5D-mab05.VL2 is as shown in SEQ ID NO: 173: 
EIVLTQSPDFQSVTPKEKVTITCRASQSIGTSIHWYQQKPDGSPKLLIKYASESISGIPSRFSGS
GSGTDFTLTINSLEAEDAATYYCQQSNSWPYTFGGGTKVEIK
The amino acid sequence of GPRC5D-mab05.VL3 is as shown in SEQ ID NO: 174: 
EIVLTQSPDFQSVTPKEKVTITCRASQSIGTSIHWYQQKTDGSPKLLIKYASESISGIPSRFSGS
GSGTDFTLTINSLEAEDAATYYCQQSNSWPYTFGGGTKVEIK
The amino acid sequence of GPRC5D-mab05.VL4 is as shown in SEQ ID NO: 175: 
EIVLTQSPATLSLSPGERATLSCRASQSIGTSIHWYQQKPGQSPRLLIKYASESISGIPARFSGSG
SGTDFTLTISSLEPEDFAVYYCQQSNSWPYTFGGGTKVEIK
The amino acid sequence of GPRC5D-mab05.VH1 is as shown in SEQ ID NO: 176: 
EVQLVQSGAEVKKPGSSVKVSCKASGYTFSSYGISWVRQAPGQGLEWMGEIYPRSGNTYYN
EKFKGRVTITADKSTSTAYMELSSLRSEDTAVYYCARGRAYGRNYPMDYWGQGTTVTVSS
The amino acid sequence of GPRC5D-mab05.VH2 is as shown in SEQ ID NO: 177: 
EVQLVQSGAEVKKPGSSVKVSCKASGYTFTSYGISWVRQAPGQGLEWMGEIYPRSGNTYYN
EKFKGRVTITADKSTSTAYMELSSLRSEDTAVYYCARGRAYGRNYPMDYWGQGTTVTVSS
The amino acid sequence of GPRC5D-mab05.VH3 is as shown in SEQ ID NO: 178: 
EVQLVQSGAEVKKPGSSVKVSCKASGYTFTSYGISWVRQAPGQGLEWMGEIYPRSGNTYYN
EKFKGRVTLTADKSTSTAYMELSSLRSEDTAVYYCARGRAYGRNYPMDYWGQGTTVTVSS
The amino acid sequence of humanization light chain template IGKV6-21*01 is as shown in SEQ ID NO: 179: 
EIVLTQSPDFQSVTPKEKVTITCRASQSIGSSLHWYQQKPDQSPKLLIKYASQSFSGVPSRFSG
SGSGTDFTLTINSLEAEDAATYYCHQSSSLP
The amino acid sequence of humanization light chain template IGKV3-11*01 is as shown in SEQ ID NO: 180: 
EIVLTQSPATLSLSPGERATLSCRASQSVSSYLAWYQQKPGQAPRLLIYDASNRATGIPARFSGS
GSGTDFTLTISSLEPEDFAVYYCQQRSNWP
The amino acid sequence of humanization light chain template IGKJ4*01 is as shown in SEQ ID NO: 181:
   *FGGGTKVEIK*
The amino acid sequence of humanization heavy chain template IGHV 1-69*02 is as shown in SEQ ID NO: 182: 
QVQLVQSGAEVKKPGSSVKVSCKASGGTFSSYTISWVRQAPGQGLEWMGRIIPILGIANYAQ
KFQGRVTITADKSTSTAYMELSSLRSEDTAVYYCAR
The amino acid sequence of humanization heavy chain template IGHJ6*01 is as shown in SEQ ID NO: 183:
   *WGQGTTVTVSS*

In the present application, different light chain and heavy chain sequences were selected from the above-mentioned mutation design of the light chain and heavy chain variable regions of the humanized antibody of GPRC5D-mab05 for cross combination, respectively, and finally a plurality of humanized antibodies of GPRC5D-mab05 were obtained. The amino acid sequences of the variable regions of each antibody are shown in Table 25 below:

**Table 25 The corresponding amino acid sequences of the variable regions of GPRCSD-mab05 antibody**

| | GPRC5D-mab05.VH1 | GPRC5D-mab05.VH2 | GPRC5D-mab05.VH3 |
|---|---|---|---|
| GPRC5D-mab05.VL1 | GPRC5D-mab05VL1VH1 | GPRC5D-mab05VL1VH2 | GPRC5D-mab05VL1VH3 |
| GPRC5D-mab05.VL2 | GPRC5D-mab05VL2VH1 | GPRC5D-mab05VL2VH2 | GPRC5D-mab05VL2VH3 |
| GPRC5D-mab05.VL3 | GPRC5D-mab05VL3VH1 | GPRC5D-mab05VL3VH2 | GPRCSD-mab05VL3VH3 |
| GPRC5D-mab05.VL4 | GPRC5D-mab05VL4VH1 | GPRC5D-mab05VL4VH2 | GPRC5D-mab05VL4VH3 |

According to the Kabat numbering system, the analysis results of the VH and VL sequences of the above-mentioned humanized antibodies are shown in Table 26.

**Table 26 Kabat analysis results of the VH and VL sequences of the humanized antibodies of GPRCSD-mab05**

| **Variable region** | **CDR1** | **CDR2** | **CDR3** |
|---|---|---|---|
| GPRCSD-mab05.VL1 | RASQSIGTSIH (SEQ ID NO: 79) | YASESIS (SEQ ID NO: 80) | QQSNSWPYT (SEQ ID NO: 81) |
| GPRCSD-mab05.VL2 | | | |
| GPRC5D-mab05.VL3 | | | |
| GPRC5D-mab05.VL4 | | | |
| GPRCSD-mab05.VH1 | SYGIS (SEQ ID NO: 73) | EIYPRSGNTYYNEKFKG (SEQ ID NO: 74) | GRAYGRNYPMDY (SEQ ID NO: 75) |
| GPRCSD-mab05.VH2 | | | |
| GPRCSD-mab05.VH3 | | | |

### 5.6 Humanization of GPRC5D-mab06

### 5.6.1 First round of humanization design of GPRC5D-mab06

The method was the same as 5.1. The humanization light chain templates of the murine antibody GPRC5D-mab06 were IGKV4-1*01/IGKV1-27*01 and IGKJ2*01, and the humanization heavy chain templates were IGHV2-26*01 and IGHJ6*01. The specific mutation designs are shown in Table 27.

**Table 27 Mutation design of humanized antibody of GPRCSD-mab06**

| **VL** | | **VH** | |
|---|---|---|---|
| L1 | Graft(IGKV4-1*01) + P43S | H1 | Graft(IGHV2-26*01) + S30T,T73N |
| L2 | Graft(IGKV4-1*01) + P43S,G66R | H2 | Graft(IGHV2-26*01) + S30T,T73N,V79F |
| L3 | Graft(IGKV4-1*01) + P43S,G66R,Q100A | H3 | Graft(IGHV2-26*01) + S30T,T73N,V79F,L80F |
| L4 | Graft(IGKV1-27*01) + V43S,S60D | | |

Notes: Graft represents the grafting of the CDRs of the murine antibody into the FR region sequence of the human germline template; P43S represents the mutation of P at position 43 of Graft into S, and so on. The numbering of mutated amino acids is according to the natural sequence numbering.

The specific sequences of the variable regions of the humanized antibodies of GPRC5D-mab06 are as follows:
The amino acid sequence of GPRC5D-mab06.VL1 is as shown in SEQ ID NO: 184: 
DIVMTQSPDSLAVSLGERATINCKASQDVRTAVAWYQQKPGQSPKLLIYWASTRHTGVPDRF
SGSGSGTDFTLTISSLQAEDVAVYYCQQHYSTPLTFGQGTKLEIK
The amino acid sequence of GPRC5D-mab06.VL2 is as shown in SEQ ID NO: 185: 
DIVMTQSPDSLAVSLGERATINCKASQDVRTAVAWYQQKPGQSPKLLIYWASTRHTGVPDRF
SGSRSGTDFTLTISSLQAEDVAVYYCQQHYSTPLTFGQGTKLEIK
The amino acid sequence of GPRC5D-mab06.VL3 is as shown in SEQ ID NO: 186: 
DIVMTQSPDSLAVSLGERATINCKASQDVRTAVAWYQQKPGQSPKLLIYWASTRHTGVPDRF
SGSRSGTDFTLTISSLQAEDVAVYYCQQHYSTPLTFGAGTKLEIK
The amino acid sequence of GPRC5D-mab06.VL4 is as shown in SEQ ID NO: 187: 
DIQMTQSPSSLSASVGDRVTITCKASQDVRTAVAWYQQKPGKSPKLLIYWASTRHTGVPDRF
SGSGSGTDFTLTISSLQPEDVATYYCQQHYSTPLTFGQGTKLEIK
The amino acid sequence of GPRCSD-mab06.VH1 is as shown in SEQ ID NO: 188: 
EVTLKESGPVLVKPTETLTLTCTVSGFSLTSYGVNWIRQPPGKALEWLAVIWSGGNTDYNAA
FRSRLTISKDNSKSQVVLTMTNMDPVDTATYYCARGQLGRPYWYFDVWGQGTTVTVSS
The amino acid sequence of GPRCSD-mab06.VH2 is as shown in SEQ ID NO: 189: 
EVTLKESGPVLVKPTETLTLTCTVSGFSLTSYGVNWIRQPPGKALEWLAVIWSGGNTDYNAA
FRSRLTISKDNSKSQVFLTMTNMDPVDTATYYCARGQLGRPYWYFDVWGQGTTVTVSS
The amino acid sequence of GPRC5D-mab06.VH3 is as shown in SEQ ID NO: 190: 
EVTLKESGPVLVKPTETLTLTCTVSGFSLTSYGVNWIRQPPGKALEWLAVIWSGGNTDYNAA
FRSRLTISKDNSKSQVFFTMTNMDPVDTATYYCARGQLGRPYWYFDVWGQGTTVTVSS
The amino acid sequence of humanization light chain template IGKV4-1*01 is as shown in SEQ ID NO: 191: 
DIVMTQSPDSLAVSLGERATINCKSSQSVLYSSNNKNYLAWYQQKPGQPPKLLIYWASTRESG
VPDRFSGSGSGTDFTLTISSLQAEDVAVYYCQQYYSTP
The amino acid sequence of humanization light chain template IGKV1-27*01 is as shown in SEQ ID NO: 192: 
DIQMTQSPSSLSASVGDRVTITCRASQGISNYLAWYQQKPGKVPKLLIYAASTLQSGVPSRFS
GSGSGTDFTLTISSLQPEDVATYYCQKYNSAP
The amino acid sequence of humanization light chain template IGKJ2*01 is as shown in SEQ ID NO: 193:
   *FGQGTKLEIK*
The amino acid sequence of humanization heavy chain template IGHV2-26*01 is as shown in SEQ ID NO: 194: 
QVTLKESGPVLVKPTETLTLTCTVSGFSLSNARMGVSWIRQPPGKALEWLAHIFSNDEKSYST
SLKSRLTISKDTSKSQVVLTMTNMDPVDTATYYCARI
The amino acid sequence of humanization heavy chain template IGHJ6*01 is as shown in SEQ ID NO: 195:
   *WGQGTTVTVSS*

In the present application, different light chain and heavy chain sequences were selected from the above-mentioned mutation design of the light chain and heavy chain variable regions of the first round of humanized antibody of GPRC5D-mab06 for cross combination, respectively, and finally a plurality of humanized antibodies of GPRC5D-mab06 were obtained. The amino acid sequences of the variable regions of each antibody are shown in Table 28 below:

**Table 28 The corresponding amino acid sequences of the variable regions of GPRCSD-mab06 antibody**

| | GPRC5D-mab06.VH1 | GPRC5D-mab06.VH2 | GPRC5D-mab06.VH3 |
|---|---|---|---|
| GPRC5D-mab06.VL1 | GPRC5D-mab06.VL1VH1 | GPRC5D-mab06.VL1VH2 | GPRC5D-mab06.VL1VH3 |
| GPRC5D-mab06.VL2 | GPRC5D-mab06.VL2VH1 | GPRC5D-mab06.VL2VH2 | GPRC5D-mab06.VL2VH3 |
| GPRC5D-mab06.VL3 | GPRC5D-mab06.VL3VH1 | GPRC5D-mab06.VL3VH2 | GPRC5D-mab06.VL3VH3 |
| GPRC5D-mab06.VL4 | GPRC5D-mab06.VL4VH1 | GPRC5D-mab06.VL4VH2 | GPRC5D-mab06.VL4VH3 |

According to the Kabat numbering system, the analysis results of the VH and VL sequences of the above-mentioned humanized antibodies are shown in Table 29.

**Table 29 Kabat analysis results of the VH and VL sequences of the humanized antibodies of GPRCSD-mab06**

| **Variable region** | **CDR1** | **CDR2** | **CDR3** |
|---|---|---|---|
| GPRC5D-mab06.VL1 | KASQDVRTAVA (SEQ ID NO: 90) | WASTRHT (SEQ ID NO: 91) | QQHYSTPLT (SEQ ID NO: 92) |
| GPRC5D-mab06.VL2 | | | |
| GPRC5D-mab06.VL3 | | | |
| GPRCSD-mab06. VL4 | | | |
| GPRCSD-mab06. VH 1 | SYGVN (SEQ ID NO: 84) | VIWSGGNTDYNAAFRS (SEQ ID NO: 85) | GQLGRPYWYFDV (SEQ ID NO: 86) |
| GPRCSD-mab06. VH2 | | | |
| GPRCSD-mab06. VH3 | | | |

### 5.6.2 Second round of humanization design of GPRC5D-mab06

The method was the same as 5.1. The second round humanization light chain templates of the murine antibody GPRC5D-mab06 were IGKV1-27*01 and IGKJ2*01, and the second round humanization heavy chain templates were IGHV2-26*01 and IGHJ6*01. The specific mutation designs are shown in Table 30.

**Table 30 Mutation design of humanized antibody of GPRCSD-mab06**

| **VL** | | **VH** | |
|---|---|---|---|
| L4 | Graft(IGKV1-27*01) + V43S,S60D | H4 | Graft(IGHV2-26*01) + T3Q,S30T,T73N + E1Q |
| L5 | Graft(IGKV1-27*01) + Q3V,V43S,S60D | H5 | Graft(IGHV2-26*01) + T3Q,S30T,T73N,Q112T + E1Q |
| | | H6 | Graft(IGHV2-26*01) + S30T,T68S,T73N |
| | | H7 | Graft(IGHV2-26*01) + S30T,T73N,T81K |

Notes: Graft represents the grafting of the CDRs of the murine antibody into the FR region sequence of the human germline template; V43S represents the mutation of V at position 43 of Graft into S, and so on. The numbering of mutated amino acids is according to the natural sequence numbering.

The specific sequences of the variable regions of the humanized antibodies of GPRC5D-mab06 are as follows:
The amino acid sequence of GPRC5D-mab06.VL4 is as shown in SEQ ID NO: 196: 
DIQMTQSPSSLSASVGDRVTITCKASQDVRTAVAWYQQKPGKSPKLLIYWASTRHTGVPDRFS
GSGSGTDFTLTISSLQPEDVATYYCQQHYSTPLTFGQGTKLEIK
The amino acid sequence of GPRC5D-mab06.VL5 is as shown in SEQ ID NO: 197: 
DIVMTQSPSSLSASVGDRVTITCKASQDVRTAVAWYQQKPGKSPKLLIYWASTRHTGVPDRFS
GSGSGTDFTLTISSLQPEDVATYYCQQHYSTPLTFGQGTKLEIK
The amino acid sequence of GPRCSD-mab06.VH4 is as shown in SEQ ID NO: 198: 
QVQLKESGPVLVKPTETLTLTCTVSGFSLTSYGVNWIRQPPGKALEWLAVIWSGGNTDYNAA
FRSRLTISKDNSKSQVVLTMTNMDPVDTATYYCARGQLGRPYWYFDVWGQGTTVTVSS
The amino acid sequence of GPRC5D-mab06.VH5 is as shown in SEQ ID NO: 199: 
QVQLKESGPVLVKPTETLTLTCTVSGFSLTSYGVNWIRQPPGKALEWLAVIWSGGNTDYNAA
FRSRLTISKDNSKSQVVLTMTNMDPVDTATYYCARGQLGRPYWYFDVWGTGTTVTVSS
The amino acid sequence of GPRCSD-mab06.VH6 is as shown in SEQ ID NO: 200: 
EVTLKESGPVLVKPTETLTLTCTVSGFSLTSYGVNWIRQPPGKALEWLAVIWSGGNTDYNAA
FRSRLSISKDNSKSQVVLTMTNMDPVDTATYYCARGQLGRPYWYFDVWGQGTTVTVSS
The amino acid sequence of GPRCSD-mab06.VH7 is as shown in SEQ ID NO: 201: 
EVTLKESGPVLVKPTETLTLTCTVSGFSLTSYGVNWIRQPPGKALEWLAVIWSGGNTDYNAA
FRSRLTISKDNSKSQVVLKMTNMDPVDTATYYCARGQLGRPYWYFDVWGQGTTVTVSS
The amino acid sequence of humanization light chain template IGKV1-27*01 is as shown in SEQ ID NO: 202: 
DIQMTQSPSSLSASVGDRVTITCRASQGISNYLAWYQQKPGKVPKLLIYAASTLQSGVPSRFS
GSGSGTDFTLTISSLQPEDVATYYCQKYNSAP
The amino acid sequence of humanization light chain template IGKJ2*01 is as shown in SEQ ID NO: 203:
   *FGQGTKLEIK*
The amino acid sequence of humanization heavy chain template IGHV2-26*01 is as shown in SEQ ID NO: 204: 
QVTLKESGPVLVKPTETLTLTCTVSGFSLSNARMGVSWIRQPPGKALEWLAHIFSNDEKSYST
SLKSRLTISKDTSKSQVVLTMTNMDPVDTATYYCARI
The amino acid sequence of humanization heavy chain template IGHJ6*01 is as shown in SEQ ID NO: 205:
   *WGQGTTVTVSS*

In the present application, different light chain and heavy chain sequences were selected from the above-mentioned mutation design of the light chain and heavy chain variable regions of the second round of humanized antibody of GPRC5D-mab06 for cross combination, respectively, and finally a plurality of humanized antibodies of GPRC5D-mab06 were obtained. The amino acid sequences of the variable regions of each antibody are shown in Table 31:

**Table 31 The corresponding amino acid sequences of the variable regions of GPRCSD-mab06 antibody**

| | GPRC5D-mab06.VH4 | GPRC5D-mab06.VH5 | GPRC5D-mab06.VH6 | GPRC5D-mab06.VH7 |
|---|---|---|---|---|
| GPRC5D-mab06.VL4 | GPRC5D-mab06.VL4VH4 | GPRC5D-mab06.VL4VH5 | GPRC5D-mab06.VL4VH6 | GPRC5D-mab06.VL4VH7 |
| GPRC5D-mab06.VL5 | GPRC5D-mab06.VL5VH4 | GPRC5D-mab06.VL5VH5 | GPRC5D-mab06.VL5VH6 | GPRC5D-mab06.VL5VH7 |

According to the Kabat numbering system, the analysis results of the VH and VL sequences of the above-mentioned humanized antibodies are shown in Table 32.

**Table 32 Kabat analysis results of the VH and VL sequences of the humanized antibodies of GPRCSD-mab06**

| **Variable region** | **CDR1** | **CDR2** | **CDR3** |
|---|---|---|---|
| GPRC5D-mab06.VL4 | KASQDVRTAVA (SEQ ID NO: 90) | WASTRHT (SEQ ID NO: 91) | QQHYSTPLT (SEQ ID NO: 92) |
| GPRC5D-mab06.VL5 | | | |
| GPRC5D-mab06.VH4 | SYGVN (SEQ ID NO: 84) | VIWSGGNTDYNAAFRS (SEQ ID NO: 85) | GQLGRPYWYFDV (SEQ ID NO: 86) |
| GPRC5D-mab06.VH5 | | | |
| GPRC5D-mab06.VH6 | | | |
| GPRC5D-mab06.VH7 | | | |

### 5.7 Humanization of GPRC5D-mab07

### 5.7.1 First round of humanization design of GPRC5D-mab07

The method was the same as 5.1. The humanization light chain templates of the murine antibody GPRC5D-mab07 were IGKV2-40*01 and IGKJ4*01, and the humanization heavy chain templates were IGHV1-69*02 and IGHJ6*01. The specific mutation designs are shown in Table 33.

**Table 33 Mutation design of humanized antibody of GPRCSD-mab07**

| **VL** | | **VH** | |
|---|---|---|---|
| L1 | Graft(IGKV2-40*01) | H1 | Graft(IGHV1-69*02) + G27Y,T28A,S30T |
| L2 | Graft(IGKV2-40*01) + Q50K | H2 | Graft(IGHV1-69*02) + G27Y,T28A,S30T,I70L |
| L2a | Graft(IGKV2-40*01) + Q50K + G34A | H3 | Graft(IGHV1-69*02) + G27Y,T28A,S30T,I70L,S75A |
| | | H4 | Graft(IGHV2-69*02) + V5Q,G27Y,T28A,S30T,I70L + E1Q |

Notes: Graft represents the grafting of the CDRs of the murine antibody into the FR region sequence of the human germline template; Q50K represents the mutation of Q at position 50 of Graft into K, and so on. The numbering of mutated amino acids is according to the natural sequence numbering.

The specific sequences of the variable regions of the humanized antibodies of GPRC5D-mab07 are as follows:
The amino acid sequence of GPRC5D-mab07.VL1 is as shown in SEQ ID NO: 206: 
DIVMTQTPLSLPVTPGEPASISCRSSQSLVYSNGNTYLHWYLQKPGQSPQLLIYKVSNRFSGV
PDRFSGSGSGTDFTLKISRVEAEDVGVYYCSQSTHVPWTFGGGTKVEIK
The amino acid sequence of GPRC5D-mab07.VL2 is as shown in SEQ ID NO: 207: 
DIVMTQTPLSLPVTPGEPASISCRSSQSLVYSNGNTYLHWYLQKPGQSPKLLIYKVSNRFSGVP
DRFSGSGSGTDFTLKISRVEAEDVGVYYCSQSTHVPWTFGGGTKVEIK
The amino acid sequence of GPRC5D-mab07.VL2a is as shown in SEQ ID NO: 208: 
DIVMTQTPLSLPVTPGEPASISCRSSQSLVYSNANTYLHWYLQKPGQSPKLLIYKVSNRFSGVP
DRFSGSGSGTDFTLKISRVEAEDVGVYYCSQSTHVPWTFGGGTKVEIK
The amino acid sequence of GPRCSD-mab07.VH1 is as shown in SEQ ID NO: 209: 
EVQLVQSGAEVKKPGSSVKVSCKASGYAFTNYLIEWVRQAPGQGLEWMGMINPGRGNTNY
NEKFKGRVTITADKSTSTAYMELSSLRSEDTAVYYCARNWDVWGQGTTVTVSS
The amino acid sequence of GPRCSD-mab07.VH2 is as shown in SEQ ID NO: 210: 
EVQLVQSGAEVKKPGSSVKVSCKASGYAFTNYLIEWVRQAPGQGLEWMGMINPGRGNTNY
NEKFKGRVTLTADKSTSTAYMELSSLRSEDTAVYYCARNWDVWGQGTTVTVSS
The amino acid sequence of GPRC5D-mab07.VH3 is as shown in SEQ ID NO: 211: 
EVQLVQSGAEVKKPGSSVKVSCKASGYAFTNYLIEWVRQAPGQGLEWMGMINPGRGNTNY
NEKFKGRVTLTADKATSTAYMELSSLRSEDTAVYYCARNWDVWGQGTTVTVSS
The amino acid sequence of GPRCSD-mab07.VH4 is as shown in SEQ ID NO: 212: 
QVQLQQSGAEVKKPGSSVKVSCKASGYAFTNYLIEWVRQAPGQGLEWMGMINPGRGNTNY
NEKFKGRVTLTADKSTSTAYMELSSLRSEDTAVYYCARNWDVWGQGTTVTVSS
The amino acid sequence of humanization light chain template IGKV2-40*01 is as shown in SEQ ID NO: 213: 
DIVMTQTPLSLPVTPGEPASISCRSSQSLLDSDDGNTYLDWYLQKPGQSPQLLIYTLSYRASG
VPDRFSGSGSGTDFTLKISRVEAEDVGVYYCMQRIEFP
The amino acid sequence of humanization light chain template IGKJ4*01 is as shown in SEQ ID NO: 214:
   *FGGGTKVEIK*
The amino acid sequence of humanization heavy chain template IGHV 1-69*02 is as shown in SEQ ID NO: 215: 
QVQLVQSGAEVKKPGSSVKVSCKASGGTFSSYTISWVRQAPGQGLEWMGRIIPILGIANYAQ
KFQGRVTITADKSTSTAYMELSSLRSEDTAVYYCAR
The amino acid sequence of humanization heavy chain template IGHJ6*01 is as shown in SEQ ID NO: 216:
   *WGQGTTVTVSS*

In the present application, different light chain and heavy chain sequences were selected from the above-mentioned back mutation design of the light chain and heavy chain variable regions of the first round of humanized antibody of GPRC5D-mab07 for cross combination, respectively, and finally a plurality of humanized antibodies of GPRC5D-mab07 were obtained. The amino acid sequences of the variable regions of each antibody are shown in Table 34:

**Table 34 The corresponding amino acid sequences of the variable regions of GPRCSD-mab07 antibody**

| | GPRC5D-mab07.VH1 | GPRC5D-mab07.VH2 | GPRC5D-mab07.VH3 | GPRC5D-mab07.VH4 |
|---|---|---|---|---|
| GPRC5D-mab07.VL1 | GPRC5D-mab07.VL1VH1 | GPRC5D-mab07.VL1VH2 | GPRC5D-mab07.VL1VH3 | GPRC5D-mab07.VL1VH4 |
| GPRC5D-mab07.VL2 | GPRC5D-mab07.VL2VH1 | GPRC5D-mab07.VL2VH2 | GPRC5D-mab07.VL2VH3 | GPRC5D-mab07.VL2VH4 |
| GPRC5D-mab07.VL2a | GPRC5D-mab07.VL2aVH1 | GPRC5D-mab07.VL2aVH2 | GPRC5D-mab07.VL2aVH3 | GPRC5D-mab07.VL2aVH4 |

According to the Kabat numbering system, the analysis results of the VH and VL sequences of the above-mentioned humanized antibodies are shown in Table 35.

**Table 35 Kabat analysis results of the VH and VL sequences of the humanized antibodies of GPRCSD-mab07**

| **Variable region** | **CDR1** | **CDR2** | **CDR3** |
|---|---|---|---|
| GPRC5D-mab07.VL1 | RSSQSLVYSNGNTYLH (SEQ ID NO: 101) | KVSNRFS (SEQ ID NO: 102) | SQSTHVPWT (SEQ ID NO: 103) |
| GPRC5D-mab07.VL2 | | | |
| GPRC5D-mab07.VL2a | RSSQSLVYSNANTYLH (SEQ ID NO: 217) | KVSNRFS (SEQ ID NO: 102) | SQSTHVPWT (SEQ ID NO: 103) |
| GPRC5D-mab07.VH1 | NYLIE (SEQ ID NO: 95) | MINPGRGNTNYNEKFKG (SEQ ID NO: 96) | NWDV (SEQ ID NO: 97) |
| GPRC5D-mab07.VH2 | | | |
| GPRC5D-mab07.VH3 | | | |
| GPRC5D-mab07.VH4 | | | |

### 5.7.2 Second round of humanization design of GPRC5D-mab07

The method was the same as 5.1. The second round humanization light chain templates of the murine antibody GPRC5D-mab07 were IGKV2-40*01 and IGKJ4*01, and the second round humanization heavy chain templates were IGHV1-69*02 and IGHJ6*01. The specific mutation designs are shown in Table 36.

**Table 36 Mutation design of humanized antibody of GPRCSD-mab07**

| **VL** | | **VH** | |
|---|---|---|---|
| L1a | Graft(IGKV2-40*01) + G34A | H4 | Graft(IGHV1-69*02) + V5Q,G27Y,T28A,S30T,I70L + E1Q |
| L1b | Graft(IGKV2-40*01) + G34S | | |
| L1c | Graft(IGKV2-40*01) + N33D | | |
| L1d | Graft(IGKV2-40*01) + N33Q | | |

Notes: Graft represents the grafting of the CDRs of the murine antibody into the FR region sequence of the human germline template; G43A represents the mutation of G at position 43 of Graft into A, and so on. The numbering of mutated amino acids is according to the natural sequence numbering.

The specific sequences of the variable regions of the humanized antibodies of GPRC5D-mab07 are as follows:
The amino acid sequence of GPRC5D-mabO7.VL1a is as shown in SEQ ID NO: 218: 
DIVMTQTPLSLPVTPGEPASISCRSSQSLVYSNANTYLHWYLQKPGQSPQLLIYKVSNRFSGVP
DRFSGSGSGTDFTLKISRVEAEDVGVYYCSQSTHVPWTFGGGTKVEIK
The amino acid sequence of GPRC5D-mab07.VL1b is as shown in SEQ ID NO: 219: 
DIVMTQTPLSLPVTPGEPASISCRSSQSLVYSNSNTYLHWYLQKPGQSPQLLIYKVSNRFSGVP
DRFSGSGSGTDFTLKISRVEAEDVGVYYCSQSTHVPWTFGGGTKVEIK
The amino acid sequence of GPRC5D-mab07.VL1c is as shown in SEQ ID NO: 220: 
DIVMTQTPLSLPVTPGEPASISCRSSQSLVYSDGNTYLHWYLQKPGQSPQLLIYKVSNRFSGV
PDRFSGSGSGTDFTLKISRVEAEDVGVYYCSQSTHVPWTFGGGTKVEIK
The amino acid sequence of GPRC5D-mab07.VL1d is as shown in SEQ ID NO: 221: 
DIVMTQTPLSLPVTPGEPASISCRSSQSLVYSQGNTYLHWYLQKPGQSPQLLIYKVSNRFSGV
PDRFSGSGSGTDFTLKISRVEAEDVGVYYCSQSTHVPWTFGGGTKVEIK
The amino acid sequence of GPRCSD-mab07.VH4 is as shown in SEQ ID NO: 222: 
QVQLQQSGAEVKKPGSSVKVSCKASGYAFTNYLIEWVRQAPGQGLEWMGMINPGRGNTNY
NEKFKGRVTLTADKSTSTAYMELSSLRSEDTAVYYCARNWDVWGQGTTVTVSS
The amino acid sequence of humanization light chain template IGKV2-40*01 is as shown in SEQ ID NO: 223: 
DIVMTQTPLSLPVTPGEPASISCRSSQSLLDSDDGNTYLDWYLQKPGQSPQLLIYTLSYRASG
VPDRFSGSGSGTDFTLKISRVEAEDVGVYYCMQRIEFP
The amino acid sequence of humanization light chain template IGKJ4*01 is as shown in SEQ ID NO: 224:
   *FGGGTKVEIK*
The amino acid sequence of humanization heavy chain template IGHV 1-69*02 is as shown in SEQ ID NO: 225: 
QVQLVQSGAEVKKPGSSVKVSCKASGGTFSSYTISWVRQAPGQGLEWMGRIIPILGIANYAQ
KFQGRVTITADKSTSTAYMELSSLRSEDTAVYYCAR
The amino acid sequence of humanization heavy chain template IGHJ6*01 is as shown in SEQ ID NO: 226:
   *WGQGTTVTVSS*

In the present application, different light chain and heavy chain sequences were selected from the above-mentioned mutation design of the light chain and heavy chain variable regions of the second round of humanized antibody of GPRC5D-mab07 for cross combination, respectively, and finally a plurality of humanized antibodies of GPRC5D-mab07 were obtained. The amino acid sequences of the variable regions of each antibody are shown in Table 37:

**Table 37 The corresponding amino acid sequences of the variable regions of GPRCSD-mab07 antibody**

| | GPRC5D-mab07.VH4 |
|---|---|
| GPRC5D-mab07.VL1a | GPRC5D-mab07.VL1aVH4 |
| GPRC5D-mab07.VL1b | GPRC5D-mab07.VL1bVH4 |
| GPRC5D-mab07.VL1c | GPRC5D-mab07.VL1cVH4 |
| GPRC5D-mab07.VL1d | GPRC5D-mab07.VL1dVH4 |

According to the Kabat numbering system, the analysis results of the VH and VL sequences of the above-mentioned humanized antibodies are shown in Table 38.

**Table 38 Kabat analysis results of the VH and VL sequences of the humanized antibodies of GPRCSD-mab07**

| **Variable region** | **CDR1** | **CDR2** | **CDR3** |
|---|---|---|---|
| GPRC5D-mab07.VL1a | RSSQSLVYSNANTYLH (SEQ ID NO: 227) | KVSNRFS (SEQ ID NO: 102) | SQSTHVPWT (SEQ ID NO: 103) |
| GPRC5D-mab07.VL1b | RSSQSLVYSNSNTYLH (SEQ ID NO: 228) | KVSNRFS (SEQ ID NO: 102) | SQSTHVPWT (SEQ ID NO: 103) |
| GPRC5D-mab07.VL1c | RSSQSLVYSDGNTYLH (SEQ ID NO: 229) | KVSNRFS (SEQ ID NO: 102) | SQSTHVPWT (SEQ ID NO: 103) |
| GPRC5D-mab07.VL1d | RSSQSLVYSQGNTYLH (SEQ ID NO: 230) | KVSNRFS (SEQ ID NO: 102) | SQSTHVPWT (SEQ ID NO: 103) |
| GPRCSD-mab07.VH4 | NYLIE (SEQ ID NO: 95) | MINPGRGNTNYNEKFKG (SEQ ID NO: 96) | NWDV (SEQ ID NO: 97) |

### 5.8 Humanization of GPRC5D-mab08

The method was the same as 5.1. The humanization light chain templates of the murine antibody GPRC5D-mab08 were IGKV1-27*01/IGKV2-29*02 and IGKJ4*01, and the humanization heavy chain templates were IGHV1-3*01 and IGHJ6*01. The specific mutation designs are shown in Table 39.

**Table 39 Mutation design of humanized antibody of GPRCSD-mab08**

| **VL** | | **VH** | |
|---|---|---|---|
| L4 | Graft(IGKV2-29*02) + L46A | H6 | Graft(IGHV1-3*01) + R72A,T74K,A97S |
| L5 | Graft(IGKV2-29*02) + Q38H, L46A, Y87I | H7 | Graft(IGHV1-3*01) + T28I,R72A,T74K,A97S |
| L6 | Graft(IGKVl-27*01) + Q38H, V43S, L46A, Y87I | H8 | Graft(IGHV1-3*01) + A24T,T28I,R72A,T74K,A97S |
| | | H9 | Graft(IGHV1-3*01) + A24T,T28I,I70L,R72A,T74K,A97S |
| | | H10 | Graft(IGHV1-3*01) + T28I,R72A,T74K,A76S,S77N,A97S |

Notes: Graft represents the grafting of the CDRs of the murine antibody into the FR region sequence of the human germline template; L46A represents the mutation of L at position 46 of Graft into A, and so on. The numbering of mutated amino acids is according to the natural sequence numbering.

The specific sequences of the variable regions of the humanized antibodies of GPRC5D-mab08 are as follows:
The amino acid sequence of GPRC5D-mab08.VL4 is as shown in SEQ ID NO: 231: 
DIVMTQTPLSLSVTPGQPASISCKASQNVGTNVAWYLQKPGQSPQALIYSASYQYSGVPDRFS
GSGSGTDFTLKISRVEAEDVGVYYCQQYKNYPYTFGGGTKVEIK
The amino acid sequence of GPRC5D-mab08.VL5 is as shown in SEQ ID NO: 232: 
DIVMTQTPLSLSVTPGQPASISCKASQNVGTNVAWYLHKPGQSPQALIYSASYQYSGVPDRFS
GSGSGTDFTLKISRVEAEDVGVYICQQYKNYPYTFGGGTKVEIK
The amino acid sequence of GPRC5D-mab08.VL6 is as shown in SEQ ID NO: 233: 
DIQMTQSPSSLSASVGDRVTITCKASQNVGTNVAWYQHKPGKSPKALIYSASYQYSGVPSRFS
GSGSGTDFTLTISSLQPEDVATYICQQYKNYPYTFGGGTKVEIK
The amino acid sequence of GPRC5D-mab08.VH6 is as shown in SEQ ID NO: 234: 
EVQLVQSGAEVKKPGASVKVSCKASGYTFTNFGITWVRQAPGQRLEWMGENYPRRINTYYN
EKFKGRVTITADKSASTAYMELSSLRSEDTAVYYCSRKDIFGLSYALDYWGQGTTVTVSS
The amino acid sequence of GPRC5D-mab08.VH7 is as shown in SEQ ID NO: 235: 
EVQLVQSGAEVKKPGASVKVSCKASGYIFTNFGITWVRQAPGQRLEWMGENYPRRINTYYN
EKFKGRVTITADKSASTAYMELSSLRSEDTAVYYCSRKDIFGLSYALDYWGQGTTVTVSS
The amino acid sequence of GPRC5D-mab08.VH8 is as shown in SEQ ID NO: 236: 
EVQLVQSGAEVKKPGASVKVSCKTSGYIFTNFGITWVRQAPGQRLEWMGENYPRRINTYYN
EKFKGRVTITADKSASTAYMELSSLRSEDTAVYYCSRKDIFGLSYALDYWGQGTTVTVSS
The amino acid sequence of GPRC5D-mab08.VH9 is as shown in SEQ ID NO: 237: 
EVQLVQSGAEVKKPGASVKVSCKTSGYIFTNFGITWVRQAPGQRLEWMGENYPRRINTYYN
EKFKGRVTLTADKSASTAYMELSSLRSEDTAVYYCSRKDIFGLSYALDYWGQGTTVTVSS
The amino acid sequence of GPRC5D-mab08.VH10 is as shown in SEQ ID NO: 238: 
EVQLVQSGAEVKKPGASVKVSCKASGYIFTNFGITWVRQAPGQRLEWMGENYPRRINTYYN
EKFKGRVTITADKSSNTAYMELSSLRSEDTAVYYCSRKDIFGLSYALDYWGQGTTVTVSS
The amino acid sequence of humanization light chain template IGKV1-27*01 is as shown in SEQ ID NO: 239: 
DIQMTQSPSSLSASVGDRVTITCRASQGISNYLAWYQQKPGKVPKLLIYAASTLQSGVPSRFS
GSGSGTDFTLTISSLQPEDVATYYCQKYNSAP
The amino acid sequence of humanization light chain template IGKV2-29*02 is as shown in SEQ ID NO: 240: 
DIVMTQTPLSLSVTPGQPASISCKSSQSLLHSDGKTYLYWYLQKPGQSPQLLIYEVSSRFSGVP
DRFSGSGSGTDFTLKISRVEAEDVGVYYCMQGIHLP
The amino acid sequence of humanization light chain template IGKJ4*01 is as shown in SEQ ID NO: 241:
   *FGGGTKVEIK*
The amino acid sequence of humanization heavy chain template IGHV1-3*01 is as shown in SEQ ID NO: 242: 
QVQLVQSGAEVKKPGASVKVSCKASGYTFTSYAMHWVRQAPGQRLEWMGWINAGNGNTK
YSQKFQGRVTITRDTSASTAYMELSSLRSEDTAVYYCAR
The amino acid sequence of humanization heavy chain template IGHJ6*01 is as shown in SEQ ID NO: 243:
   *WGQGTTVTVSS*

In the present application, different light chain and heavy chain sequences were selected from the above-mentioned mutation design of the light chain and heavy chain variable regions of the humanized antibody of GPRC5D-mab08 for cross combination, respectively, and finally a plurality of humanized antibodies of GPRC5D-mab08 were obtained. The amino acid sequences of the variable regions of each antibody are shown in Table 40:

**Table 40 The corresponding amino acid sequences of the variable regions of GPRCSD-mab08 antibody**

| | GPRC5D-mab08.VH6 | GPRC5D-mab08.VH7 | GPRC5D-mab08.VH8 | GPRC5D-mab08. VH9 | GPRC5D-mab08.VH10 |
|---|---|---|---|---|---|
| GPRC5D-mab08.VL4 | GPRC5D-mab08.VL4VH6 | GPRC5D-mab08.VL4VH7 | GPRC5D-mab08.VL4VH8 | GPRC5D-mab08.VL4VH9 | GPRC5D-mab08.VL4VH10 |
| GPRC5D-mab08.VL5 | GPRC5D-mab08.VL5VH6 | GPRC5D-mab08.VL5VH7 | GPRC5D-mab08.VL5VH8 | GPRC5D-mab08.VL5VH9 | GPRC5D-mab08.VL5VH10 |
| GPRC5D-mab08.VL6 | GPRC5D-mab08.VL6VH6 | GPRC5D-mab08.VL6VH7 | GPRC5D-mab08.VL6VH8 | GPRC5D-mab08.VL6VH9 | GPRC5D-mab08.VL6VH10 |

According to the Kabat numbering system, the analysis results of the VH and VL sequences of the above-mentioned humanized antibodies are shown in Table 41.

**Table 41 Kabat analysis results of the VH and VL sequences of the humanized antibodies of GPRCSD-mab08**

| **Variable region** | **CDR1** | **CDR2** | **CDR3** |
|---|---|---|---|
| GPRC5D-mab08.VL4 | KASQNVGTNVA (SEQ ID NO: 112) | SASYQYS (SEQ ID NO: 113) | QQYKNYPYT (SEQ ID NO: 114) |
| GPRC5D-mab08.VL5 | | | |
| GPRC5D-mab08.VL6 | | | |
| GPRC5D-mab08.VH6 | NFGIT (SEQ ID NO: 106) | ENYPRRINTYYNEKFKG (SEQ ID NO: 107) | KDIFGLSYALDY (SEQ ID NO: 108) |
| GPRC5D-mab08.VH7 | | | |
| GPRC5D-mab08.VH8 | | | |
| GPRC5D-mab08.VH9 | | | |
| GPRC5D-mab08.VH10 | | | |

### Example 6 Identification of humanized antibodies of GPRCSD

### 6.1 Cell-based enzyme-linked immunosorbent assay (cell-based ELISA) detection of the binding of humanized antibodies with human GPRCSD protein

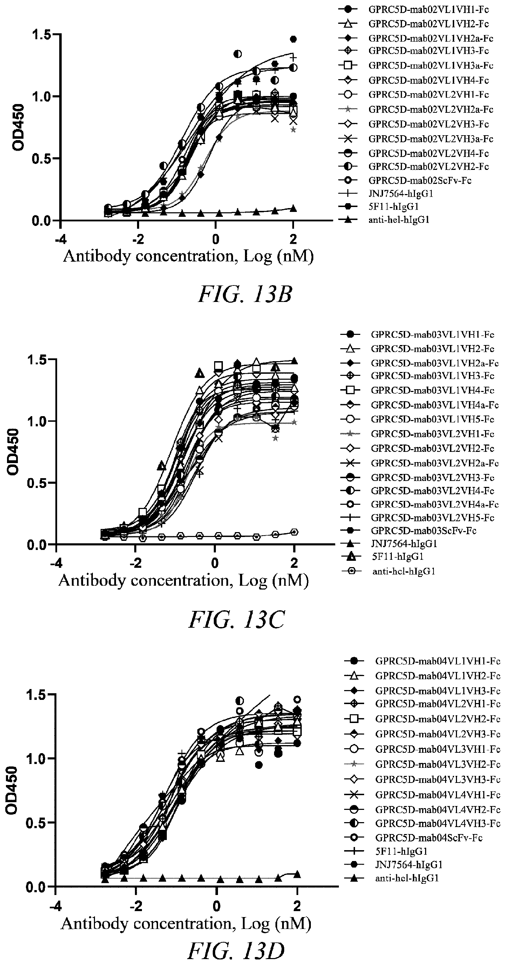

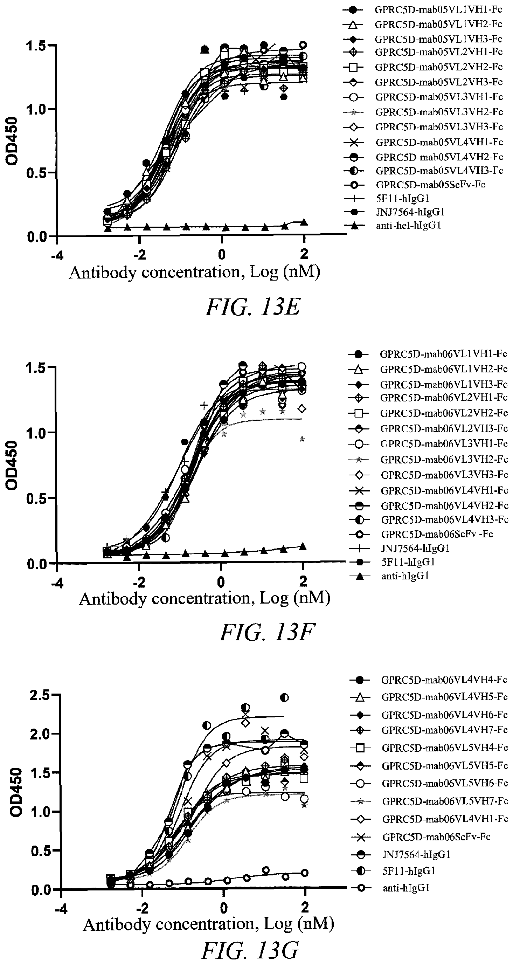

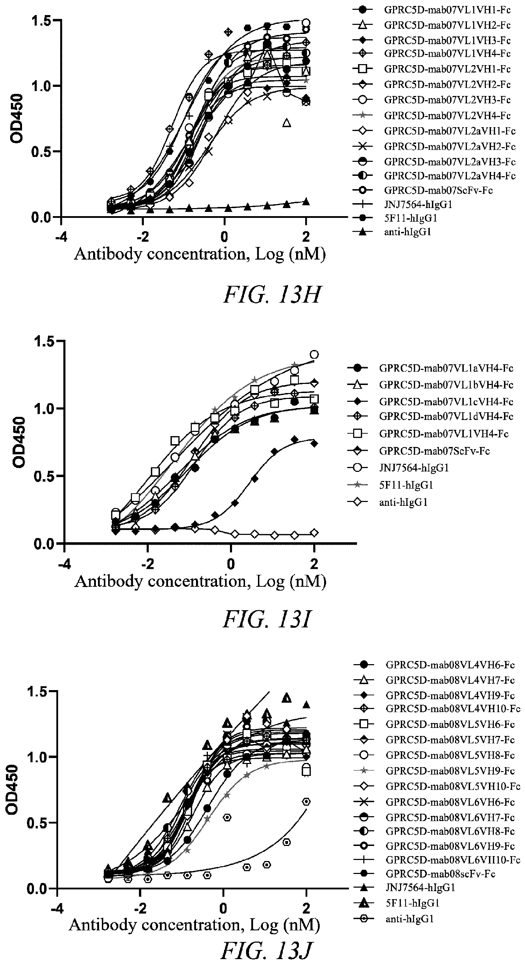

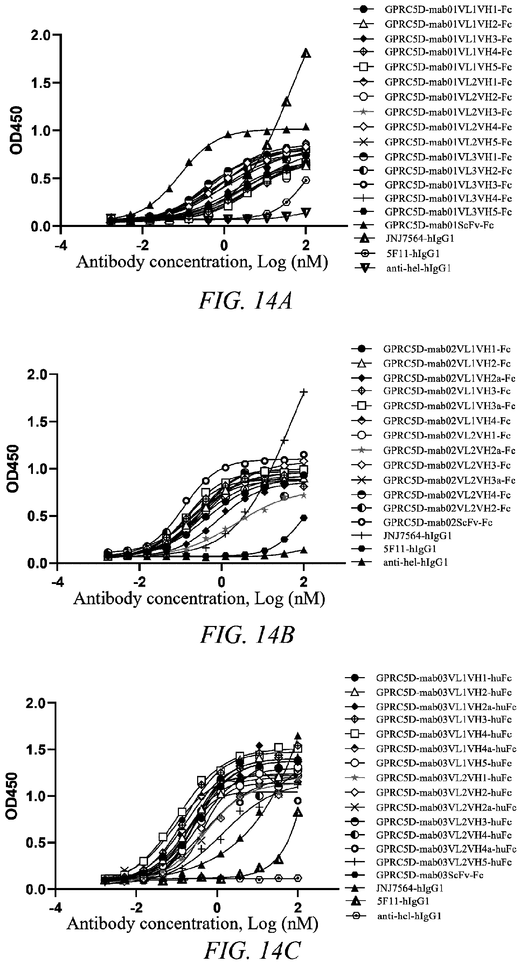

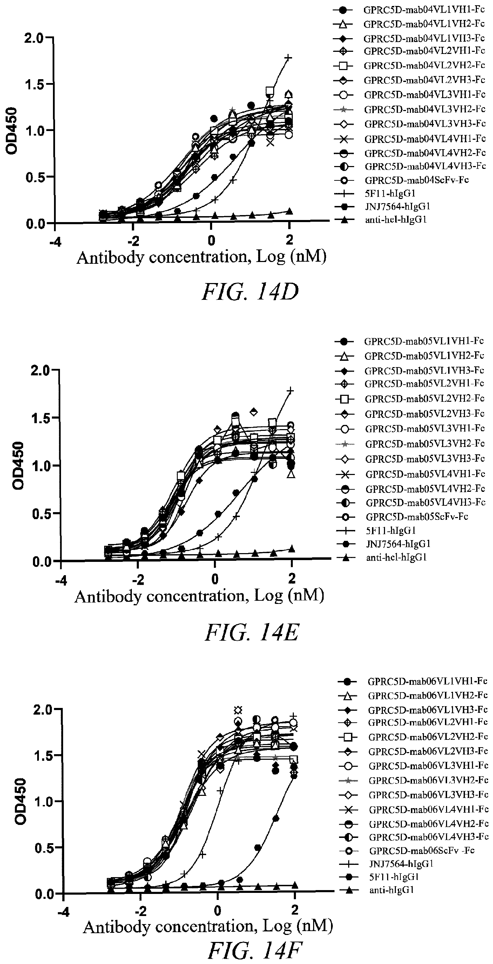

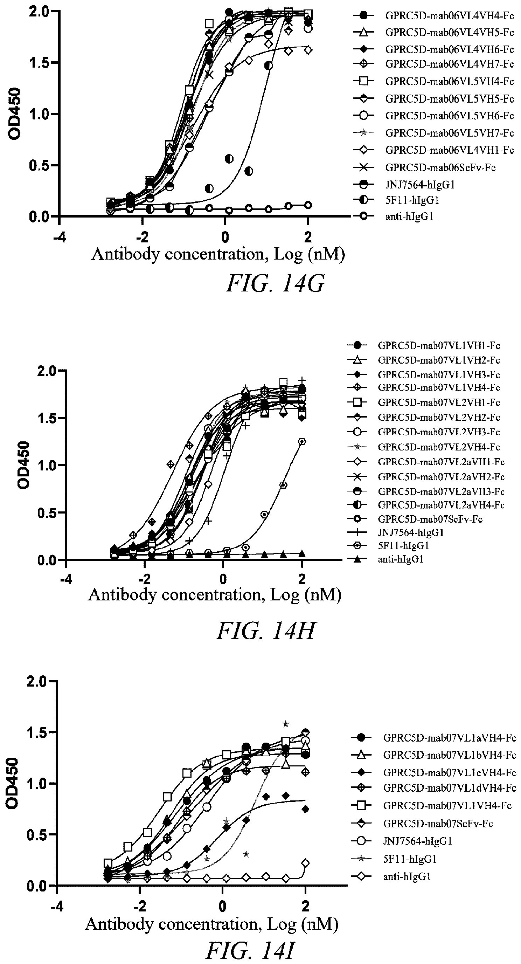

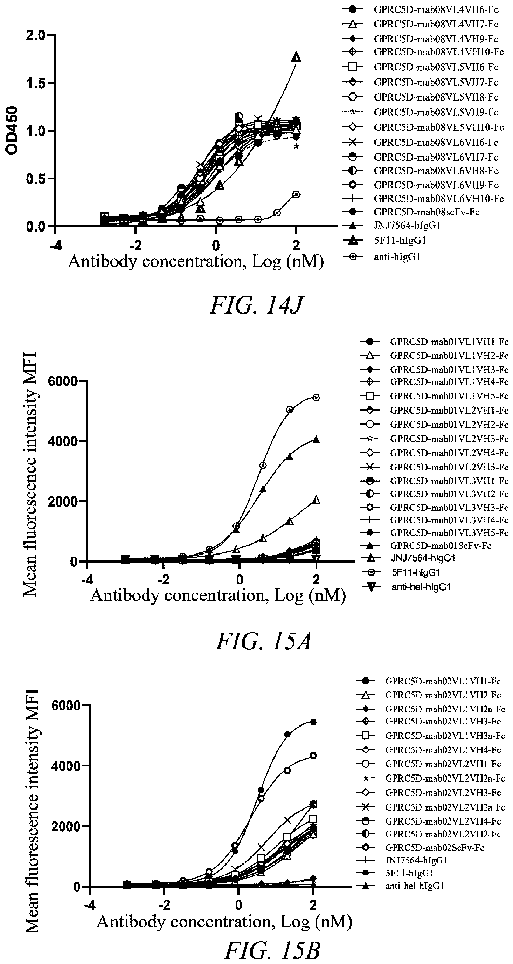

In order to detect the binding activity of GPRC5D humanized antibodies with human GPRCSD full-length protein, the same detection method as the cell-based enzyme-linked immunosorbent assay (cell-based ELISA) in Example 4 was used to detect the binding of humanized antibodies with human GPRCSD protein. The detection results are shown in Figs. 13A-13J and 14A-14J. The results show that the produced pure humanized antibodies bind to human GPRC5D full-length protein to varying degrees at the ELISA level.

### 6.2 Flow cytometry experiment (FACS) to detect the binding activity of humanized antibodies with NCI-H929 cells endogenously expressed human GPRCSD

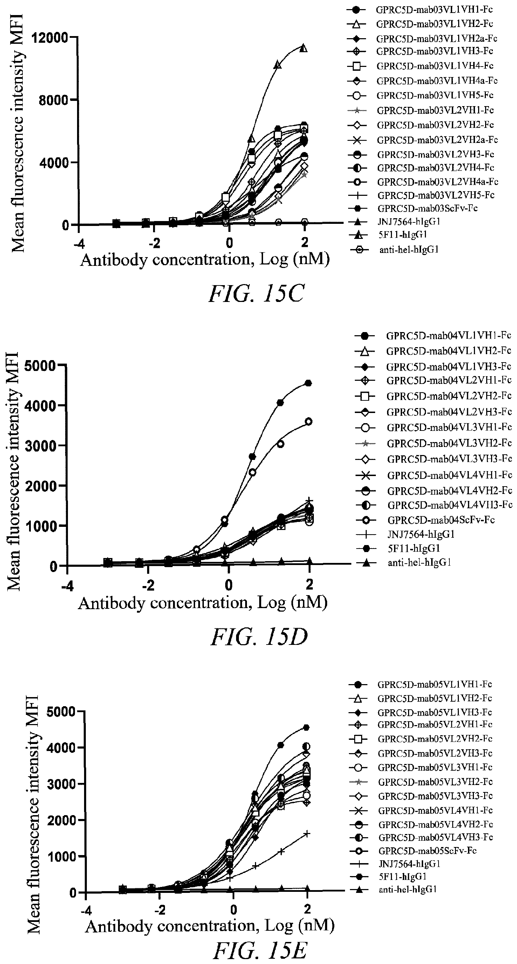

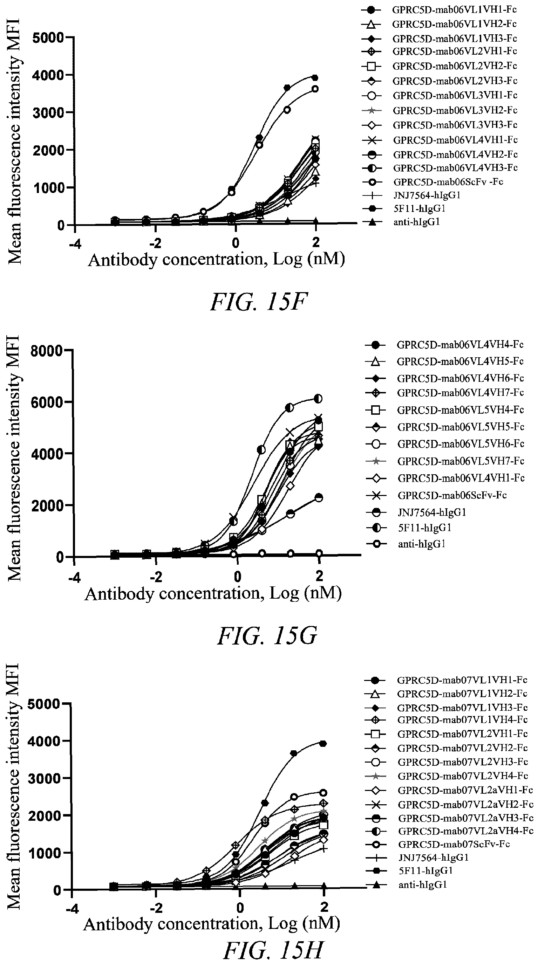

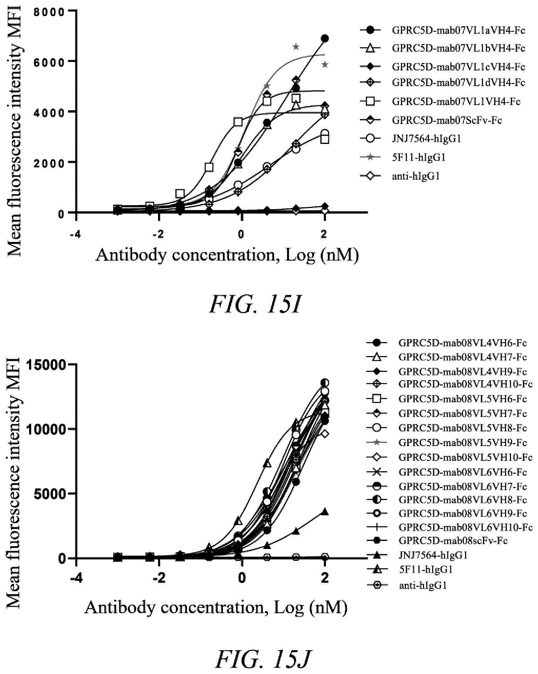

In order to detect the binding activity of GPRC5D humanized antibodies with human GPRCSD full-length protein, the same detection method as the FACS in Example 4 was used to detect the binding of humanized antibodies with human GPRCSD protein. The detection results are shown in Figs. 15A-15J. The results show that humanized antibody can bind to NCI-H929 cells to varying degrees with good specificity.

## Claims

1. An antibody or an antigen-binding fragment thereof that specifically binds to G protein coupled receptor C5 family subtype D (GPRC5D), **characterized in that** the antibody or the antigen-binding fragment thereof comprises a heavy chain variable region and a light chain variable region, and wherein
(1) the light chain variable region comprises LCDR1, LCDR2 and LCDR3, the LCDR1 has any of a sequence of the following LCDR1, or a sequence with 1, 2, 3 or more amino acid insertions, deletions and/or substitutions compared with the sequence, the LCDR2 has any of a sequence of the following LCDR2, or a sequence with 1, 2, 3 or more amino acid insertions, deletions and/or substitutions compared with the sequence, and the LCDR3 has any of a sequence of the following LCDR3, or a sequence with 1, 2, 3 or more amino acid insertions, deletions and/or substitutions compared with the sequence:
| **No.** | **LCDR1** | **LCDR2** | **LCDR3** |
|---|---|---|---|
| L1 | SEQ ID NO: 35 | SEQ ID NO: 36 | SEQ ID NO: 37 |
| L2 | SEQ ID NO: 38 | SEQ ID NO: 39 | SEQ ID NO: 37 |
| L3 | SEQ ID NO: 46 | SEQ ID NO: 47 | SEQ ID NO: 48 |
| L4 | SEQ ID NO: 49 | SEQ ID NO: 50 | SEQ ID NO: 48 |
| L5 | SEQ ID NO: 57 | SEQ ID NO: 58 | SEQ ID NO: 59 |
| L6 | SEQ ID NO: 60 | SEQ ID NO: 61 | SEQ ID NO: 59 |
| L7 | SEQ ID NO: 68 | SEQ ID NO: 69 | SEQ ID NO: 70 |
| L8 | SEQ ID NO: 71 | SEQ ID NO: 72 | SEQ ID NO: 70 |
| L9 | SEQ ID NO: 79 | SEQ ID NO: 80 | SEQ ID NO: 81 |
| L10 | SEQ ID NO: 82 | SEQ ID NO: 83 | SEQ ID NO: 81 |
| L11 | SEQ ID NO: 90 | SEQ ID NO: 91 | SEQ ID NO: 92 |
| L12 | SEQ ID NO: 93 | SEQ ID NO: 94 | SEQ ID NO: 92 |
| L13 | SEQ ID NO: 101 | SEQ ID NO: 102 | SEQ ID NO: 103 |
| L14 | SEQ ID NO: 104 | SEQ ID NO: 105 | SEQ ID NO: 103 |
| L15 | SEQ ID NO: 112 | SEQ ID NO: 113 | SEQ ID NO: 114 |
| L16 | SEQ ID NO: 115 | SEQ ID NO: 116 | SEQ ID NO: 114 |
| L17 | SEQ ID NO: 217 | SEQ ID NO: 102 | SEQ ID NO: 103 |
| L18 | SEQ ID NO: 227 | SEQ ID NO: 102 | SEQ ID NO: 103 |
| L19 | SEQ ID NO: 228 | SEQ ID NO: 102 | SEQ ID NO: 103 |
| L20 | SEQ ID NO: 229 | SEQ ID NO: 102 | SEQ ID NO: 103 |
| L21 | SEQ ID NO: 230 | SEQ ID NO: 102 | SEQ ID NO: 103 |
and,
(2) the heavy chain variable region comprises HCDR1, HCDR2 and HCDR3, the HCDR1 has any of a sequence of the following HCDR1, or a sequence with 1, 2, 3 or more amino acid insertions, deletions and/or substitutions compared with the sequence, the HCDR2 has any of a sequence of the following HCDR2, or a sequence with 1, 2, 3 or more amino acid insertions, deletions and/or substitutions compared with the sequence, and the HCDR3 has any of a sequence of the following HCDR3, or a sequence with 1, 2, 3 or more amino acid insertions, deletions and/or substitutions compared with the sequence:
| **No.** | **HCDR1** | **HCDR2** | **HCDR3** |
|---|---|---|---|
| H1 | SEQ ID NO: 29 | SEQ ID NO: 30 | SEQ ID NO: 31 |
| H2 | SEQ ID NO: 32 | SEQ ID NO: 33 | SEQ ID NO: 34 |
| H3 | SEQ ID NO: 40 | SEQ ID NO: 41 | SEQ ID NO: 42 |
| H4 | SEQ ID NO: 43 | SEQ ID NO: 44 | SEQ ID NO: 45 |
| H5 | SEQ ID NO: 51 | SEQ ID NO: 52 | SEQ ID NO: 53 |
| H6 | SEQ ID NO: 54 | SEQ ID NO: 55 | SEQ ID NO: 56 |
| H7 | SEQ ID NO: 62 | SEQ ID NO: 63 | SEQ ID NO: 64 |
| H8 | SEQ ID NO: 65 | SEQ ID NO: 66 | SEQ ID NO: 67 |
| H9 | SEQ ID NO: 73 | SEQ ID NO: 74 | SEQ ID NO: 75 |
| H10 | SEQ ID NO: 76 | SEQ ID NO: 77 | SEQ ID NO: 78 |
| H11 | SEQ ID NO: 84 | SEQ ID NO: 85 | SEQ ID NO: 86 |
| H12 | SEQ ID NO: 87 | SEQ ID NO: 88 | SEQ ID NO: 89 |
| H13 | SEQ ID NO: 95 | SEQ ID NO: 96 | SEQ ID NO: 97 |
| H14 | SEQ ID NO: 98 | SEQ ID NO: 99 | SEQ ID NO: 100 |
| H15 | SEQ ID NO: 106 | SEQ ID NO: 107 | SEQ ID NO: 108 |
| H16 | SEQ ID NO: 109 | SEQ ID NO: 110 | SEQ ID NO: 111 |
| H17 | SEQ ID NO: 40 | SEQ ID NO: 142 | SEQ ID NO: 42 |
| H18 | SEQ ID NO: 40 | SEQ ID NO: 143 | SEQ ID NO: 42 |
| H19 | SEQ ID NO: 51 | SEQ ID NO: 158 | SEQ ID NO: 53 |
| H20 | SEQ ID NO: 51 | SEQ ID NO: 159 | SEQ ID NO: 53 |
preferably, the antibody or the antigen-binding fragment thereof comprises sequences of six CDRs in the following combination of a light chain variable region and a heavy chain variable region: L1+H1, L2+H2, L3+H3, L4+H4, L5+H5, L6+H6, L7+H7, L8+H8, L9+H9, L10+H10, L11+H11, L12+H12, L13+ H13, L14+H14, L15+H15, L16+H16, L3+H17, L3+H18, L5+H19, L5+H20, L17+H13, L18+H13, L19+H13, L20+H13 or L21+H13, or sequences of six CDRs having 1, 2, 3 or more amino acid insertions, deletions and/or substitutions compared with the sequences of six CDRs.

2. The antibody or the antigen-binding fragment thereof of claim 1, **characterized in that**:
(1) the light chain variable region sequence comprises a sequence as shown in any one of SEQ ID NOs: 14, 16, 18, 20, 22, 24, 26, 28, 118-120, 130-131, 144-145, 160-163, 172-175, 184-187, 196-197, 206-208, 218-221 and 231-233, or a sequence having 80%, 85%, 90%, 95%, 96%, 97%, 98%, 99% or higher identity to the sequence;
and,
(2) the heavy chain variable region sequence comprises a sequence as shown in any one of SEQ ID NOs: 13, 15, 17, 19, 21, 23, 25, 27, 121-125, 132-137, 146-152, 164-166, 176-178, 188-190, 198-201, 209-212, 222 and 234-238, or a sequence having 80%, 85%, 90%, 95%, 96%, 97%, 98%, 99% or higher identity to the sequence;
preferably, the antibody or the antigen-binding fragment thereof has a light chain variable region and a heavy chain variable region as follows:
(1) the light chain variable region and the heavy chain variable region comprise sequences as shown in SEQ ID NO: 14 and SEQ ID NO: 13 respectively;
(2) the light chain variable region and the heavy chain variable region comprise sequences as shown in SEQ ID NO: 16 and SEQ ID NO: 15 respectively;
(3) the light chain variable region and the heavy chain variable region comprise sequences as shown in SEQ ID NO: 18 and SEQ ID NO: 17 respectively;
(4) the light chain variable region and the heavy chain variable region comprise sequences as shown in SEQ ID NO: 20 and SEQ ID NO: 19 respectively;
(5) the light chain variable region and the heavy chain variable region comprise sequences as shown in SEQ ID NO: 22 and SEQ ID NO: 21 respectively;
(6) the light chain variable region and the heavy chain variable region comprise sequences as shown in SEQ ID NO: 24 and SEQ ID NO: 23 respectively;
(7) the light chain variable region and the heavy chain variable region comprise sequences as shown in SEQ ID NO: 26 and SEQ ID NO: 25 respectively;
(8) the light chain variable region and the heavy chain variable region comprise sequences as shown in SEQ ID NO: 28 and SEQ ID NO: 27 respectively;
(9) the light chain variable region comprises a sequence as shown in any one of SEQ ID NOs: 118-120, and the heavy chain variable region comprises a sequence as shown in any one of SEQ ID NOs: 121-125;
(10) the light chain variable region comprises a sequence as shown in any one of SEQ ID NOs: 130-131, and the heavy chain variable region comprises a sequence as shown in any one of SEQ ID NOs: 132-137;
(11) the light chain variable region comprises a sequence as shown in any one of SEQ ID NOs: 144-145, and the heavy chain variable region comprises a sequence as shown in any one of SEQ ID NOs: 146-152;
(12) the light chain variable region comprises a sequence as shown in any one of SEQ ID NOs: 160-163, and the heavy chain variable region comprises a sequence as shown in any one of SEQ ID NOs: 164-166;
(13) the light chain variable region comprises a sequence as shown in any one of SEQ ID NOs: 172-175, and the heavy chain variable region comprises a sequence as shown in any one of SEQ ID NOs: 176-178;
(14) the light chain variable region comprises a sequence as shown in any one of SEQ ID NOs: 184-187, and the heavy chain variable region comprises a sequence as shown in any one of SEQ ID NOs: 188-190;
(15) the light chain variable region comprises a sequence as shown in any one of SEQ ID NOs: 196-197, and the heavy chain variable region comprises a sequence as shown in any one of SEQ ID NOs: 198-201;
(16) the light chain variable region comprises a sequence as shown in any one of SEQ ID NOs: 206-208, and the heavy chain variable region comprises a sequence as shown in any one of SEQ ID NOs: 209-212;
(17) the light chain variable region comprises a sequence as shown in any one of SEQ ID NOs: 218-221, and the heavy chain variable region comprises a sequence as shown in SEQ ID NO: 222;
(18) the light chain variable region comprises a sequence as shown in any one of SEQ ID NOs: 231-233, and the heavy chain variable region comprises a sequence as shown in any one of SEQ ID NOs: 234-238;
or (19) the light chain variable region comprises a sequence having 80%, 85%, 90%, 95%, 96%, 97 %, 98%, 99% or higher identity to the light chain variable region as shown in any one of the above (1) to (18), and the heavy chain variable region comprises a sequence having 80%, 85%, 90%, 95%, 96%, 97 %, 98%, 99% or higher identity to the heavy chain variable region as shown in any one of the above (1) to (18).

3. The antibody or the antigen-binding fragment thereof of claim 1 or 2, **characterized in that** the antibody or the antigen-binding fragment thereof is chimeric, humanized or fully human.

4. The antibody or the antigen-binding fragment thereof of any one of claims 1 to 3, **characterized in that** the antibody or the antigen-binding fragment thereof can bind to human or monkey GPRC5D.

5. The antibody or the antigen-binding fragment thereof of any one of claims 1 to 4, **characterized in that** the antigen-binding fragment is selected from one or more of F(ab)₂, Fab', Fab, Fv, scFv, nanobody or affibody.

6. The antibody or the antigen-binding fragment thereof of any one of claims 1 to 5, **characterized in that** the antibody or the antigen-binding fragment thereof further comprises a constant region sequence of any one of human or murine antibody IgG1, IgG2, IgG3, IgG4, IgA, IgM, IgE and IgD; preferably, the antibody or the antigen-binding fragment thereof comprises a constant region sequence of human or murine antibody IgG1, IgG2, IgG3 or IgG4, or comprises a sequence having 80%, 85%, 90%, 95%, 96%, 97%, 98%, 99% or higher identity to the constant region sequence of human or murine antibody IgG1, IgG2, IgG3 or IgG4; furthermore, the antibody or the antigen-binding fragment thereof is further coupled with a therapeutic agent or a tracer; preferably, the therapeutic agent is selected from a radioisotope, a chemotherapeutic drug or an immunomodulator, and the tracer is selected from a radiological contrast agent, a paramagnetic ion, a metal, a fluorescent tag, a chemiluminescence tag, an ultrasonic contrast agent and a photosensitizer; and more preferably, the cytotoxic agent is selected from alkaloids, methotrexate, anthracycline antibiotics, taxanes, pyrrolobenzodiazepine or toxin compounds.

7. A multispecific antigen-binding molecule, **characterized in that** the multispecific antigen-binding molecule comprises the antibody or the antigen-binding fragment thereof of any one of claims 1 to 6, and an additional antigen-binding molecule binding to an antigen other than GPRCSD or binding to a different GPRCSD epitope than that of the antibody or the antigen-binding fragment thereof of any one of claims 1 to 6;
preferably, the additional antigen-binding molecule is an antibody or an antigen-binding fragment thereof;
preferably, the multispecific antigen-binding molecule can be bispecific, trispecific or tetraspecific;
preferably, the multispecific antigen-binding molecule can be bivalent, trivalent, tetravalent, pentavalent or hexavalent.

8. A chimeric antigen receptor (CAR), **characterized in that** the chimeric antigen receptor comprises at least a signal peptide, an extracellular antigen-binding domain, a hinge region, a transmembrane domain and an intracellular signaling domain, and the extracellular antigen-binding domain comprises the GPRCSD antibody or the antigen-binding fragment thereof of any one of claims 1 to 6, or the multispecific antigen-binding molecule of claim 7.

9. An immune effector cell, **characterized in that** the immune effector cell expresses the chimeric antigen receptor of claim 8, or comprises a nucleic acid fragment encoding the chimeric antigen receptor of claim 8; preferably, the immune effector cell is selected from a T cell, an NK cell, an NKT cell, a DNT cell, a monocyte, a macrophage, a dendritic cell or a mast cell, and the T cell is preferably selected from a cytotoxic T cell (CTL), a regulatory T cell or a helper T cell; and preferably, the immune effector cell is an autologous immune effector cell or an allogeneic immune effector cell.

10. An isolated nucleic acid fragment, **characterized in that** the nucleic acid fragment encodes the antibody or the antigen-binding fragment thereof of any one of claims 1 to 6, the multispecific antigen-binding molecule of claim 7 or the chimeric antigen receptor of claim 8.

11. A vector, **characterized in that** the vector comprises the nucleic acid fragment of claim 10.

12. A host cell, **characterized in that** the host cell comprises the vector of claim 11; preferably, the cell is a prokaryotic cell or a eukaryotic cell, such as a bacterial (for example, *Escherichia coli*) cell, a fungal (for example, yeast) cell, an insect cell or a mammalian cell (for example, a CHO cell line or a 293T cell line).

13. A method for preparing the antibody or the antigen-binding fragment thereof of any one of claims 1 to 6 or the multispecific antigen-binding molecule of claim 7, **characterized in that** the method comprises culturing the cell of claim 12, and isolating the antibody, the antigen-binding fragment or the multispecific antigen-binding molecule expressed by the cell.

14. A method for preparing the aforementioned immune effector cell, **characterized in that** the method comprises introducing a nucleic acid fragment encoding the CAR of claim 8 into the immune effector cell, optionally, the method further comprises enabling the immune effector cell to express the CAR of claim 8.

15. A pharmaceutical composition, **characterized in that** the pharmaceutical composition comprises the antibody or the antigen-binding fragment thereof of any one of claims 1 to 6, the multispecific antigen-binding molecule of claim 7, the immune effector cell of claim 9, the nucleic acid fragment of claim 10, the vector of claim 11 or a product prepared according to the method of claim 13 or 14; optionally, the pharmaceutical composition further comprises a pharmaceutically acceptable carrier, a diluent or an auxiliary agent; and optionally, the pharmaceutical composition further comprises an additional anti-tumor agent.

16. A method for treating a tumor or a cancer, **characterized in that** the method comprises administering to a subject an effective amount of the antibody or the antigen-binding fragment thereof of any one of claims 1 to 6, the multispecific antigen-binding molecule of claim 7, the immune effector cell of claim 9, the nucleic acid fragment of claim 10, the vector of claim 11, a product prepared according to the method of claim 13 or 14 or the pharmaceutical composition of claim 15;
preferably, the tumor or the cancer is a GPRC5D-expressing tumor or cancer, preferably a B-cell lymphoma, and more preferably multiple myeloma (MM).

17. The antibody or the antigen-binding fragment thereof of any one of claims 1 to 6, the multispecific antigen-binding molecule of claim 7, the immune effector cell of claim 9, the nucleic acid fragment of claim 10, the vector of claim 11, a product prepared according to the method of claim 13 or 14 or the pharmaceutical composition of claim 15 for use in the preparation of a drug for treating a tumor or a cancer;
preferably, the tumor or the cancer is a GPRC5D-expressing tumor or cancer, preferably a B-cell lymphoma, and more preferably multiple myeloma (MM).

18. A kit, **characterized in that** the kit comprises the antibody or the antigen-binding fragment thereof of any one of claims 1 to 6, the multispecific antigen-binding molecule of claim 7, the immune effector cell of claim 9, the nucleic acid fragment of claim 10, the vector of claim 11, a product prepared according to the method of claim 13 or 14 or the pharmaceutical composition of claim 15.

19. A method for detecting GPRCSD expression in a biological sample, **characterized in that** the method comprises contacting the biological sample with the antibody or the antigen-binding fragment thereof of any one of claims 1 to 6 under conditions that allow the formation of a complex from the antibody or the antigen-binding fragment thereof and GPRC5D; preferably, the method further comprises detecting the formation of the complex, thereby indicating the presence or expression level of GPRCSD in the sample.

20. The antibody or the antigen-binding fragment thereof of any one of claims 1 to 6 for use in the preparation of a reagent for detecting GPRC5D.
